# EUROPEAN PATENT APPLICATION

(11) **EP 2 204 365 A1**
(43) Date of publication of application: **07.07.2010**
(21) Application number: 08830551.1
(22) Date of filing: 10.09.2008
(51) Int. Cl.: C07D 213/48, A61K 31/437, A61K 31/44, A61K 31/519, A61P 25/28, A61P 43/00, C07D 213/61, C07D 213/73, C07D 213/74, C07D 213/77, C07D 213/79, C07D 213/81, C07D 471/04

(54) **ALKYLSULFONE DERIVATIVE**

(30) Priority: 11.09.2007 JP 2007235580
(71) Applicant: Daiichi Sankyo Company, Limited, Chuo-ku Tokyo 103-8426 (JP)
(72) Inventor: IMORI, Hitoshi, Shinagawa-ku Tokyo 140-8710 (JP); KUBOTA, Hideki, Shinagawa-ku Tokyo 140-8710 (JP); MIYAUCHI, Satoru, Shinagawa-ku Tokyo 140-8710 (JP); MOTOKI, Kayoko, Shinagawa-ku Tokyo 140-8710 (JP)
(74) Representative: Fairbairn, Angus Chisholm
(86) International application number: PCT/JP2008/066279
(87) International publication number: WO 2009/034976

(57) **Abstract**

[Object] To provide a compound which has activity to inhibit production and/or secretion of β-amyloid protein, and is useful for prevention and/or treatment of various diseases associated with abnormal production and/or secretion of β-amyloid protein.

[Means for Solution]

A compound represented by the general formula (I): 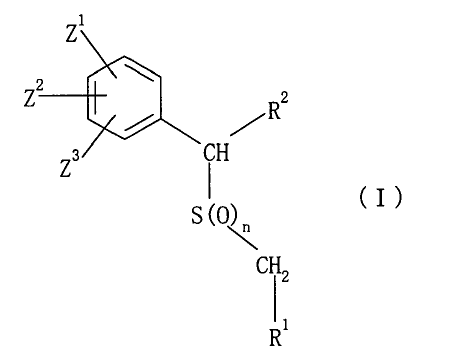 [wherein, R¹ represents a C1-C6 alkyl group which may have 1 to 6 halogen atoms as substituent groups, a C2-C6 alkenyl group which may have 1 to 6 halogen atoms as substituent groups, or a C3-C7 cycloalkyl group which may have 1 to 6 halogen atoms as substituent groups; R² represents a 6-membered nitrogen-containing monocyclic aromatic heterocyclic group having 1 to 3 substituent groups, or a 9- or 10-membered nitrogen-containing bicyclic heterocyclic group having 1 to 4 substituent groups; Z¹, Z² and Z³ each independently represent a hydrogen atom, a halogen atom or a cyano group; and n represents 0, 1 or 2], salts thereof, or solvates thereof.

## Description

### [TECHNICAL FIELD]

The present invention relates to a novel compound having activity to inhibit production and/or secretion of β-amyloid protein, and to a therapeutic agent for various diseases associated with abnormal production and/or secretion of β-amyloid protein, such as Alzheimer's disease, Down's syndrome and other diseases associated with amyloid deposition.

### [BACKGROUND ART]

Alzheimer's disease is a neurodegenerative disease having pathological characteristics in which degeneration and loss of neuronal cells, as well as formation of senile plaques and changes in neurofibrillary tangles are observed. Alzheimer's disease induces dementia symptoms, which is accompanied by progressive loss of memory, recognition, thinking, decisiveness or the like, and eventually leads to death. Until now, no useful method for preventing or treating the disease is known.

The major protein which constructs the senile plaques deposited in brain is β-amyloid protein (amyloid β protein, Aβ), and is composed of 39 to 43 amino acids. β-Amyloid protein shows cytotoxicity, and this is considered to induce Alzheimer's disease (Non-patent Document 1). It is known that the β-amyloid proteins secreted from cells are mainly polypeptides composed of 40 or 42 amino acids, and the β-amyloid protein composed of 42 amino acids has a stronger aggregability leading to early deposition in the brain, and has a strong cytotoxicity (Non-patent Document 2). The β-amyloid protein is generated ubiquitously in the body; however, its original function is not apparent.

The β-amyloid protein is generated by processing from amyloid precursor protein (APP) which is a membrane protein. Among patients of familial Alzheimer's disease, there are cases where mutation in APP gene is observed. In addition, it is known that in cells into which this mutated APP gene has been introduced, there is increase in the amount of production and/or secretion of β-amyloid protein. Accordingly, drugs which inhibit production and/or secretion of β-amyloid protein are considered to be useful for the prevention or treatment of Alzheimer's disease.

With respect to the procedure in which the β-amyloid protein is cleaved from the APP, BACE which is an aspartyl protease (APP cleavage enzyme at the β side) (Non-patent Document 3) and Asp1 (Non-patent Document 4) have been reported as a β-secretase which participates in the cleavage of the β-amyloid protein at the N-terminal side. On the other hand, regarding a γ-secretase which cleaves at the C-terminal side, it is strongly suggested that presenilin partially structures the γ-secretases (Non-patent Document 5). Inhibitors for these β-and γ-secretases have been reported (Non-patent Document 6), and most of them are peptidic compounds.

In Patent Document 1, SMITH et al. disclose a compound which has a sulfonamide backbone and controls the generation of β-amyloid protein. In addition, in Patent Document 2, BELANGER et al. disclose a compound which has a bicycloalkylsulfonamide backbone and inhibits γ-secretase. Further, in Patent Documents 3, 4 and 5, a compound having an activity to suppress the generation of β-amyloid protein is disclosed. Also in Patent Documents 6, 7 and 8, a diarylsulfone compound which inhibits γ-secretase is disclosed. In addition, in Patent Documents 9 and 10, a compound which suppresses the generation of β-amyloid protein is disclosed. On the other hand, in Patent Document 11, a thionaphthalene derivative which inhibits the aggregation of amyloid protein is disclosed. In addition, in Patent Document 12, a sulfone derivative which suppresses the generation of β-amyloid protein is disclosed.
[Patent Document 1] pamphlet of International Publication WO 00/50391
[Patent Document 2] pamphlet of International Publication WO 01/70677
[Patent Document 3] pamphlet of International Publication WO 02/40451
[Patent Document 4] pamphlet of International Publication WO 02/40508
[Patent Document 5] pamphlet of International Publication WO 02/47671
[Patent Document 6] pamphlet of International Publication WO 02/081433
[Patent Document 7] pamphlet of International Publication WO 02/081435
[Patent Document 8] pamphlet of International Publication WO 03/018543
[Patent Document 9] pamphlet of International Publication WO 03/055850
[Patent Document 10] pamphlet of International Publication WO 05/000798
[Patent Document 11] Japanese Patent Appication (Kokai) No. Hei 9-95444
[Patent Document 12] pamphlet of International Publication WO 2006/109729
[Non-patent Document 1] Science, Vol. 259, p. 514 (1993)
[Non-patent Document 2] Journal of Biological Chemistry Vol. 270, p. 7013 (1995)
[Non-patent Document 3] Science, Vol. 286, p. 735 (1999)
[Non-patent Document 4] Molecular and Cellular Neuroscience Vol. 16, p. 609 (2000)
[Non-patent Document 5] Journal of Medicinal Chemistry, Vol. 44, p. 2039 (2001)

### [DISCLOSURE OF THE INVENTION]

### [PROBLEMS TO BE SOLVED BY THE INVENTION]

An object of the present invention is to provide a compound which has strong inhibitory activity against the production and/or secretion of β-amyloid protein, and is useful for the prevention and/or treatment of various diseases associated with abnormal production and/or secretion of β-amyloid protein. Further, another object of the present invention is to provide a compound which shows sufficient solubility in water and realizes sufficient drug efficacy when administered to an animal or to a human, and is useful for the prevention and/or treatment of various diseases associated with abnormal production and/or secretion of β-amyloid protein.

### [MEANS FOR SOLVING THE PROBLEMS]

The inventors of the present invention have conducted various studies and found that a sulfide compound, a sulfoxide compound and a sulfone compound that are represented by the following general formula (I), strongly inhibit γ-secretase and thus suppress production and/or secretion of β-amyloid protein. Accordingly, the inventors have found out that the compounds are useful as a therapeutic agent for various diseases associated with abnormal production and/or secretion of β-amyloid protein, thereby leading to completion of the present invention. In addition, the inventors of the present invention have found that the sulfide compound, the sulfoxide compound and the sulfone compound that are represented by the following general formula (I) show sufficient solubility in water and realize sufficient drug efficacy when administered to an animal or to a human, thereby leading to completion of the present invention.

That is, the present invention provides:
1. a compound represented by the general formula (I): 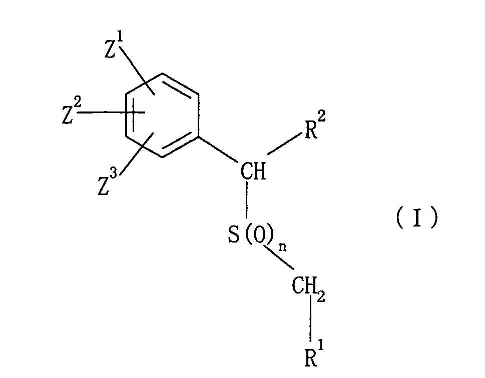 [wherein
   R¹ represents a C1-C6 alkyl group which may have 1 to 6 halogen atoms as substituent groups, a C2-C6 alkenyl group which may have 1 to 6 halogen atoms as substituent groups, or a C3-C7 cycloalkyl group which may have 1 to 6 halogen atoms as substituent groups;
   R² represents a 6-membered nitrogen-containing monocyclic aromatic heterocyclic group having 1 to 3 substituent groups, or a 9- or 10-membered nitrogen-containing bicyclic heterocyclic group having 1 to 4 substituent groups;
   Z¹, Z² and Z³ each independently represent a hydrogen atom, a halogen atom or a cyano group; and
   n represents 0, 1 or 2],
   salts thereof, or solvates thereof,
2. the compound according to the aforementioned 1, salts thereof, or solvates thereof, wherein R² in the general formula (I) is any one of a group selected from the group consisting of a pyridyl group, a triazolopyridyl group and a pyridopyrimidinyl group, having 1 to 3 substituent groups,
3. the compound according to the aforementioned 1, salts thereof, or solvates thereof, wherein R² in the general formula (I) is a pyridyl group having 1 to 3 substituent groups,
4. the compound according to the aforementioned 1, salts thereof, or solvates thereof, wherein R² in the general formula (I) is a group represented by the following formula (1): 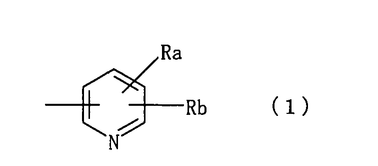 [wherein
   Rₐ represents a halogen atom or a C1-C6 alkyl group; and R_{b} represents a carboxy C1-C6 alkyl group, a carbamoyl group, a N-C1-C6 alkylcarbamoyl group, a N,N-di(C1-C6 alkyl)carbamoyl group, a N-(hydroxy C1-C6 alkyl)carbamoyl group, a carboxy group, an amino group, a N-(hydroxy C1-C6 alkyl)amino group or a C1-C6 alkylsulfonylamino group],
5. the compound according to the aforementioned 4, salts thereof, or solvates thereof, wherein Rₐ is a C1-C6 alkyl group and R_{b} is a N-(hydroxy C1-C6 alkyl)carbamoyl group in formula (1),
6. the compound according to the aforementioned 1, salts thereof, or solvates thereof, wherein R² in the general formula (I) is a group represented by the following formula (2): 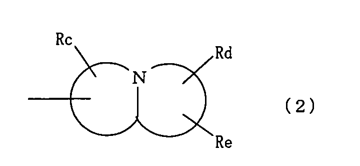 [wherein
   the group of nitrogen-containing ring structure represents a triazolopyridyl group or a pyridopyrimidinyl group; R_{c} represents a halogen atom or a C1-C6 alkyl group; R_{d} represents a hydrogen atom, a hydroxy group, an amino group, a N-(C1-C6 alkyl)amino group or a N-(hydroxy C1-C6 alkyl)amino group; and Rₑ represents a hydroxy group or an oxo group]
7. the compound according to any one of the aforementioned 1 to 6, salts thereof, or solvates thereof, wherein R¹ in the general formula (I) is a C1-C6 alkyl group which may have 1 to 6 halogen atoms as substituent groups,
8. the compound according to any one of the aforementioned 1 to 6, salts thereof, or solvates thereof, wherein R¹ in the general formula (I) is a 2,2,2-trifluoroethyl group, a 2-chloro-2,2-difluoroethyl group, a 2,2-difluoropropyl group or a 2,2,3,3,3-pentafluoropropyl group,
9. the compound according to any one of the aforementioned 1 to 8, salts thereof, or solvates thereof, wherein Z¹ and Z² are both fluorine atoms and Z³ is a hydrogen atom, or Z¹, Z² and Z³ are each a fluorine atom, in the general formula (I),
10. the compound according to any one of the aforementioned 1 to 9, salts thereof, or solvates thereof, wherein n in the general formula (I) is 2,
11. a medicament containing the compound according to any one of the aforementioned 1 to 10, salts thereof, or solvates thereof,
12. the medicament according to the aforementioned 11, which is a drug for preventing or treating a disease associated with abnormal generation and/or secretion of β-amyloid protein,
13. the medicament according to the aforementioned 12, wherein the disease associated with abnormal generation and/or secretion of β-amyloid protein is Alzheimer's disease or Down's syndrome,
14. a pharmaceutical composition containing the compound according to any one of aforementioned 1 to 10, salts thereof, or solvates thereof, and a pharmaceutically acceptable carrier,
15. use of the compound according to any one of the aforementioned 1 to 10, salts thereof, or solvates thereof, for the manufacture of a medicament,
16. the use according to the aforementioned 15, wherein the medicament is a drug for preventing or treating a disease associated with abnormal generation and/or secretion of β-amyloid protein,
17. the use according to the aforementioned 16, wherein the disease associated with abnormal generation and/or secretion of β-amyloid protein is Alzheimer's disease or Down's syndrome,
18. a method for treating a disease associated with abnormal generation and/or secretion of β-amyloid protein, comprising administration of an effective amount of the compound according to any one of the aforementioned 1 to 10, salts thereof, or solvates thereof,
19. the treatment method according to the aforementioned 18, wherein the disease associated with abnormal generation and/or secretion of β-amyloid protein is Alzheimer's disease or Down's syndrome,
   or the like.

### [EFFECT OF THE INVENTION]

According to the present invention, a means for medically preventing and treating various diseases such as Alzheimer's disease, Down's syndrome or other diseases associated with amyloid deposition can be provided.

### [BEST MODE FOR CARRYING OUT THE INVENTION]

Hereinafter, each of the groups described in the specification will be explained.

"C1-C6 alkyl group" means a straight chain or branched chain alkyl group having 1 to 6 carbon atoms. Specific examples include a methyl group, an ethyl group, a n-propyl group, an isopropyl group, a n-butyl group, an isobutyl group, a t-butyl group, a n-pentyl group, a 2-methylpentyl group, a 3,3-dimethylbutyl group and the like.

"C2-C6 alkenyl group" means a straight chain or branched chain alkenyl group having 2 to 6 carbon atoms. Specific examples include a vinyl group, an allyl group, a 1-propenyl group, a 1-butenyl group, a 2-butenyl group, a 3-butenyl group, a 2-pentenyl group and the like.

"C3-C7 cycloalkyl group" means a cycloalkyl group having 3-7 carbon atoms. Specific examples include a cyclopropyl group, a cyclobutyl group, a cyclopentyl group, a cyclohexyl group, and a cycloheptyl group.

"Halogen atom" means a fluorine atom, a chlorine atom, a bromine atom or an iodine atom.

"Carboxy C1-C6 alkyl group" means the aforementioned C1-C6 alkyl group having a carboxy group as a substituent group. The position and the number of substitution by the carboxy group is not particularly limited. Specific examples include a carboxymethyl group, a 2-carboxyethyl group and the like.

"N-C1-C6 alkylcarbamoyl group" means a carbamoyl group having one C1-C6 alkyl group mentioned above as a substituent group. The position at which the C1-C6 alkyl group substitutes is the nitrogen atom of the carbamoyl group. Specific examples include a N-methylcarbamoyl group, a N-ethylcarbamoyl group and the like.

"N,N-di(C1-C6 alkyl)carbamoyl group" means a carbamoyl group having two C1-C6 alkyl groups mentioned above as substituent groups. The positions at which the two C1-C6 alkyl group substitute are both the nitrogen atom of the carbamoyl group. The type of each C1-C6 alkyl group may be the same or different. Specific examples include a N,N-dimethylcarbamoyl group, a N-methyl-N-ethylcarbamoyl group and the like.

"N-(hydroxy C1-C6 alkyl)carbamoyl group" means a carbamoyl group having as a substituent group one C1-C6 alkyl group mentioned above having a hydroxy group as a substituent group. The position at which the C1-C6 alkyl group having the hydroxy group as a substituent group substitutes is the nitrogen atom of the carbamoyl group. Specific examples include a N-hydroxymethylcarbamoyl group, a N-hydroxyethylcarbamoyl group and the like.

"N-(hydroxy C1-C6 alkyl)amino group" means an amino group having as a substituent group one C1-C6 alkyl group mentioned above having a hydroxy group as a substituent group. Specific examples include a N-hydroxymethylamino group, a N-hydroxyethylamino group, and the like.

"C1-C6 alkylsulfonylamino group" means an amino group having as a substituent group one sulfonyl group having the aforementioned C1-C6 alkyl group as a substituent group. Specific examples include a methylsulfonylamino group, an ethylsulfonylamino group and the like.

"N-(C1-C6 alkyl)amino group" means an amino group having one C1-C6 alkyl group mentioned above as a substituent group. Specific examples include a N-methylamino group, a N-ethylamino group and the like.

"N-(hydroxy C1-C6 alkyl)amino group" means an amino group having as a substituent group one C1-C6 alkyl group mentioned above having a hydroxy group as a substituent group. Specific examples include a N-hydroxymethylamino group, a N-hydroxyethylamino group and the like.

"6-membered nitrogen-containing monocyclic aromatic heterocyclic group" means a 6-membered monocyclic aromatic heterocyclic group containing at least one nitrogen atom. The 6-membered nitrogen-containing monocyclic aromatic heterocyclic group may have 1 to 3 hetero atoms selected from a nitrogen atom, an oxygen atom and a sulfur atom, in addition to the one nitrogen atom. Specific examples include a pyridyl group, a pyridazinyl group, a pyrimidinyl group, a pyrazinyl group and the like.

"9- or 10-membered nitrogen-containing bicyclic heterocyclic group" means a 9- or 10-membered bicyclic heterocyclic group containing at least one nitrogen atom. The 9- or 10-membered nitrogen-containing bicyclic heterocyclic group may have 1 to 3 hetero atoms selected from a nitrogen atom, an oxygen atom and a sulfur atom, in addition to the one nitrogen atom. Specific examples include a benzothiazolyl group, an indolyl group, a quinolyl group, an isoquinolyl group, a benzoxazolyl group, benzothiazolyl group, a benzoimidazolyl group, a benzoisothiazolyl group, a benzoisoxazolyl group, an indolynyl group, an indazolyl group, an indolidinyl group, an isoindolyl group, an isoindolinyl group, a quinolizinyl group, a quinoxalinyl group, a quinazolinyl group, a cinnolinyl group, a phthaladinyl group, a naphthylidinyl group, a purinyl group, a tetrahydrothiazolopyridyl group, an imidazopyridyl group, a triazolopyridyl group, a pyrrolopyridyl group, a pyridopyrimidinyl group, pyridotriazinyl group, a tetrahydrothienopyridyl group and a dihydropyridooxazinyl group and the like.

Preferred embodiments of R¹, R², Z¹, Z², Z³ and n in the general formula (I) are described hereinafter.

R¹ in the general formula (I) represents a C1-C6 alkyl group which may have 1 to 6 halogen atoms as substituent groups, a C2-C6 alkenyl group which may have 1 to 6 halogen atoms as substituent groups, or a C3-C7 cycloalkyl group which may have 1 to 6 halogen atoms as substituent groups, wherein the C1-C6 alkyl group which may have 1 to 6 halogen atoms as substituent groups is preferred, and a C1-C6 alkyl group which has 1 to 6 halogen atoms as substituent groups is more preferred.

Hereinafter, the case in which R¹ is the C1-C6 alkyl group which may have 1 to 6 halogen atoms as substituent groups will be described in detail. R¹ means a C1-C6 alkyl group with no substituent group or a C1-C6 alkyl group having 1 to 6 halogen atoms as substituent groups. As R¹, a C1-C6 alkyl group having 1 to 6 halogen atoms as substituent groups is preferred, and a C1-C6 alkyl group having 3 to 6 halogen atoms as substituent groups is more preferred. Here, specific examples of the halogen atoms include those mentioned above, and a fluorine atom or a chlorine atom is preferred, and a fluorine atom is more preferred. In the case where the number of halogen atoms in R¹ is two or more, the type of each halogen atom may be identical or different. In addition, the position at which the halogen atom substitutes is not particularly limited. Specific examples of R¹ include a methyl group, an ethyl group, a n-propyl group, an isopropyl group, a n-butyl group, an isobutyl group, a 2,2-dimethylpropyl group, a 2-fluoroethyl group, a 2,2-difluoroethyl group, a 2,2,2-trifluoroethyl group, a 2,2-difluoropropyl group, a 3,3,3-trifluoropropyl group, a 2,2,4-trifluorobutyl group, a 2-chloroethyl group, a 2,2,2-trichloroethyl group, a 2-chloro-2,2-difluoroethyl group, a 1,1,2,2-tetrafluoroethyl group, a 2,2,3,3,3-pentafluoropropyl group and the like, and a 2,2,2-trifluoroethyl group, a 2-chloro-2,2-difluoroethyl group, a 2,2-difluoropropyl group or a 2,2,3,3,3-pentafluoropropyl group is preferred.

Hereinafter, the case in which R¹ is the C2-C6 alkenyl group which may have 1 to 6 halogen atoms as a substituent group will be described in detail. R¹ means a C2-C6 alkenyl group with no substituent group or a C2-C6 alkenyl group having 1 to 6 halogen atoms as substituent groups. As R¹, a C2-C6 alkenyl group having 1 to 6 halogen atoms as substituent groups is preferred, and a C2-C6 alkenyl group having 2 to 3 halogen atoms as substituent groups is more preferred. Here, specific example of the halogen atoms include those mentioned above, and a fluorine atom or a chlorine atom is preferred, and a fluorine atom is more preferred. In the case where the number of halogen atoms in R¹ is two or more, the type of each halogen atom may be identical or different. In addition, the position at which the halogen atom substitutes is not particularly limited. Specific examples of R¹ include a vinyl group, an allyl group, a 1-propenyl group, a 1-butenyl group, a 2-butenyl group, a 2,2-difluorovinyl group, a 3,3-difluoroallyl group, a 2,3,3-trifluoroallyl group and the like.

Hereinafter, the case in which R¹ is the C3-C7 cycloalkyl group which may have 1 to 6 halogen atoms as substituent groups will be described in detail. R¹ means a C3-C7 cycloalkyl group with no substituent group or a C3-C7 cycloalkyl group having 1 to 6 halogen atoms as substituent groups. As R¹, a C3-C7 cycloalkyl group having 1 to 6 halogen atoms as substituents group is preferred, and a C3-C7 cycloalkyl group having 2 to 3 halogen atoms as substituent groups is more preferred. Here, specific examples of the halogen atoms include those mentioned above, and a fluorine atom or a chlorine atom are preferred, and a fluorine atom is more preferred. In the case where the number of halogen atoms in R¹ is two or more, the type of each halogen atom may be identical or different. In addition, the position at which the halogen atom substitutes is not particularly limited. Specific examples of R¹ include a cyclopropyl group, a cyclobutyl group, a cyclopentyl group, a cyclohexyl group, a cycloheptyl group, a 3,3-difluorocyclobutyl group, a 4,4-difluorocyclohexyl group and the like.

R² in the general formula (I) represents a 6-membered nitrogen-containing monocyclic aromatic heterocyclic group having 1 to 3 substituent groups or a 9- or 10-membered nitrogen-containing bicyclic heterocyclic group having 1 to 4 substituent groups. As R², either one of a group selected from a pyridyl group, a pyridazinyl group, a pyrimidinyl group and a pyrazinyl group, having 1 to 3 substituent groups, or either one of a group selected from the group consisting of an imidazopyridyl group, a triazolopyridyl group, a pyridopyrimidinyl group and a pyridotriazinyl group, having 1 to 4 substituent groups, is preferred, a pyridyl group having 1 to 3 substituent groups or either one of a group selected from triazolopyridyl group and a pyridopyrimidinyl group, having 1 to 4 substituent groups, is more preferred, and a pyridyl group having 1 to 3 substituent groups is further preferred.

The substituent group(s) in R² include a halogen atom, a cyano group, a C1-C6 alkyl group, a hydroxy group, a C1-C6 alkoxy group, a hydroxy C1-C6 alkoxy group, a C2-C6 alkanoyl group, a carboxy C1-C6 alkyl group, a hydroxy C1-C6 alkyl group, a heterocycle-C1-C6 alkyl group, an amino C1-C6 alkyl group, a C1-C6 alkylamino C1-C6 alkyl group, a di(C1-C6 alkyl)amino C1-C6 alkyl group, a hydroxy C1-C6 alkylamino C1-C6 alkyl group, a C1-C6 alkylthio C1-C6 alkyl group, a C1-C6 alkylsulfinyl C1-C6 alkyl group, a C1-C6 alkylsulfonyl C1-C6 alkyl group, a C2-C6 alkanoylamino C1-C6 alkyl group, a formyl group, a carboxy group, a heterocycle-carbonyl group, a C1-C6 alkoxycarbonyl group, a N-alkylaminothiocarbonyl group, a N,N-di(C1-C6 alkyl)aminothiocarbonyl group, a carbamoyl group, a N-C1-C6 alkylcarbamoyl group, a N-(halogeno C1-C6 alkyl)carbamoyl group, a N,N-di(C1-C6 alkyl)carbamoyl group, a N-(hydroxy C3-C7 cycloalkyl)carbamoyl group, a N-C3-C7 cycloalkylcarbamoyl group, a C3-C7 cycloalkyl C1-C6 alkylcarbamoyl group, a C1-C6 alkylthio C1-C6 alkylcarbamoyl group, a C1-C6 alkylsulfinyl C1-C6 alkylcarbamoyl group, a C1-C6 alkylsulfonyl C1-C6 alkylcarbamoyl group, a hydroxycarbamoyl group, a C1-C6 alkoxycarbamoyl group, a C2-C6 alkanoylcarbamoyl group, a N-(hydroxy C1-C6 alkyl)carbamoyl group, a N,N-di(hydroxy C1-C6 alkyl)carbamoyl group, a N-(hydroxy C1-C6 alkyl)-N-C1-C6 alkylcarbamoyl group, a N-C1-C6 alkoxy-N-C1-C6 alkylcarbamoyl group, a N-(carboxy C1-C6 alkyl)carbamoyl group, a C1-C6 alkoxy C1-C6 alkylcarbamoyl group, an amino C1-C6 alkylcarbamoyl group, a C2-C6 alkanoylamino C1-C6 alkylcarbamoyl group, a C2-C6 alkanoyloxy C1-C6 alkylcarbamoyl group, a hydroxy C2-C6 alkanoyloxy C1-C6 alkylcarbamoyl group, a hydroxy C1-C6 alkyloxy C1-C6 alkylcarbamoyl group, a carboxy C1-C6 alkyloxy C1-C6 alkylcarbamoyl group, a C1-C6 alkoxycarbonyl C1-C6 alkyloxy C1-C6 alkylcarbamoyl group, a C1-C6 alkylamino C1-C6 alkylcarbamoyl group, a N,N-di(C1-C6 alkyl)amino C1-C6 alkylcarbamoyl group, a C1-C6 alkoxycarbonyl C1-C6 alkylcarbamoyl group, a (C1-C6 alkoxycarbonylamino)C1-C6 alkylcarbamoyl group, a N-(mercapto C1-C6 alkyl)carbamoyl group, a heterocycle-carbamoyl group, a heterocyle-C1-C6 alkylcarbamoyl group, a heterocycle-C1-C6 alkyloxy C1-C6 alkylcarbamoyl group, a C6-C10 aromatic hydrocarbon-C1-C6 alkyloxy C1-C6 alkylcarbonyloxy C1-C6 alkylcarbamoyl group, an amino group, a N-(C1-C6 alkyl)amino group, a N-(hydroxy C1-C6 alkyl)amino group, a C1-C6 alkoxy C1-C6 alkylamino group, an amino C1-C6 alkylamino group, a (C1-C6 alkylamino C1-C6 alkyl)amino group, a N-(C1-C6 alkylamino C1-C6 alkyl)-N-C1-C6 alkylamino group, (C1-C6 alkoxycarbonylamino C1-C6 alkyl)amino group, a (C1-C6 alkylcarbonylamino C1-C6 alkyl)amino group, a (C1-C6 alkylsulfonylamino C1-C6 alkyl)amino group, a (di(C1-C6 alkyl)amino C1-C6 alkyl)amino group, a (heterocycle-amino C1-C6 alkyl)amino group, a carboxy C1-C6 alkylamino group, a N-carboxy C1-C6 alkyl-N-C1-C6 alkylamino group, a carbamoyl C1-C6 alkylamino group, a C1-C6 alkylcarbamoyl C1-C6 alkylamino group, a di(C1-C6 alkyl)carbamoyl C1-C6 alkylamino group, a heterocycle-C1-C6 alkylamino group, a N-(heterocycle-C1-C6 alkyl)-N-C1-C6 alkylamino group, a N-hydroxy C1-C6 alkyl-N-C1-C6 alkylamino group, a N-(C1-C6 alkylaminocarbonyloxy C1-C6 alkyl)-N-C1-C6 alkylamino group, a C1-C6 alkylthio C1-C6 alkylamino group, a C1-C6 alkylsulfinyl C1-C6 alkylamino group, a C1-C6 alkylsulfonyl C1-C6 alkylamino group, a group represented by the general formula -N-(R¹²)SO₂R¹¹ (wherein, R¹¹ is a C1-C6 alkyl group, a hyterocyclic group, a C1-C6 alkyl-heterocyclic group, a heterocycle-C1-C6 alkyl group, a hydroxy C1-C6 alkyl group, an amino C1-C6 alkyl group, a C1-C6 alkylamino C1-C6 alkyl group, a di(C1-C6 alkyl)amino C1-C6 alkyl group, a carboxy C1-C6 alkyl group, a carbamoyl C1-C6 alkyl group, a trifluoromethyl group, a difluoromethyl group or a fluoromethyl group, R¹² represents a hydrogen atom, a C1-C6 alkyl group, a hydroxy group or an amino group), a hydroxy C1-C6 alkoxy C1-C6 alkylamino group, a C1-C6 alkoxycarbonylamino group, a hydroxy C1-C6 alkylcarbonylamino group, a C2-C6 alkanoylamino group, a C1-C6 alkylsulfonylamino group, a heterocycle-amino group, a heterocycle-carbonylamino group, a heterocycle-carbonyl C1-C6 alkylamino group, a hydrazino group, a C1-C6 alkylsulfonyl group, an oxo group, a heterocyclic group, a C6-C10 aromatic hydrocarbon-heterocyclic group, a heterocycle-heterocyclic group (here, a C6-C10 aromatic hydrocarbon, a heterocyclic ring and a heterocyclic group may be each independently substituted with 1 to 3 substituents selected from a halogen atom, a C1-C6 alkyl group, a hydroxy C1-C6 alkyl group, a carboxy C1-C6 alkyl group, an amino C1-C6 alkyl group, a C1-C6 alkoxy C1-C6 alkyl group, a C1-C6 alkoxy group, a C2-C6 alkenyl group, a formyl group, a C2-C6 alkanoyl group, a carboxy group, an oxo group, a hydroxy group, a thioxo group, an amino group, a C1-C6 alkylamino group and a di(C1-C6 alkyl)amino group). In the case where the number of substituent groups in R² is two or more, the type of each substituent group may be identical or different. In addition, the position at which each substituent group substitutes is not particularly limited.

Here, "heterocyclic ring" means a ring having 1 to 4 hetero atoms (N, O, S or the like) as a constituent part of the ring structure, and may be any one of saturated, unsaturated or aromatic ring, and may be either one of monocyclic or polycyclic ring. In addition, a polycyclic heterocyclic ring includes a heterocyclic spiro compound and a heterocyclic compound having a bridged cyclic structure. The "heterocycle" in the case where it is described in "heterocycle-C1-C6 alkyl group" or the like means a heterocyclic group derived from the aforementioned heterocyclic ring. Here, the "heterocyclic group" means a monovalent group derived from a "heterocyclic ring". A saturated monocylic heterocyclic group includes a 3 to 7 membered ring having 1 to 4 hetero atoms selected from a nitrogen atom, an oxygen atom and a sulfur atom, and specific examples include a pyrrolidinyl group, a tetrahydrofuranyl group, an oxetanyl group, a tetrahydrothienyl group, a piperidinyl group, a piperazinyl group, a homopiperazinyl group, a morpholinyl group, a thiomorpholinyl group, an oxylanyl group, a thiolanyl group, a dioxanyl group, an aziridinyl group, an imidazolidinyl group, a pyrazolidinyl group, a tetrahydropyranyl group, a tetrahydrothiopyranyl group, an oxazolidinyl group, a thiazolidinyl group, an isoxazolidinyl group, an isothiazolidinyl group, a dioxolanyl group, an oxathiolanyl group, a hexahydropyrimidinyl group and the like.

The unsaturated or aromatic monocyclic heterocyclic group includes a 4 to 7 membered group having 1 to 4 hetero atoms selected from a nitrogen atom, an oxygen atom and a sulfur atom, and specific examples include a pyrrolyl group, a furyl group, a thienyl group, a pyrazolyl group, an imidazolyl group, a triazolyl group, an oxazolyl group, a thiazolyl group, an isoxazolyl group, an isothiazolyl group, a triazinyl group, a tetrazolyl group, a thiadiazolyl group, an oxadiazolyl group, a pyridyl group, a pyrimidinyl group, a pyrazinyl group, a pyridazinyl group, a pyrrolidinyl group, an imidazolidinyl group, a pyrazolidinyl group, an oxazolidinyl group, a thiazolidinyl group, an isoxazolidinyl group, an isothiazolidinyl group, a pyranyl group, a dihydropyridyl group, a tetrahydropyridyl group, a dihydropyridazinyl group, a dihydropyrimidinyl group, a tetrahydropyridazinyl group, a tetrahydropyrimidinyl group and the like.

The polycyclic heterocyclic group includes a 8 to 14 membered group having 1 to 4 hetero atoms selected from a nitrogen atom, an oxygen atom and a sulfur atom, and specific examples include a benzofuranyl group, a benzothiazolyl group, an indolyl group, a quinolyl group, an isoquinolyl group, a benzopyranyl group, a benzoxazolyl group, a benzothiazolyl group, a benzimidazolyl group, a benzodioxanyl group, a benzothiophenyl group, a benzisothiazolyl group, a benzisoxazolyl group, a chromenyl group, a chromanyl group, an isochromenyl group, an isochromanyl group, an indolinyl group, an indazolyl group, an indolidinyl group, an isoindolyl group, an isoindolinyl group, a quinolidinyl group, a quinoxalinyl group, a quinazolinyl group, a cinnolinyl group, a phthaladinyl group, a naphthylidinyl group, a purinyl group, a tetrahydrothiazolopyridyl group, an imidazopyridyl group, a pyrrolopyridyl group, a carbazolyl group, a xanthenyl group, an acrydinyl group, a phenadinyl group, a phenoxazinyl group, a phenothiazinyl group, a quinuclidinyl group and the like.

In addition, the "C6-C10 aromatic hydrocarbon" means an aromatic hydrocarbon ring having 6 to 10 carbon atoms, and includes benzene, naphthalene and the like. The "C6-C10 aromatic hydrocarbon" in the case where it is described in C6-C10 aromatic hydrocarbon-C1-C6 alkyl group or the like means a monovalent C6-C10 aromatic hydrocarbon group derived from a C6-C10 aromatic hydrocarbon, and includes a phenyl group, a naphthyl group and the like.

Hereinafter, the case in which R² is the 6-membered nitrogen-containing monocyclic aromatic heterocyclic group having 1 to 3 substituent groups will be described in detail. The nitrogen-containing monocyclic aromatic heterocyclic group includes a pyridyl group, a pyridazinyl group, a pyrimidinyl group, a pyrazinyl group and the like, and the pyridyl group is preferred. The substituent group(s) include those described above, and a halogen atom, a C1-C6 alkyl group, a carboxy C1-C6 alkyl group, a carbamoyl group, a N-C1-C6 alkylcarbamoyl group, a N,N-di(C1-C6 alkyl)carbamoyl group, a N-(hydroxy C1-C6 alkyl)carbamoyl group, an amino group, a N-(hydroxy C1-C6 alkyl)amino group and a C1-C6 alkylsulfonylamino group are preferred among them. The number of substituent groups is 1 to 3, preferably 1 to 2, and more preferably 2. In the case where the number of substituent groups is 2 or 3, the type of each substituent group may be identical or different. In addition, the position at which the substituent group substitutes is not particularly limited.

In a preferred example, R² is a group represented by the following formula (1).

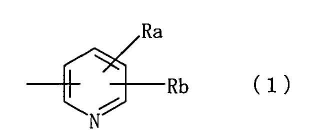

Here, Rₐ represents a halogen atom or a C1-C6 alkyl group; and R_{b} represents a carboxy C1-C6 alkyl group, a carbamoyl group, a N-C1-C6 alkylcarbamoyl group, a N,N-di(C1-C6 alkyl)carbamoyl group, a N-(hydroxy C1-C6 alkyl)carbamoyl group, a carboxy group, an amino group, a N-(hydroxy C1-C6 alkyl)amino group or a C1-C6 alkylsulfonylamino group.

Here, the halogen atom includes those described above, and a chlorine atom is preferred. The C1-C6 alkyl group includes the aforementioned ones, and a methyl group is preferred. The N-(hydroxy C1-C6 alkyl)carbamoyl group includes those described above, and a N-hydroxymethylcarbamoyl group or a N-hydroxyethylcarbamoyl group is preferred. The carboxy C1-C6 alkyl group, the N-C1-C6 alkylcarbamoyl group, the N,N-di(C1-C6 alkyl)carbamoyl group, the N-(hydroxy C1-C6 alkyl)amino group and the C1-C6 alkylsulfonylamino group include those described above.

With respect to formula (1), it is preferred that Rₐ is a halogen atom or a C1-C6 alkyl group, and R_{b} is a carbamoyl group or a N-(hydroxy C1-C6 alkyl)carbamoyl group; and it is more preferred that Rₐ is a C1-C6 alkyl group, and R_{b} is a N-(hydroxy C1-C6 alkyl)carbamoyl group.

Specific examples of formula (1) include: 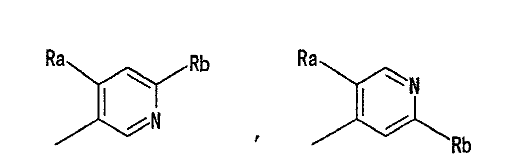 and the like. Here, Rₐ and R_{b} represent the same as those described above, and the preferred embodiments are also the same as those described above.

Further specific examples of formula (1) include a 6-carbamoyl-4-methylpyridin-3-yl group, a 6-[N,N-dimethylcarbamoyl]-4-methylpyridin-3-yl group, a 6-[N-(hydroxymethyl)carbamoyl]-4-methylpyridin-3-yl group, a 6-[N-(hydroxyethyl)carbamoyl]-4-methylpyridin-3-yl group, a 2-amino-5-chloropyridin-4-yl group, a 2-carbamoyl-5-chloropyridin-4-yl group, a 5-chloro-2-[N-(hydroxymethyl)carbamoyl]pyridin-4-yl group and the like.

Hereinafter, the case in which R² is the 9- or 10-membered nitrogen-containing bicyclic heterocyclic group having 1 to 4 substituent groups will be described in detail. The 9- or 10-membered nitrogen-containing bicyclic heterocyclic group includes a benzothiazolyl group, an indolyl group, a quinolyl group, an isoquinolyl group, a benzoxazolyl group, a benzothiazolyl group, a benzoimidazolyl group, a benzoisothiazolyl group, a benzoisoxazolyl group, an indolinyl group, an indazolyl group, an indolidinyl group, an isoindolyl group, an isoindolinyl group, a quinolidinyl group, a quinoxalinyl group, a quinazolinyl group, a cinnolinyl group, a phthalazinyl group, a naphthyridinyl group, a purinyl group, a tetrahydrothiazolopyridyl group, an imidazopyridyl group, a triazolopyridyl group, a pyrrolopyridyl group, a pyridopyrimidinyl group, a pyridotriazinyl group, a tetrahydrothienopyridyl group, a dihydropyridoxazinyl group and the like, and an imidazopyridyl group, a triazolopyridyl group, a pyridopyrimidinyl group, a pyridotriazinyl group, a triazolopyridyl group or a pyridopyrimidinyl group is preferred, and a triazolopyridyl group or a pyridopyrimidinyl group is more preferred. The substituent groups include those described above, and a halogen atom, a C1-C6 alkyl group, a hydroxy group, an amino group, a N-(C1-C6 alkyl)amino group, a N-(hydroxy C1-C6 alkyl)amino group or an oxo group is preferred among them. The number of substituent groups is 1 to 4, preferably 1 to 3, and more preferably 2 or 3. In the case where the number of substituent groups is 2 to 4, the type of each substituent group may be identical or different. In addition, the position at which the substituent group substitutes is not particularly limited.

In addition, in a preferred example, R² is a group represented by the following formula (2).

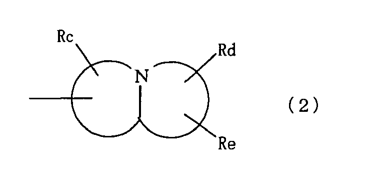

Here, a group having a nitrogen-containing ring structure represents a triazolopyridyl group or a pyridopyrimidinyl group; R_{c} represents a halogen atom or a C1-C6 alkyl group; R_{d} represents a hydrogen atom, a hydroxy group, an amino group, a N-(C1-C6 alkyl)amino group or a N-(hydroxy C1-C6 alkyl)amino group; and Rₑ represents a hydroxy group or an oxo group.

Here, the halogen atom, the C1-C6 alkyl group, the N-(C1-C6 alkyl)amino group and the N-(hydroxy C1-C6 alkyl)amino group include those described above. With respect to the halogen atom, a chlorine atom is preferred.

With respect to formula (2), it is preferred that the group having a nitrogen-containing cyclic structure is a triazolopyridyl group, R_{c} is a halogen atom, R_{d} is a hydrogen atom, and Rₑ is an oxo group; or that the group having a nitrogen-containing cyclic structure is a pyridopyrimidinyl group, R_{c} is a halogen atom, R_{d} is a hydroxy group or an amino group, and Rₑ is an oxo group.

Further specific examples of formula (2) include a 6-chloro-3-oxo-2H-[1,2,4]triazolo[4,3-a]pyridin-7-yl group, a 7-chloro-2-hydroxy-4-oxo-4H-pyrido[1,2-a]pyrimidin-8-yl group, and a 2-amino-7-chloro-4-oxo-4H-pyrido[1,2-a]pyrimidin-8-yl group.

Z¹, Z² and Z³ in the general formula (I) each independently represent a hydrogen atom, a halogen atom or a cyano group. The halogen atom includes those described above, and a fluorine atom or a chlorine atom is preferred. With respect to Z¹, Z² and Z³, it is preferred that Z¹ and Z² are both fluorine atoms and Z³ is a hydrogen atom; or that each one of Z¹, Z² and Z³ is a fluorine atom. It is more preferred that each one of Z¹, Z² and Z³ is a fluorine atom.

Specific examples of Z¹, Z² and Z³, and a phenyl group which is substituted by them include:

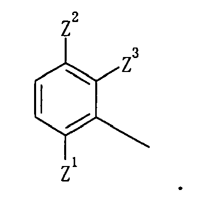

Here, Z¹, Z² and Z³ are the same as those described above, and the preferred embodiments are also the same as those described above. Further specific examples of Z¹, Z² and Z³, and the phenyl group which is substituted by them include a 2,3,6-trifluorophenyl group, a 2,5-difluorophenyl group and the like.

The n in the general formula (I) represents 0, 1 or 2. It is preferred that n is 1 or 2, and more preferably, n is 2.

There is no particular limitation with respect to the combination of R¹, R², Z¹, Z², Z³ and n in the general formula (I), and one preferred example of the combination is the case where R¹ is a C1-C6 alkyl group which may have 1 to 6 halogen atoms as substituent groups, R² is a group represented by the following formula (1): 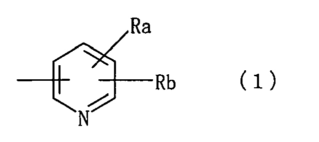 [wherein
Rₐ is a C1-C6 alkyl group; and
R_{b} is a N-(hydroxy C1-C6 alkyl)carbamoyl group], each one of Z¹, Z² and Z³ is a fluorine atom, and n is 2.

With respect to the compound represented by the general formula (I) according to the present invention, there are cases where a stereoisomer or an optical isomer derived from an asymmetric carbon exist. Such stereoisomer, optical isomer and a mixture thereof are also included in the present invention.

In addition, in the case where a heterocyclic group having a nitrogen atom is included in the structure of the compound represented by the general formula (I) according to the present invention, a N-oxide exists, and this is also included in the present invention. Further, in the case where a heterocyclic group having a sulfur atom is included in the structure of the compound represented by the general formula (I) according to the present invention, a S-oxide exists, and this S-oxide includes both of a mono-oxide and a di-oxide. Both of these are also included in the present invention.

A salt of the compound represented by the general formula (I) according to the present invention is not particularly limited so long as it is a salt which is medically acceptable. Specific examples include mineral acid salts such as a hydrochloride, a hydrobromide, a hydroiodide, a phosphate, a nitrate and a sulfate; a benzoate; an organic sulfonates such as a methanesulfonate, a 2-hydroxyethanesulfonate and a p-toluenesulfonate; organic carboxylates such as an acetate, a propanoate, an oxalate, a malonate, a succinate, a glutarate, an adipate, a tartrate, a maleate, a malate, a mandelate and the like.

In addition, in the case where the compound represented by the general formula (I) has an acidic group, it may be a salt of alkali metal ion or alkaline earth metal ion. With respect to solvates, there is no particular limitation so long as they are medically acceptable. Specific examples include a hydrate, an ethanolate and the like.

Hereinafter, a manufacturing method for the compound represented by the general formula (I) according to the present invention will be described.

The compound represented by the general formula (I), salts thereof and solvates thereof according to the present invention can be manufactured by combining known general chemical manufacturing methods, and a typical synthetic method will be described hereinafter.

Typical methods regarding a manufacturing method for a sulfide compound (Ia), a sulfoxide compound (Ib) and a sulfone compound (Ic) represented by the general formula (I) according to the present invention are given hereinafter.

### 1) Manufacturing Method For Sulfide Compound (Ia)

The sulfide compound (Ia) of the present invention can be manufactured by the following method. 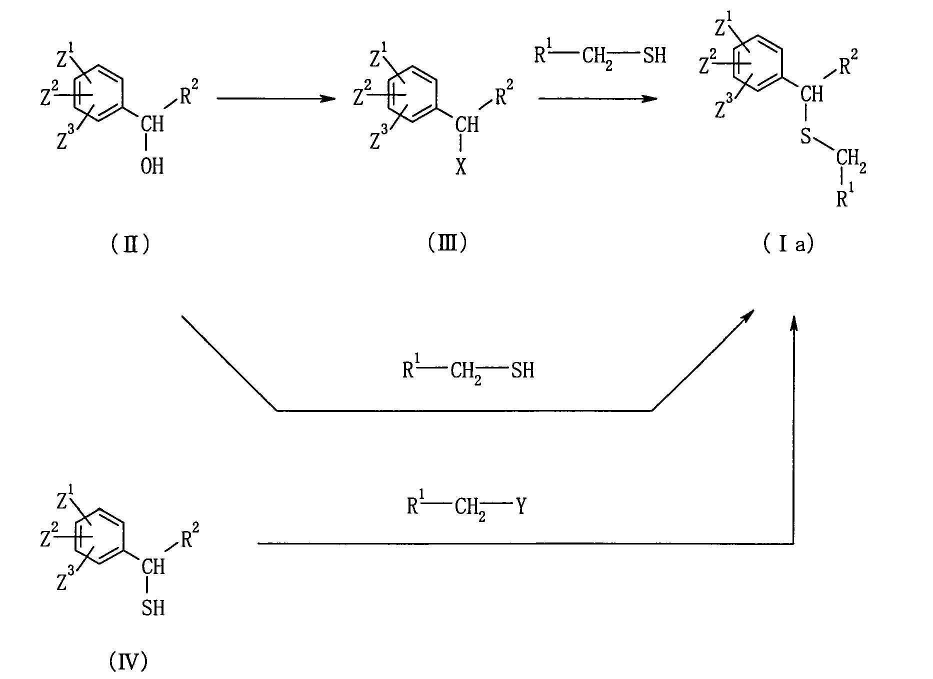 [wherein X and Y represent a leaving group, and R¹, R² and Z¹ to Z³ represent the same as described above.]

After leading an alcohol derivative (II) to compound (III), the obtained compound (III) and a thiol compound (R¹-CH₂-SH) are reacted in the presence of a base, and thus the sulfide compound (Ia) of the present invention can be manufactured. In this case, the thiol compound can be used as an alkali metal salt or as an alkaline earth metal salt (for example, a lithium salt, a sodium salt, or a potassium salt).

The temperature during the reaction of the compound (III) and the thiol compound (R¹-CH₂-SH) is generally -20 to 200°C, preferably from room temperature to 100°C. Depending on the type of the compound (III) or the thiol compound (R¹-CH₂-SH), there are cases where a higher reaction temperature is preferred, and cases where it is preferred to carry out the reaction in a sealed tube. The reaction time is generally from 0.5 hours to 1 day.

With respect to the base, hydrides of alkali metal or alkaline earth metal (for example, lithium hydride, sodium hydride, potassium hydride and calcium hydride); amides of alkali metal or alkaline earth metal (for example, lithium amide, sodium amide, lithium diisopropylamide, lithium dicyclohexylamide, lithium hexamethyldisilazide, sodium hexamethyldisilazide and potassium hexamethyldisilazide); lower-alkoxides of alkali metal or alkaline earth metal (for example, sodium methoxide, sodium ethoxide and potassium t-butoxide); hydroxides of alkali metal, alkaline earth metal or silver (for example, silver hydroxide, sodium hydroxide, potassium hydroxide, lithium hydroxide and barium hydroxide); carbonates of alkali metal, alkaline earth metal or silver (for example, sodium carbonate, potassium carbonate, cesium carbonate and silver carbonate); hydrogen carbonates of alkali metal (for example, sodium hydrogen carbonate and potassium hydrogen carbonate); alkyl lithiums (for example, n-butyl lithium) or an alkyl Grignards (for example, magnesium methylbromide); inorganic bases such as silver oxide, or organic bases such as amines (for example, triethylamine, diisopropylethylamine and N-methylmorpholine); basic heterocyclic compounds (for example, dimethylaminopyridine, pyridine, imidazole, 2,6-lutidine, collidine, 1,8-diazabicyclo[5,4,0]undec-7-ene, 1,5-diazabicyclo[4,3,0]non-5-ene, and 1,4-diazabicyclo[2,2,2]octane) and the like can be mentioned.

With respect to solvents, alcohol solvents, ether solvents, halogenated solvents, aromatic solvents, nitrile solvents, amide solvents, ketone solvents, sulfoxide solvents and water can be mentioned, and two or more of these can be blended for usage. Among these, tetrahydrofuran, methylene chloride, acetonitrile, N,N-dimethylformamide and the like are preferred.

The alcohol derivative (II) used in the aforementioned manufacturing process can be manufactured by a known method, and various examples are known as manufacturing methods. One example is given below. 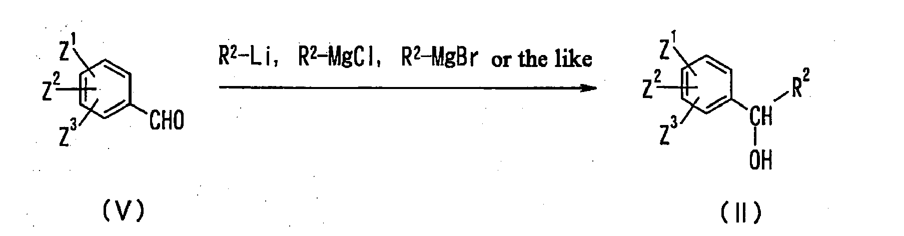 [wherein R² and Z¹ to Z³ represent the same as described above].

The alcohol derivative (II) can be obtained by reacting an aldehyde (V) with an equivalent or excess amount of an organic metal reagent (typically, organic lithium reagents represented by R²-Li, or Grignard reagents represented by R²-MgCl or R²-MgBr or the like), in a solvent such as tetrahydrofuran or diethyl ether. The aforementioned organic metal reagents can be prepared easily by adding an alkyl lithium reagent or an alkyl Grignard reagent to a halogenated aryl or a halogenated heteroaryl to allow transmetalation, as described in the paper of H. Gilman et al., J. Org. Chem. Vol. 16, 1788-1791 (1951), or in the paper of F. Trecourt et al., Tetrahedron, Vol. 56, 1349-1460 (2000).

The compound (III) having a leaving group X can be manufactured, starting from the alcohol derivative (II), via conversion of a hydroxyl group to a leaving group by a known method. With respect to the leaving group represented by X, a halogen atom (a chlorine atom, a bromine atom, an iodine atom or the like), a C1-C6 alkylsulfonyloxy group which may be halogenated (methanesulfonyloxy, ethanesulfonyloxy, trifluoromethanesulfonyloxy or the like), a C6-C10 aromatic hydrocarbon sulfonyloxy group which may have a substituent group(s), and the like can be mentioned. With respect to the substituent group(s) of the aromatic hydrocarbon sulfonyloxy group, 1 to 3 halogen atoms, a C1-C6 alkyl group which may be halogenated, a C1-C6 alkoxy group and the like can be mentioned. As preferred examples of the leaving group, a benzenesulfonyloxy group, a p-toluenesulfonyloxy group, a 1-naphthalenesulfonyloxy group, a 2-naphthalenesulfonyloxy group and the like can be mentioned.

In addition, as an alternative synthetic method for the sulfide compound (Ia), Mitsunobu reaction of the alcohol derivative (II) and a thiol compound (R¹-CH₂-SH) can be mentioned. Specifically, the alcohol derivative (II) and 1 to 3 equivalents of the thiol compound (R¹-CH₂-SH) are allowed to react in a solvent in the presence of 1 to 3 equivalents of triarylphosphine (for example, triphenylphosphine or the like) or trialkylphosphine (for example, tributylphosphine) and 1 to 2 equivalents of an azodicarboxylic acid compound (for example, diethyl azodicarboxylate, diisopropyl azodicarboxylate, azodicarboxylic acid dipiperidineamide or azodicarboxylic acid bisdimethylamide).

The reaction temperature is generally -20 to 150°C, preferably from room temperature to 80°C. The reaction time is generally from 0.5 hours to 1 day. With respect to the solvent, ether solvents, halogenated solvents and aromatic solvents can be mentioned, and two or more types of these can be blended for usage. Among these, tetrahydrofuran is preferred.

In addition, as an alternative synthetic method for the sulfide compound (Ia), a substitution reaction of a compound having a leaving group Y (R¹-CH₂-Y) with a thiol compound (IV) in the presence of a base can be mentioned. In this case, the thiol compound (IV) may be used as an alkali metal salt or as alkaline earth metal salt (for example, a lithium salt, a sodium salt or a potassium salt). Further, with respect to the leaving group represented by Y, a halogen atom (a chlorine atom, a bromine atom, an iodine atom or the like), a C1-C6 alkylsulfonyloxy group which may be halogenated (a methanesulfonyloxy group, an ethanesulfonyloxy group, a trifluoromethanesulfonyloxy group or the like), a C6-C10 aromatic hydrocarbon sulfonyloxy group which may have a substituent group(s), and the like can be mentioned. With respect to the substituent group(s) of the aromatic hydrocarbon sulfonyloxy group, 1 to 3 halogen atoms, a C1-C6 alkyl group which may be halogenated, a C1-C6 alkoxy group and the like can be mentioned. As preferred examples of the leaving group, a benzenesulfonyloxy group, a p-toluenesulfonyloxy group, a 1-naphthalenesulfonyloxy group, a 2-naphthalenesulfonyloxy group and the like can be mentioned.

The temperature during the reaction of the thiol compound (IV) and the compound (R¹-CH₂-Y) is generally -20 to 200°C, preferably from room temperature to 100°C. Depending on the type of the thiol compound (IV) or the compound (R¹-CH₂-Y), there are cases where a higher reaction temperature is preferred, and cases where it is preferred to carry out the reaction in a sealed tube. The reaction time is generally from 0.5 hours to 1 day.

With respect to the base, hydrides of alkali metal or alkaline earth metal (for example, lithium hydride, sodium hydride, potassium hydride and calcium hydride); amides of alkali metal or alkaline earth metal (for example, lithium amide, sodium amide, lithium diisopropylamide, lithium dicyclohexylamide, lithium hexamethyldisilazide, sodium hexamethyldisilazide and potassium hexamethyldisilazide); lower-alkoxides of alkali metal or alkaline earth metal (for example, sodium methoxide, sodium ethoxide and potassium t-butoxide); hydroxides of alkali metal, alkaline earth metal or silver (for example, silver hydroxide, sodium hydroxide, potassium hydroxide, lithium hydroxide and barium hydroxide); carbonates of alkali metal, alkaline earth metal or silver (for example, sodium carbonate, potassium carbonate, cesium carbonate and silver carbonate); hydrogen carbonates of alkali metal (for example, sodium hydrogen carbonate and potassium hydrogen carbonate); alkyl lithiums (for example, n-butyl lithium) or alkyl Grignards (for example, magnesium methylbromide); inorganic bases such as silver oxide; or organic bases such as amines (for example, triethylamine, diisopropylethylamine and N-methylmorpholine); basic heterocyclic compounds (for example, dimethylaminopyridine, pyridine, imidazole, 2,6-lutidine, collidine, 1,8-azabicyclo[5,4,0]undec-7-ene, 1,5-diazabicyclo[4,3,0]non-5-ene, and 1,4-diazabicyclo[2,2,2]octane) and the like can be mentioned.

With respect to solvents, alcohol solvents, ether solvents, halogenated solvents, aromatic solvents, nitrile solvents, amide solvents, ketone solvents, sulfoxide solvents and water can be mentioned, and two or more of these can be blended for usage. Among these, tetrahydrofuran, methylene chloride, acetonitrile, N,N-dimethylformamide and the like are preferred.

The thiol compound (IV) used in the aforementioned manufacturing process can be manufactured by a known method, and various examples are known as manufacturing methods. One example is given below. 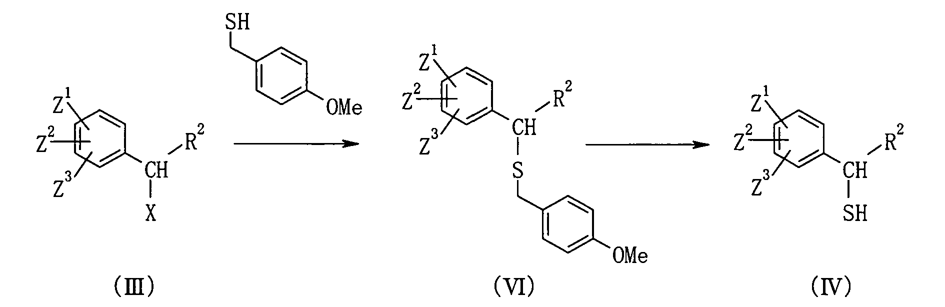 [wherein X represents a leaving group, and R² and Z¹ to Z³ represent the same as described above].

The thiol compound (IV) can be manufactured by manufacturing the sulfide compound (VI) from the compound (III) and (4-methoxyphenyl)methanethiol in accordance with the aforementioned manufacturing method of the sulfide compound (Ia), followed by cleavage of 4-methoxybenzyl from the sulfide compound (VI) by a known method.

### 2) Manufacturing Method For Sulfoxide Compound (Ib)

The sulfoxide compound (Ib) of the present invention can be manufactured by oxidation of the sulfide compound (Ia) with an oxidizing agent in a solvent, as shown below. 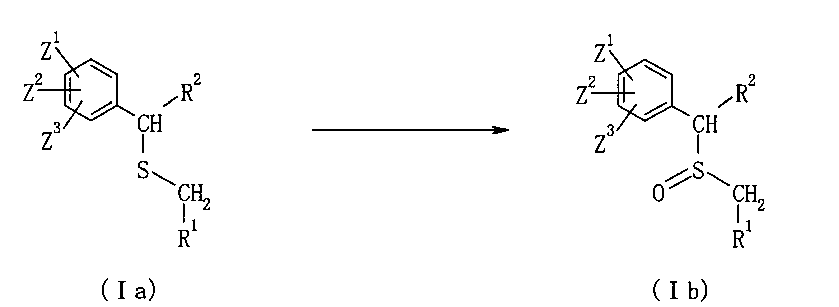 [wherein R¹, R² and Z¹ to Z³ represent the same as described above].

The reaction temperature is generally -20 to 200°C, preferably 0 to 100°C. With respect to the solvent, alcohol solvents, ether solvents, halogenated solvents, aromatic solvents, nitrile solvents, amide solvents, ketone solvents, sulfoxide solvents and water can be mentioned, and two or more types of these can be blended for usage. Among these, methylene chloride, chloroform, methanol, ethanol and the like are preferred.

As the oxidizing agent, hydrogen peroxide, organic peroxide compounds (for example, peracetic acid and metha-chloroperbenzoic acid), metha-periodic acid salts (for example, sodium metha-periodate), acyl nitrate, dinitrogen tetraoxide, halogen, N-halogenated compounds (for example, N-chlorosuccinimide and N-bromosuccinimide), hydroperoxides (for example, t-butylhydroperoxide), iodobenzene diacetate, iodobenzene dichloride, t-butyl hypochlorite, sulfuryl chloride, singlet oxygen, ozone, selenium oxide, seleninic acid and the like can be mentioned.

An example of particular reaction conditions is to treat the sulfide compound (Ia) with 1 to 2 equivalents of metha-chloroperbenzoic acid, sodium periodate or hydrogen peroxide, in a solvent such as methylene chloride, tetrahydrofuran-water, methanol or the like, at 0 to 100°C for approximately 1 hour to 2 days, thereby enabling the manufacture of the sulfoxide compound (Ib).

In addition, in the case where an optically active sulfoxide (Ib) is manufactured, titanium tetraisopropoxide/optically pure diethyl tartrate/ t-butyl hydroperoxide, titanium tetraisopropoxide/ optically pure diethyl tartrate/ peracetic acid and the like can be used as the oxidizing agent.

### 3-1) Manufacturing Method For Sulfone Compound (Ic)

The sulfone compound (Ic) of the present invention can be manufactured by oxidation of the sulfide compound (Ia) or the sulfoxide compound (Ib) with an oxidizing agent in a solvent, as shown below. 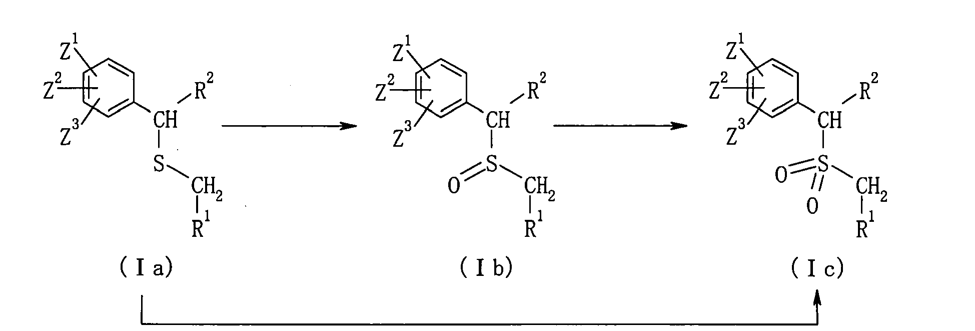 [wherein R¹, R² and Z¹ to Z³ represent the same as described above].

The reaction temperature is generally -20 to 150°C, preferably 0 to 80°C.

As the solvent, alcohol solvents, ether solvents, halogenated solvents, aromatic solvents, carboxylic acid solvents, nitrile solvents, amide solvents, ketone solvents, sulfoxide solvents and water can be mentioned, and two or more types of these can be blended for usage. Among these, methylene chloride, chloroform, methanol, ethanol, acetic acid and the like are preferred.

As the oxidizing agent, hydrogen peroxide, hydrogen peroxide-transition metal catalyst (for example, ammonium molybdate, ferric chloride (III) or the like), organic peroxide compounds (for example, peracetic acid and metha-chloroperbenzoic acid), metha-periodic acid salts (for example, sodium metha-periodate or the like), potassium peroxysulfate, permanganates (for example, potassium permanganate or the like), sodium perborate, halogen, N-halogenated compounds (for example, N-chlorosuccinimide, N-bromosuccinimide or the like), hydroperoxides (for example, t-butylhydroperoxide or the like), iodobenzene diacetate, iodobenzene dichloride, hypochlorous acids (for example, sodium hypochlorite, t-butyl hypochlorite and the like), singlet oxygen, ozone, selenium oxide, seleninic acid and the like can be used. With respect to preferred reaction conditions, for example, the sulfide compound (Ia) and 2 to 5 equivalents of an oxidizing agent (for example, metha-chloroperbenzoic acid, sodium periodate, hydrogen peroxide, hydrogen peroxide-ammonium molybdate or the like) can be allowed to react in methylene chloride, tetrahydrofuran-water or methanol, at 0 to 100°C for approximately from 1 hour to 2 days.

### 3-2) Manufacturing Method For Sulfone Compound (Ic)

The sulfone compound (Ic) can be manufactured also by the following method. 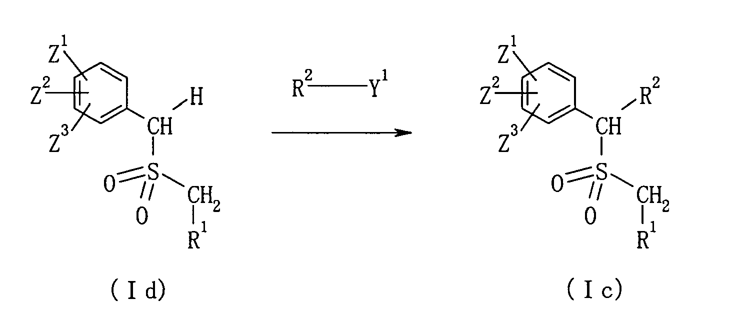 [wherein Y¹ represents a leaving group or a hydroxyl group, and R¹, R² and Z¹ to Z³ represent the same as described above].

The sulfone compound (Ic) having various types of R² group can be manufactured by allowing the sulfone compound (Id), which can be prepared by a known method or by applying the method with modification, to react with an electrophilic agent (R²-Y¹) in the presence of a base.

Specifically, compound (Id) and an equivalent to excess amount of a base are allowed to react with an equivalent to excess amount of R²-Y¹. The reaction temperature is generally - 78 to 200°C, and the reaction time is generally from 0.5 hours to 1 day.

With respect to the solvent, ether solvents, halogenated solvents, aromatic solvents, nitrile solvents, amide solvents and the like can be used alone or blended for usage. Among these, tetrahydrofuran, dimethoxyethane, dimethylether, N,N-dimethylformamide, toluene and the like are preferred.

With respect to the leaving group represented by Y¹, a halogen atom (a chlorine atom, a bromine atom, an iodine atom or the like), a C1-C6 alkylsulfonyloxy group which may be halogenated (methanesulfonyloxy, ethanesulfonyloxy, trifluoromethanesulfonyloxy or the like), a C6-C10 aromatic hydrocarbon sulfonyloxy group which may have a substituent group(s), and the like can be mentioned. With respect to the substituent group(s) of the aromatic hydrocarbon sulfonyloxy group, 1 to 3 halogen atoms, a C1-C6 alkyl group which may be halogenated, a C1-C6 alkoxy group and the like can be mentioned. As specific examples of the leaving group, a benzenesulfonyloxy group, a p-toluenesulfonyloxy group, a 1-naphthalenesulfonyloxy group, a 2-naphthalenesulfonyloxy group and the like can be mentioned.

With respect to the base, alkyl lithiums (for example, n-butyl lithium, sec-butyl lithium and t-butyl lithium); hydrides of alkali metal or alkaline earth metal (for example, lithium hydride, sodium hydride, potassium hydride and calcium hydride); amides of alkali metal or alkaline earth metal (for example, lithium amide, sodium amide, lithium diisopropylamide, lithium dicyclohexylamide, lithium hexamethyldisilazide, sodium hexamethyldisilazide and potassium hexamethyldisilazide); lower-alkoxides of alkali metal or alkaline earth metal (for example, sodium methoxide, sodium ethoxide and potassium t-butoxide); hydroxides of alkali metal, alkaline earth metal or silver (for example, silver hydroxide, sodium hydroxide, potassium hydroxide, lithium hydroxide and barium hydroxide); carbonates of alkali metal, alkaline earth metal or silver (for example, sodium carbonate, potassium carbonate, cesium carbonate and silver carbonate); hydrogen carbonates of alkali metal (for example, sodium hydrogen carbonate and potassium hydrogen carbonate); silver oxide and the like can be mentioned.

In addition, in the case where R²-Y¹ is R²-OH, the sulfone compound (Ic) according to the present invention can be manufactured also by allowing the sulfone compound (Id) and 1 to 3 equivalents of R²-OH to react in a solvent in the presence of a condensing agent.

As the condensing agent which can be used in the aforementioned reaction, any one of cyanomethylene trialkylphospholanes (for example, cyanomethylene trimethylphospholane and cyanomethylene tri-n-butylphospholane); triarylphosphines (for example, triphenylphosphine); or trialkylphosphines (for example, tributylphosphine), and an azodicaroboxylic acid compound (for example, diethyl azodicarboxylate, diisopropyl azodicarboxylate, azodicarboxylic acid dipiperidineamide and azodicarboxylic acid bisdimethylamide) and the like can be mentioned.

The reaction temperature is generally -20 to 200°C, preferably 0 to 150°C. The reaction time is generally from 0.5 hours to 3 days. With respect to the solvent, ether solvents, halogenated solvents and aromatic solvents can be mentioned, and 2 or more types of these can be blended for usage. Among these, tetrahydrofuran, toluene and the like are preferred.

### 3-3) Manufacturing Method For Sulfone Compound (Ic)

The sulfone compound (Ic) of the present invention can be manufactured also by allowing compound (III) to react with sulfinic acid salt (VII), as described below. 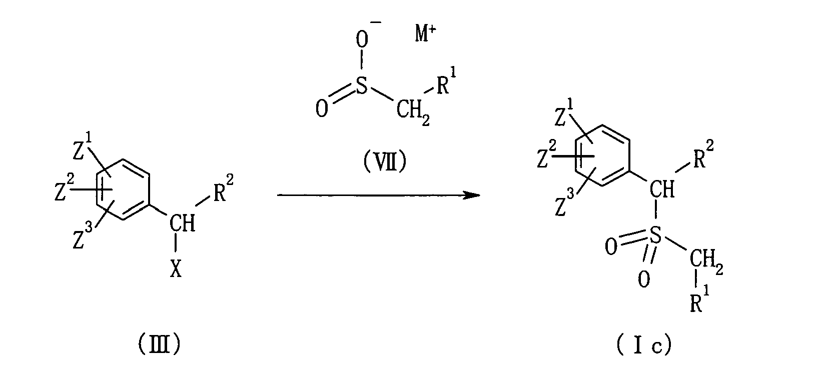 [wherein X represents a leaving group, M⁺ represents a metal ion (for example, a lithium ion, a sodium ion, and a potassium ion or the like), and R¹, R² and Z¹ to Z³ represent the same as described above].

Specifically, compound (III) is allowed to react with an equivalent to excess amount of sulfinic acid salt (VII) in a solvent. The reaction temperature is generally -20 to 200°C, preferably from room temperature to 100°C. Depending on the type of compound (III) or sulfinic acid salt (VII), there are cases where a higher reaction temperature is preferred, and cases where it is preferred to carry out the reaction in a sealed tube. The reaction time is generally from 0.5 hours to 1 day.

With respect to the solvent, alcohol solvents, ether solvents, halogenated solvents, aromatic solvents, nitrile solvents, amide solvents, ketone solvents, sulfoxide solvents and water can be mentioned, and these may be blended for usage. Among these, butanol, dimethoxyethane and the like are preferred.

### 4) Manufacturing Method For 2H-[1,2,4]triazolo[4,3-a]pyridine Compound (Ic-2)

2H-[1,2,4]triazolo[4,3-a]pyridine compound (Ic-2) of the present invention can be manufactured by a known method or by applying the method with modification. Various examples are known as manufacturing methods and one example is shown below. 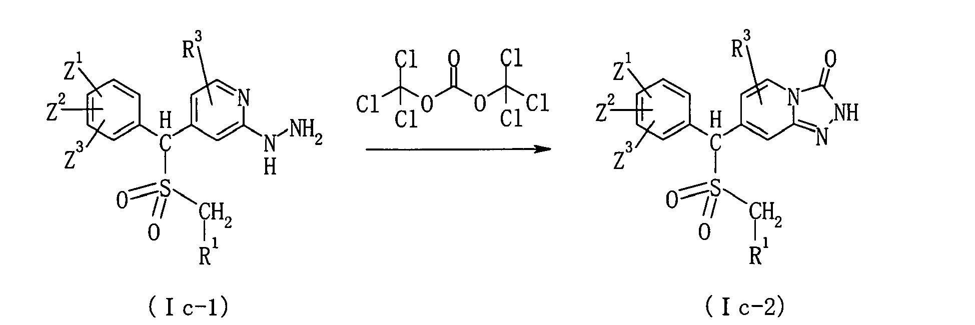 [wherein R³ represents a halogen atom or a C1-C6 alkyl group, and R¹, R² and Z¹ to Z³ represent the same as described above].

2H-[1,2,4]triazolo[4,3-a]pyridine compound (Ic-2) can be manufactured by allowing the hydrazinopyridine compound (Ic-1), which can be prepared by a known method or by applying the method with modification, to react with an equivalent to excess amount of triphosgene in a solvent.

### 5) Manufacturing Method For 4H-pyrido[1,2-a]pyrimidine Compound (Ic-4)

4H-pyrido[1,2-a]pyrimidine compound (Ic-4) of the present invention can be manufactured by a known method or by applying the method with modification. Various examples are known as the manufacturing method, and one example is shown below. 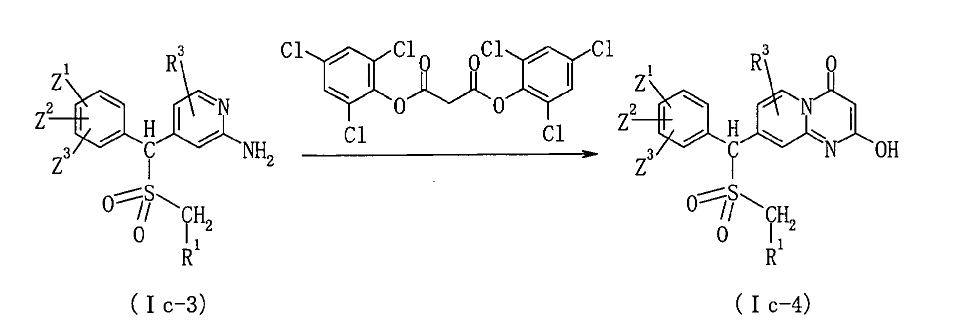 [wherein R¹, R³ and Z¹ to Z³ represent the same as described above].

4H-pyrido[1,2-a]pyrimidine compound (Ic-4) can be manufactured by allowing the aminopyridine compound (Ic-3), which can be prepared by a known method or by applying the method with modification, to react with an equivalent to excess amount of bis(2,4,6-trichlorophenyl)malonate ester in a solvent. In addition, the hydroxyl group of compound (Ic-4) can be converted into a group such as an amino group, a N-(C1-C6 alkyl)amino group or a N-(hydroxy C1-C6 alkyl)amino group, by a known method.

The manufacturing method of the compound (I) according to the present invention as exemplified above may require protection of a nitrogen atom, and a substituent group such as a hydroxyl group and a carboxy group. In such case, general protecting groups which are known and are appropriately removable can be used, and these protecting groups can be removed by a general method in organic chemistry, when necessary.

Structures of 1 or more substituents in R² of sulfide compound (Ia), sulfoxide compound (Ib) and sulfone compound (Ic) manufactured by the aforementioned method, can be further converted. For example, in the case where it has a substituent substituted with a 1,3-dioxolan-2-yl group, it can be deprotected by a known method and converted into a compound substituted with a formyl group. The formyl group can be converted into a carboxylic acid, an aminomethyl group, a hydroxymethyl group or the like by a known method. Further, the hydroxyl group portion of the hydroxymethyl group can be converted into a group such as ester, carbonate, carbamate, halogen or sulfonate by a known method. In addition, these groups can be converted into a group such as alkoxy, amine, amide or sulfide. Such conversion can be conducted for various functional groups in addition to the hydroxyl group, and their conversion method can be carried out by known techniques. Reagents, solvents and reaction conditions used in these conversion processes can be adopted from those known to a person skilled in the art.

General methods can lead the compound (I) according to the present invention, which is manufactured by the aforementioned method, to a salt or a solvate.

The compound represented by the general formula (I) according to the present invention strongly inhibited the production secretion of β-amyloid protein in vitro. Therefore, the compound represented by the general formula (I) according to the present invention is useful as a preventive and therapeutic drug for diseases associated with abnormal production-secretion of β-amyloid protein such as Alzheimer's disease and Down's syndrome, and other diseases associated with amyloid deposition.

In addition, the compound represented by the general formula (I) according to the present invention showed sufficient solubility in water under acidic to neutral conditions. In the case where a compound having poor solubility in water is orally administered, the extent of absorption of the compound from the gastrointestinal tract is generally low, due to the low solubility. Further, even in the case where the extent of absorption of the compound from the gastrointestinal tract is relatively high, individual differences for the extent of absorption in administered animal or human are observed, due to the low solubility. In this regard, since the compound represented by the general formula (I) according to the present invention shows sufficient solubility in water under acidic to neutral conditions, sufficient drug efficacy can be achieved when the compound is administered to an animal or human. Accordingly, it is considered to be extremely useful as a preventive and therapeutic drug for diseases associated with abnormal production and/or secretion of β**-**amyloid protein such as Alzheimer's disease and Down's syndrome, and other diseases associated with amyloid deposition.

In the case where the compound according to the present invention is used as a medicament for the human body, dosage is in the range of 1 mg to 1 g, preferably 10 to 300 mg per day. Although the daily dosage for an animal varies depending on the aim of administration (therapy or prevention), type and size of the animal to be treated, it is generally in the range of 0.1 to 200 mg, preferably 0.5 to 100 mg per 1 kg of the animal's weight. This daily dosage can be administered once a day, or may be administered in portions 2 to 4 times a day. The daily dosage may exceed the above amount if necessary.

A pharmaceutical composition containing the compound according to the present invention may adopt a suitable preparation depending on the administration method, and can be formulated by various kinds of formulating methods that are generally used. As examples of dosage forms of the pharmaceutical composition which contains the compound according to the present invention as a main component, tablets, powders, granules, capsules, solutions, syrups, elixirs, and oil-based or aqueous suspensions and the like can be mentioned as oral formulations.

With respect to injections, stabilizers, preservatives and solubilizing agents may be used in the formulation. Here, the solution which may contain such adjuvants may be stored in a container, followed by lyophilization or the like to obtain a solid formulation as a formulation for preparation before use. In addition, an amount required for one dose may be stored in one container, or an amount required for a plurality of doses may be stored in one container.

In addition, solutions, suspensions, emulsions, ointments, gels, creams, lotions, sprays, patches and the like can be mentioned as examples of topical formulations.

Regarding solid formulations, pharmaceutically acceptable additives are included with the compound according to the present invention. For example, fillers, bulking agents, binders, disintegrants, solubilizing agents, moistening agents, lubricants and the like are selected as necessary and are blended to obtain a formulation.

With respect to liquid formulations, solutions, suspensions, emulsions and the like can be mentioned; however, there are cases where suspending agents, emulsifiers and the like are included as additives.

### [EXAMPLES]

Hereinafter, the present invention will be specifically explained with reference to the examples; however, the scope of the present invention is not limited to the following examples. Here, in the following examples, when there is no description with respect to E-form or Z-form, the resulting compound is either one of the E-form or the Z-form.

The symbols of "IR", "¹H-NMR" and "MS" in the examples mean "infrared absorption spectrum", "nuclear magnetic resonance spectrum" and "mass spectrometry" respectively. The ratio of eluting solvent described in isolation and purification by chromatography refers to volume ratio, unless otherwise mentioned. "IR" was measured by ATR method. The content of the parentheses in "¹H-NMR" shows the solvent used for measurement, and TMS (tetramethylsilane) was used as an internal standard. With respect to the multiplicity in ¹H-NMR, the meanings are: s=singlet, d=doublet, t=triplet, q=quintet, m=multiplet and br s=broad singlet.

In addition, the following abbreviations are used in the specification.
- CDCl₃:: Deuterochloroform
- DMSO:: Dimethylsulfoxide
- DMSO-d₆:: Hexadeuterodimethylsulfoxide

### Reference Example 1: 2-Bromo-5-[[(4-methoxybenzyl)thio](2,3,6-trifluorophenyl)methyl]-4-methylpyridine

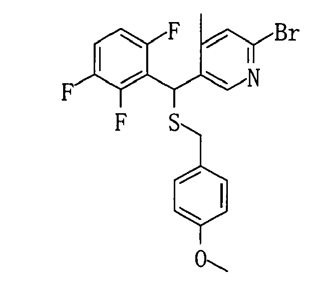

A suspension of 2-bromo-5-[chloro(2,3,6-trifluorophenyl)methyl]-4-methylpyridine (4.91 g, 14.0 mmol), 4-methoxytoluenethiol (2.59 g, 16.8 mmol) and potassium carbonate (2.32 g, 16.8 mmol) in N,N-dimethylformamide (10 ml) was stirred at room temperature for 29 hours. 4-Methoxytoluenethiol (1.08 mmol, 7.0 mmol) and potassium carbonate (967 mg, 7.0 mmol) were added to the reaction suspension, and the suspension was stirred at room temperature for 4 hours. Water was added to the reaction suspension, followed by extraction with ethyl acetate. The organic layer was washed with saturated saline, dried over anhydrous sodium sulfate, then filtered, and the filtrates were concentrated under reduced pressure. The concentrated residue obtained was subjected to filtering column eluting with hexane:ethyl acetate = 1:1, and the eluent was concentrated under reduced pressure. The concentrated residue obtained was subjected to flash silica gel column chromatography using 20% ethyl acetate/hexane as eluent. The resulting fractions were concentrated under reduced pressure to give the title compound (5.59 g, 11.9 mmol, 85%) as a solid.
¹H-NMR (400 MHz, CDCl₃) δ: 1.99 (3H, s), 3.61-3.74 (2H, m), 3.80 (3H, s), 5.20 (1H, s), 6.74-6.85 (3H, m), 6.99-7.13 (3H, m), 7.21 (1H, s), 8.86 (1H, s).
IR (ATR) cm⁻¹: 1608, 1579, 1508, 1490, 1461, 1434, 1344, 1301, 1249, 1234, 1168.
MS (m/z): 468, 470 (M⁺+H).
Elemental analysis: C₁₉H₁₂C1₃F₂NO₃S: Theoretical value: C 47.67; H 2.53; Cl 22.22; F 7.94; N 2.93; S 6.70. Observed value: C 47.35; H 2.53; C 121.95; F 7.89; N 2.99; S 6.82.

### Reference Example 2: (6-Bromo-4-methylpyridin-3-yl) (2,3,6-trifluorophenyl)methanethiol

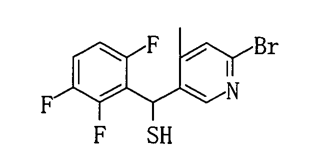

A trifluoroacetic acid (30 ml) solution of 2-bromo-5-[[(4-methoxybenzyl)thio](2,3,6-trifluorophenyl)methyl]-4-methylpyridine (3.27 g, 6.98 mmol) was stirred at 60°C for 2 days. The reaction solution was allowed to cool to room temperature, and then concentrated under reduced pressure. A methanol (30 ml) solution of the concentrated residue obtained was heated at reflux for 2.5 hours. The reaction solution was allowed to cool to room temperature, and then concentrated under reduced pressure. Methylene chloride was added to the concentrated residue obtained, and the resulting mixture was washed with saturated aqueous sodium hydrogen carbonate. The organic layer was dried over anhydrous magnesium sulfate, then filtered, and the filtrates were concentrated under reduced pressure. The concentrated residue obtained was subjected to flash silica gel column chromatography using 15% ethyl acetate/hexane as eluent. The resulting fractions were concentrated under reduced pressure to give the title compound (2.30 g, 6.61 mmol, 95%) as a solid.
¹H-NMR (400 MHz, CDCl₃) δ: 2.25 (3H, s), 2.66 (1H, d, J=8.9Hz), 5.59 (1H, d, J=8.9Hz), 6.81-6.90 (1H, m), 7.05-7.15 (1H, m), 7.25-7.28 (1H, m), 8.73 (1H, s).

### Example 1: 2-Bromo-4-methyl-5-[(2,3,6-trifluorophenyl)[(3,3,3-trifluoropropyl)thio]methyl]pyridine

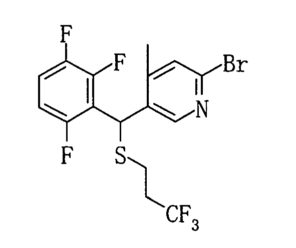

Sodium hydride (60 mg, 1.38 mmol) and 1,1,1-trifluoro-3-iodopropane (165 µ1, 1.38 mmol) were added to a N,N-dimethylformamide (5 ml) solution of (6-bromo-4-methylpyridin-3-yl)(2,3,6-trifluorophenyl)methanethiol (400 mg, 1.15 mmol) at 0°C, and the solution was stirred at room temperature for 2 hours. Water was added to the reaction solution, followed by extraction with ethyl acetate. The organic layer was washed sequentially with water and saturated saline. The resulting organic layer was dried over anhydrous magnesium sulfate, and then concentrated under reduced pressure. The resulting residue was subjected to flash silica gel column chromatography (hexane/ethyl acetate) to give the title compound (390 mg, 0.878 mmol, 76%) as a yellow oil.
¹H-NMR (400 MHz, CDCl₃) δ: 2.23 (3H, s), 2.29-2.46 (2H, m), 2.65-2.77 (2H, m), 5.48 (1H, s), 6.84-6.90 (1H, m), 7.08-7.16 (1H, m), 7.28 (1H, s), 8.79 (1H, s).
MS (m/z) : 444 (M⁺+H).

### Example 2: Methyl 4-methyl-5-[(2,3,6-trifluorophenyl)[(3,3,3-trifluoropropyl)thio]methyl]pyridine-2-carboxylate

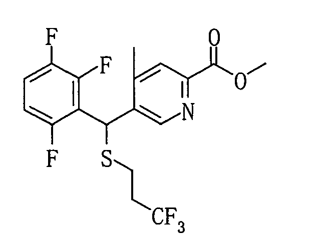

Under argon atmosphere, n-butyl lithium (1.59 M hexane solution, 0.592 ml, 0.941 mmol) was added to a toluene (8 ml) solution of 2-bromo-4-methyl-5-[(2,3,6-trifluorophenyl)[(3,3,3-trifluoropropyl)thio]methyl]pyridine (380 mg, 0.855 mmol) at - 76°C. The temperature of the reaction mixture was raised to - 40°C, and the mixture was stirred for 30 minutes. Then, the mixture was cooled again to -75°C, and the system was replaced with carbon dioxide gas. Subsequently, the mixture was stirred for 1 hour whilst raising to room temperature. 1N hydrochloric acid (3 ml) and water were added to the reaction solution, followed by extraction with ethyl acetate. The organic layer was washed with saturated saline, dried over anhydrous magnesium sulfate, and then concentrated under reduced pressure.

Under nitrogen atmosphere, trimethylsilyldiazomethane (2.0 M hexane solution, 0.855 ml, 1.71 mmol) was added to the solution of the resulting residue in a combined solvent of diethyl ether (6 ml) and methanol (1 ml) under ice-cold conditions. The mixture was stirred at room temperature for 1 hour. The residue obtained by concentrating the reaction solution under reduced pressure was subjected to flash silica gel column chromatography (hexane/ethyl acetate) to give the title compound (168 mg, 0.397 mmol, 46%) as a pale yellow oil.
¹H-NMR (400 MHz, CDCl₃) δ: 2.30-2.46 (2H, m), 2.33 (3H, s), 2.67-2.76 (2H, m), 4.01 (3H, s), 5.59 (1H, s), 6.84-6.90 (1H, m), 7.06-7.16 (1H, m), 7.93 (1H, s), 9.19 (1H, s).
MS (m/z): 424 (M⁺+H).

### Example 3: 4-Methyl-5-[(2,3,6-trifluorophenyl)[(3,3,3-trifluoropropyl)sulfonyl]methyl]pyridine-2-carboxamide

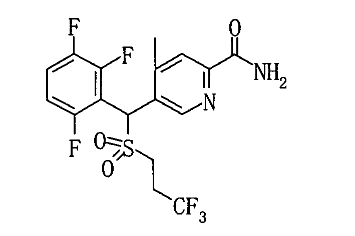

Hexaammonium heptamolybdate tetrahydrate (20 mg) and 30% aqueous hydrogen peroxide (5 ml) were added to a solution mixture of methyl 4-methyl-5-[(2,3,6-trifluorophenyl)[(3,3,3-trifluoropropyl)thio]methyl]pyridine-2-carboxylate (160 mg, 0.378 mmol) in a combined solvent of methanol (3 ml) and ethyl acetate (3 ml), and the mixture was stirred for 3 hours. Water was added to the reaction solution, followed by extraction with ethyl acetate. The organic layer was washed sequentially with saturated aqueous sodium hydrogen carbonate, saturated aqueous sodium thiosulfate and saturated saline. The resulting organic layer was dried over anhydrous magnesium sulfate, and then concentrated under reduced pressure.

Under nitrogen atmosphere, 7N ammonia methanol solution (5 ml) was added to the resulting residue, and the mixture was stirred at room temperature for 18 hours. The reaction solution was concentrated under reduced pressure, and the resulting residue was subjected to flash silica gel column chromatography (hexane/ethyl acetate) to give the title compound (58 mg, 0.132 mmol, 35%) as a white powder.
¹H-NMR (400 MHz, CDCl₃) δ: 2.39 (3H, s), 2.61-2.79 (2H, m), 3.18-3.36 (2H, m), 5.56 (1H, br s), 5.94 (1H, s), 6.98-7.05 (1H, m), 7.23-7.34 (1H, m), 7.84 (1H, br s), 8.09 (1H, s), 9.31 (1H, s).
IR (ATR) cm⁻¹: 3434, 3183, 1683, 1637, 1600, 1552, 1498, 1446, 1421, 1384, 1342, 1301, 1278, 1245, 1214, 1155, 1143, 1089, 1037.
mp: 190-191°C.
MS (m/z) : 441 (M⁺+H).
Elemental analysis: C₁₇H₁₄F₆N₂O₃S: Theoretical value: C 46.37; H 3.20; F 25.89; N 6.36; S 7.28. Observed value: C 46.25; H 3.02; F 25.99; N 6.22; S 7.38.

### Example 4: N-(Hydroxymethyl)-4-methyl-5-[(2,3,6-trifluorophenyl)[(3,3,3-trifluoropropyl)sulfonyl]methyl]pyridine-2-carboxamide

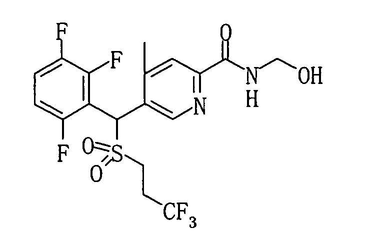

37% Aqueous formaldehyde (116 µl) and 1N aqueous sodium hydroxide (48 µl, 0.048 mmol) were added to an ethylene glycol dimethyl ether (5 ml) solution of 4-methyl-5-[(2,3,6-trifluorophenyl)[(3,3,3-trifluoropropyl)sulfonyl]methyl]pyridine-2-carboxamide (210 mg, 0.477 mmol), and the mixture was stirred at room temperature for 3 hours. Water was added to the reaction solution, followed by extraction with ethyl acetate. The organic layer was washed with saturated saline. The resulting organic layer was dried over anhydrous magnesium sulfate, and then concentrated under reduced pressure. The resulting residue was subjected to flash silica gel column chromatography (hexane/ethyl acetate) to give the title compound (145 mg, 0.308 mmol, 65%) as a white powder.
¹H-NMR (400 MHz, CDCl₃) δ: 2.39 (3H, s), 2.59-2.78 (2H, m), 3.11-3.35 (3H, m), 4.99-5.03 (2H, m), 5.94 (1H, s), 6.99-7.05 (1H, m), 7.24-7.33 (1H, m), 8.07 (1H, s), 8.86 (1H, br s), 9.30 (1H, s).
IR (ATR) cm⁻¹: 3295, 1658, 1602, 1513, 1496, 1446, 1382, 1340, 1303, 1272, 1251, 1211, 1145, 10951045.
mp: 146-148°C.
MS (m/z): 471 (M⁺+H).
Elemental analysis: C₁₈H₁₆F₆N₂O₄S: Theoretical value: C 45.96; H 3.43; F 24.23; N 5.96; S 6.82. Observed value: C 46.01; H 3.56; F 23.96; N 6.00; S 6.92.

### Example 5: 2-Bromo-4-methyl-5-[(2,3,6-trifluorophenyl)[(4,4,4-trifluorobutyl)thio]methyl]pyridine

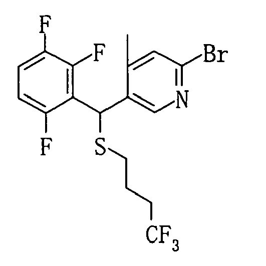

Sodium hydride (60 mg, 1.38 mmol) and 1,1,1-trifluoro-4-iodobutane (424 µl, 1.38 mmol) were added at 0°C to a N,N-dimethylformamide (5 ml) solution of (6-bromo-4-methylpyridin-3-yl)(2,3,6-trifluorophenyl)methanethiol (400 mg, 1.15 mmol) obtained in Reference Example 2, and the mixture was stirred at room temperature for 6 hours. Water was added to the reaction solution, followed by extraction with ethyl acetate. The organic layer was washed sequentially with water and saturated saline. The resulting organic layer was dried over magnesium sulfate, and then concentrated under reduced pressure. The resulting residue was subjected to flash silica gel column chromatography (hexane/ethyl acetate) to give the title compound (360 mg, 0.786 mmol, 68%) as a pale yellow oil.
¹H-NMR (400 MHz, CDCl₃) δ: 1.81-1.89 (2H, m), 2.14-2.24 (2H, m), 2.20 (3H, s), 2.57-2.64 (2H, m), 5.45 (1H, s), 6.82-6.88 (1H, m), 7.06-7.14 (1H, m), 7.27 (1H, s), 8.81 (1H, s).
MS (m/z): 458 (M⁺+H).

### Example 6: Methyl 4-methyl-5-[(2,3,6-trifluorophenyl)[(4,4,4-trifluorobutyl)thio]methyl]pyridine-2-carboxylate

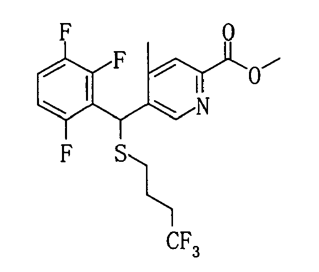

Under argon atmosphere, n-butyl lithium (1.59 M hexane solution, 0.528 ml, 0.840 mmol) was added to a toluene (6 ml) solution of 2-bromo-4-methyl-5-[(2,3,6-trifluorophenyl)[(4,4,4-trifluorobutyl)thio]methyl]pyridine (350 mg, 0.764 mmol) at - 75°C. The temperature of the reaction mixture was raised to - 40°C, and the mixture was stirred for 30 minutes. Then, the mixture was cooled again to -78°C, and the system was replaced with carbon dioxide gas. Subsequently, the mixture was stirred for 30 minutes whilst raising the temperature to room temperature. 1N hydrochloric acid (3 ml) and water were added to the reaction solution, followed by extraction with ethyl acetate. The organic layer was washed with saturated saline, dried over anhydrous magnesium sulfate, and then concentrated under reduced pressure.

Under nitrogen atmosphere, trimethylsilyldiazomethane (2.0 M hexane solution, 0.764 ml, 1.53 mmol) was added to the solution of the resulting residue in a combined solvent of diethyl ether (6 ml) and methanol (1 ml) under ice-cold conditions. The mixture was stirred at room temperature for 2 hours. The residue obtained after concentrating the reaction solution under reduced pressure was subjected to flash silica gel column chromatography (hexane/ethyl acetate) to give the title compound (168 mg, 0.384 mmol, 50%) as a brown oil.
¹H-NMR (400 MHz, CDCl₃) δ: 1.81-1.89 (2H, m), 2.13-2.24 (2H, m), 2.32 (3H, s), 2.59-2.66 (2H, m), 4.00 (3H, s), 5.56 (1H, s), 6.82-6.88 (1H, m), 7.05-7.15 (1H, m), 7.91 (1H, s), 9.21 (1H, s).
MS (m/z): 438 (M⁺+H).

### Example 7: 4-Methyl-5-[(2,3,6-trifluorophenyl)[(4,4,4-trifluorobutyl)sulfonyl]methyl]pyridine-2-carboxamide

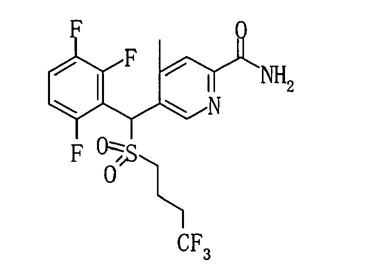

Hexaammonium heptamolybdate tetrahydrate (20 mg) and 30% aqueous hydrogen peroxide (3 ml) were added to a solution of methyl 4-methyl-5-[(2,3,6-trifluorophenyl)[(4,4,4-trifluorobutyl)thio]methyl]pyridine-2-carboxylate (160 mg, 0.366 mmol) in a combined solvent of methanol (3 ml) and ethyl acetate (3 ml), and the mixture was stirred for 1 hour. Water was added to the reaction solution, followed by extraction with ethyl acetate. The organic layer was washed sequentially with saturated aqueous sodium hydrogen carbonate, saturated aqueous sodium thiosulfate and saturated saline. The resulting organic layer was dried over anhydrous magnesium sulfate, and then concentrated under reduced pressure.

Under nitrogen atmosphere, 7N ammonia methanol solution (5 ml) was added to the resulting residue, and the mixture was stirred at room temperature for 18 hours. The reaction solution was concentrated under reduced pressure, and the resulting residue was subjected to flash silica gel column chromatography (hexane/ethyl acetate), followed by recrystallization with hexane/ethyl acetate to give the title compound (60 mg, 0.132 mmol, 36%) as a white powder.
¹H-NMR (400 MHz, CDCl₃) δ: 2.10-2.35 (4H, m), 2.38 (3H, s), 3.05-3.22 (2H, m), 5.55 (1H, br s), 5.91 (1H, s), 6.96-7.02 (1H, m), 7.23-7.33 (1H, m), 7.85 (1H, br s), 8.08 (1H, s), 9.32 (1H, s).
IR (ATR) cm⁻¹: 3419, 3154, 1697, 1635, 1602, 1494, 1444, 1419, 1340, 1313, 1263, 1247, 1238, 1197, 1143, 1135, 1083.
mp: 201-203°C.
MS (m/z) : 455 (M⁺+H).
Elemental analysis: C₁₈H₁₆F₆N₂O₃S: Theoretical value: C 47.58; H 3.55; F 25.09; N 6.17; S 7.06. Observed value: C 47.50; H 3.64; F 24.98; N 6.17; S 7.15.

### Reference Example 3: 2-Bromo-5-[[[3-[[t-butyl(dimethyl)silyl]oxy]propyl]thio](2,3,6-trifluorophenyl)methyl]-4-methylpyridine

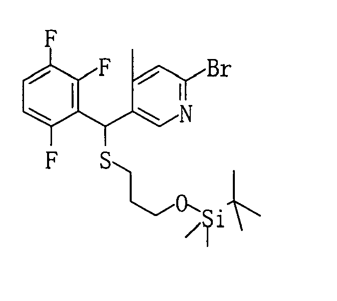

Sodium hydride (75 mg, 1.72 mmol) and t-butyl(3-bromopropoxy)dimethylsilane (75 mg, 1.72 mmol) were added at 0°C to a N,N-dimethylformamide (5 ml) solution of (6-bromo-4-methylpyridin-3-yl)(2,3,6-trifluorophenyl)methanethiol (500 mg, 1.44 mmol) obtained in Reference Example 2, and the mixture was stirred at room temperature for 40 minutes. Water was added to the reaction solution, followed by extraction with ethyl acetate. The organic layer was washed sequentially with water and saturated saline. The resulting organic layer was dried over anhydrous magnesium sulfate, and then concentrated under reduced pressure. The resulting residue was subjected to flash silica gel column chromatography (hexane/ethyl acetate) to give the title compound (629 mg, 1.21 mmol, 84%) as a pale yellow oil.
¹H-NMR (400 MHz, CDCl₃)δ: 0.01 (6H, s), 0.85 (9H, s), 1.73-1.82 (2H, m), 2.21 (3H, s), 2.56-2.65 (2H, m), 3.65 (2H, td, J=5.9, 2.4Hz), 5.44 (1H, s), 6.79-6.85 (1H, m), 7.03-7.11 (1H, m), 7.25 (1H, s), 8.83 (1H, s).
MS (m/z) : 520 (M⁺+H).

### Reference Example 4: Methyl 5-[[[3-[[t-butyl(dimethyl)silyl]oxy]propyl]thio](2,3,6-trifluorophenyl)methyl]-4-methylpyridine-2-carboxylate

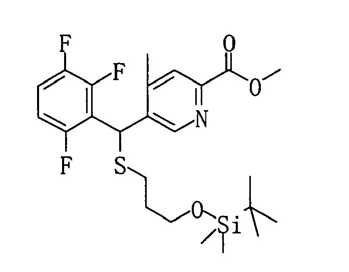

Under argon atmosphere, n-butyl lithium (1.59 M hexane solution, 1.6 ml, 2.54 mmol) was added to a toluene (20 ml) solution of 2-bromo-5-[[[3-[[t-butyl(dimethyl)silyl]oxy]propyl]thio](2,3,6-trifluorophenyl)methyl]-4-methylpyridine (1.2 g, 2.31 mmol) at - 78°C. The temperature of the reaction mixture was raised to - 40°C, and the mixture was stirred for 30 minutes. Then, the mixture was cooled again to -78°C, and the system was replaced with carbon dioxide gas. Subsequently, the mixture was stirred for 20 minutes whilst raising the temperature to room temperature. Saturated aqueous ammonium chloride was added to the reaction solution, followed by extraction with ethyl acetate. The organic layer was washed with saturated saline, dried over anhydrous magnesium sulfate, and then concentrated under reduced pressure.

Under nitrogen atmosphere, trimethylsilyldiazomethane (2.0 M hexane solution, 2.3 ml, 4.61 mmol) was added to the solution of the resulting residue in a combined solvent of diethyl ether (20 ml) and methanol (5 ml) under ice-cold conditions. The mixture was stirred at room temperature for 2 hours. The residue obtained after concentrating the reaction solution under reduced pressure was subjected to flash silica gel column chromatography (hexane/ethyl acetate) to give the title compound (270 mg, 0.540 mmol, 23%) as a pale yellow oil.
¹H-NMR (400 MHz, CDCl₃) δ: 0.01 (6H, s), 0.85 (9H, s), 1.74-1.82 (2H, m), 2.31 (3H, s), 2.59-2.66 (2H, m), 3.61-3.69 (2H, m), 4.00 (3H, s), 5.56 (1H, s), 6.79-6.85 (1H, m), 7.03-7.11 (1H, m), 7.90 (1H, s), 9.23 (1H, s).
MS (m/z) : 500 (M⁺+H).

### Reference Example 5: Methyl 5-[[(3-hydroxypropyl)thio](2,3,6-trifluorophenyl)methyl]-4-methylpyridine-2-carboxylate

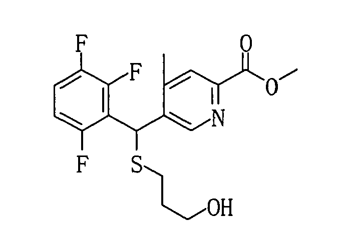

Tetrabutylammonium fluoride (1.0 M tetrahydrofuran solution, 0.8 ml, 0.78 mmol) was added to a tetrahydrofuran (5 ml) solution of methyl 5-[[[3-[[t-butyl(dimethyl)silyl]oxy]propyl]thio](2,3,6-trifluorophenyl)methyl]-4-methylpyridine-2-carboxylate (260 mg, 0.520 mmol) under ice-cold conditions, and the mixture was stirred at room temperature for 1.5 hours. Water was added to the reaction solution, followed by extraction with ethyl acetate. The organic layer was washed with saturated saline. The resulting organic layer was dried over anhydrous magnesium sulfate, and then concentrated under reduced pressure. The resulting residue was subjected to flash silica gel column chromatography (hexane/ethyl acetate) to give the title compound (118 mg, 0.306 mmol, 59%) as a pale yellow oil.
¹H-NMR (400 MHz, CDCl₃)δ: 1.37 (1H, t, J=5.2Hz), 1.82-1.88 (2H, m), 2.32 (3H, s), 2.63-2.73 (2H, m), 3.71-3.75 (2H, m), 4.08 (3H, s), 5.59 (1H, s), 6.80-6.86 (1H, m), 7.04-7.13 (1H, m), 7.90 (1H, s), 9.24 (1H, s).
MS (m/z): 386 (M⁺+H).

### Reference Example 6: 5-[[(3-Hydroxypropyl)sulfonyl](2,3,6-trifluorophenyl)methyl]-4-methylpyridine-2-carboxamide

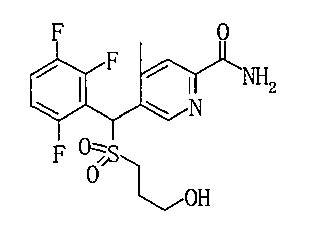

Hexaammonium heptamolybdate tetrahydrate (10 mg) and 30% aqueous hydrogen peroxide (3 ml) were added to a solution of methyl 5-[[(3-hydroxypropyl)thio](2,3,6-trifluorophenyl)methyl]-4-methylpyridine-2-carboxylate (110 mg, 0.285 mmol) in a combined solvent of methanol (3 ml) and ethyl acetate (3 ml), and the mixture was stirred for 4 hours. Water was added to the reaction solution, followed by extraction with ethyl acetate. The organic layer was washed sequentially with saturated aqueous sodium hydrogen carbonate, saturated aqueous sodium thiosulfate and saturated saline. The resulting organic layer was dried over anhydrous magnesium sulfate, and then concentrated under reduced pressure.

Under nitrogen atmosphere, 7N ammonia methanol solution (7 ml) was added to the resulting residue, and the mixture was stirred at room temperature for 18 hours. The reaction solution was concentrated under reduced pressure, and the resulting residue was subjected to flash silica gel column chromatography (hexane/ethyl acetate) to give the title compound (72 mg, 0.179 mmol, 63%) as a white powder.
¹H-NMR (400 MHz, CDCl₃) δ: 1.55 (1H, t, J=5.1Hz), 2.06-2.18 (2H, m), 2.38 (3H, s), 3.16-3.30 (2H, m), 3.77-3.81 (2H, m), 5.54 (1H, br s), 5.97 (1H, s), 6.94-7.00 (1H, m), 7.21-7.29 (1H, m), 7.85 (1H, br s), 8.06 (1H, s), 9.37 (1H, s).
IR (ATR) cm⁻¹: 3399, 3199, 1673, 1637, 1596, 1552, 1496, 1427, 1351, 1330, 1284, 1243, 1209, 1137, 1108, 1064, 1029.
mp: 148-150°C.
MS (m/z) : 403 (M⁺+H). Elemental analysis: C₁₇H₁₇F₃N₂O₄S: Theoretical value: C 50.74; H 4.26; F 14.16; N 6.96; S 7.97. Observed value: C 50.70; H 4.19; F 13.94; N 6.82; S 7.78.

### Example 8: 5-[[(3-Fluoropropyl)sulfonyl](2,3,6-trifluorophenyl)methyl]-4-methylpyridine-2-carboxamide

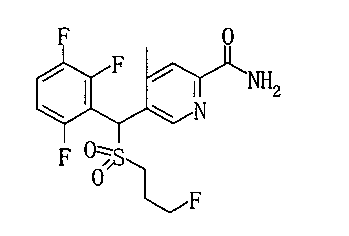

(Diethylamino)sulfur trifluoride (23 µl, 0.171 mmol) was added to a dichloromethane (3 ml) solution of 5-[[(3-hydroxypropyl)sulfonyl](2,3,6-trifluorophenyl)methyl]-4-methylpyridine-2-carboxamide (46 mg, 0.114 mmol), and the mixture was stirred for 20 minutes. The reaction solution was concentrated under reduced pressure, and the resulting residue was subjected to flash silica gel column chromatography (hexane/ethyl acetate) to give the title compound (27 mg, 0.0668 mmol, 59%) as a white powder.
¹H-NMR (400 MHz, CDCl₃) δ: 2.20-2.37 (2H, m), 2.38 (3H, s), 3.14-3.30 (2H, m), 4.56 (2H, dt, J=46.8, 5.5Hz), 5.57 (1H, br s), 5.93 (1H, s), 6.95-7.02 (1H, m), 7.23-7.31 (1H, m), 7.85 (1H, br s), 8.07 (1H, s), 9.35 (1H, s).
IR (ATR) cm⁻¹:3409, 1695, 1637, 1602, 1550, 1496, 1421, 1348, 1322, 1284, 1245, 1205, 1132, 1027.
mp: 194-196°C.
MS (m/z): 405 (M⁺+H).
TOF-ESI-MS: 405.0864 (Calculated value as C₁₇H₁₆F₄N₂O₃S: 405.0896).

### Reference Example 7: 2-Bromo-4-methyl-5-[[[2-(2-methyl-1,3-dioxolan-2-yl)ethyl]thio](2,3,6-trifluorophenyl)methyl]pyridine

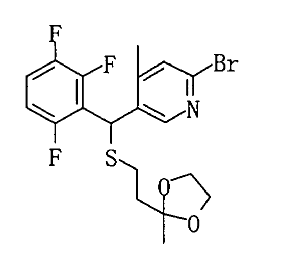

Under argon atmosphere, potassium carbonate (228 mg, 1.65 mmol) was added at 0°C to a N,N-dimethylformamide (8 ml) solution of (6-bromo-4-methylpyridin-3-yl)(2,3,6-trifluorophenyl)methanethiol (522 mg, 1.50 mmol) obtained in Reference Example 2 and 2-(2-bromomethyl)-2-methyl-1,3-dioxolane (0.227 ml, 1.65 mmol), and the mixture was stirred at room temperature for 6 hours. The reaction solution was cooled to 0°C, diluted with ethyl acetate, and then water was added to the reaction solution. The resulting mixture was extracted with ethyl acetate, and the extracts were washed with water and saturated saline. The extracts were dried over magnesium sulfate, and then concentrated. The resulting residue was subjected to flash silica gel column chromatography using hexane:ethyl acetate = 7:1 as the eluent. The resulting fractions were concentrated to give the title compound (494 mg, 1.07 mmol, 71%) as a colorless oil.
¹H-NMR (400 MHz, CDCl₃) δ: 1.28 (3H, s), 1.87-2.02 (2H, m), 2.22 (3H, s), 2.52-2.66 (2H, m), 3.84-3.96 (4H, m), 5.45 (1H, s), 6.82 (1H, tdd, J=9.3, 3.9, 2.2Hz), 7.07 (1H, ddd, J=18.1, 9.3, 4.9Hz), 7.25 (1H, s), 9.16 (1H, s).
MS (m/z) : 462 (M⁺+H).

### Reference Example 8: 4-Methyl-5-[[[2-(2-methyl-1,3-dioxolan-2-yl)ethyl]thio](2,3,6-trifluorophenyl)methyl]pyridine-2-carboxamide

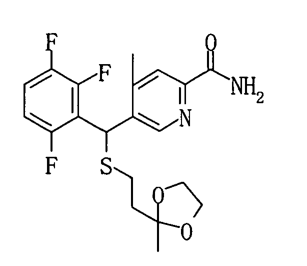

Under argon atmosphere, n-butyl lithium (1.60 M hexane solution, 0.794 ml, 1.27 mmol) was added dropwise to a toluene (15 ml) solution of 2-bromo-4-methyl-5-[[[2-(2-methyl-1,3-dioxolan-2-yl)ethyl]thio](2,3,6-trifluorophenyl)methyl]pyridine (492 mg, 1.06 mmol) at -78°C. The reaction solution was stirred at -40°C for 30 minutes, and then cooled to -78°C. Argon was replaced with carbon dioxide gas. Subsequently, the reaction solution was stirred at room temperature for 2 hours. The reaction solution was cooled to 0°C, and then 1N hydrochloric acid (1.5 ml) was added, followed by extraction with ethyl acetate. The extracts were combined, washed sequentially with water and saturated saline, then dried over magnesium sulfate and concentrated. The resulting residue was dissolved in tetrahydrofuran (5 ml), and then triethylamine (0.209 ml, 1.50 mmol) was added under argon atmosphere. The mixture was cooled to -5°C, and isobutyl chloroformate (0.170 ml, 1.30 mmol) was added dropwise. After the reaction solution was stirred for 15 minutes, ammonia (7N methanol solution, 0.700 ml) was added. The reaction solution was stirred at room temperature for 2 hours, cooled to 0°C, and then water was added. The resulting mixture was extracted with ethyl acetate, and the extracts were combined. The combined extracts were washed with water and saturated saline, then dried over magnesium sulfate, and concentrated. The resulting residue was subjected to flash silica gel column chromatography using hexane:ethyl acetate = 3:2 as the eluent. The resulting fractions were concentrated to give the title compound (290 mg, 0.681 mmol, 64%) as a colorless oil.
¹H-NMR (400 MHz, CDCl₃) δ: 1.29 (3H, s), 1.88-2.04 (1H, m), 2.33 (3H, s), 2.54-2.68 (2H, m), 3.83-3.96 (4H, m), 5.53-5.59 (2H, brm), 6.84 (1H, tdd, J=9.3, 3.9, 2.2Hz), 7.08 (1H, ddd, J=18.1, 9.3, 4.9Hz), 7.85 (1H, s), 7.97 (1H, s), 9.05 (1H, s).
MS (m/z): 427 (M⁺+H).

### Reference Example 9: 4-Methyl-5-[[(3-oxobutyl)thio](2,3,6-trifluorophenyl)methyl]pyridine-2-carboxamide

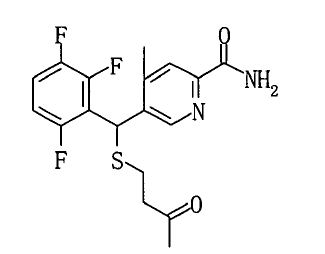

Water (2.0 ml) and concentrated hydrochloric acid (2.0 ml) were added to a 1,4-dioxane (8 ml) solution of 4-methyl-5-[[[2-(2-methyl-1,3-dioxolan-2-yl)ethyl]thio](2,3,6-trifluorophenyl)methyl]pyridine-2-carboxamide (339 mg, 0.796 mmol) at 0°C, and then the mixture was stirred at room temperature for 4 hours. The reaction solution was concentrated to about half volume, and then the resulting residue was extracted with ethyl acetate. The resulting extracts were washed with water and saturated saline, dried over magnesium sulfate, and then concentrated. The resulting residue was subjected to flash silica gel column chromatography using hexane:ethyl acetate = 1:2 as the eluent. The resulting fractions were concentrated to give the title compound (281 mg, 0.736 mmol, 92%) as a white amorphous.
¹H-NMR (400 MHz, CDCl₃) δ: 2.14 (3H, s), 2.33 (3H, s), 2.69-2.86 (4H, m), 5.56 (1H, s), 5.63 (1H, s), 6.84 (1H, tdd, J=9.3, 3.7, 2.2Hz), 7.09 (1H, ddd, J=18.1, 9.3, 4.9Hz), 7.84 (1H, s), 7.97 (1H, s), 9.04 (1H, s).
MS (m/z): 383 (M⁺+H).

### Example 9: 5-[[(3,3-Difluorobutyl)thio](2,3,6-trifluorophenyl)methyl]-4-methylpyridine-2-carboxamide

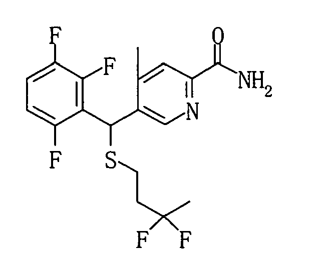

Under argon atmosphere, diethylamino sulfur trifluoride (1.50 ml, 11.4 mmol) was added to a dichloromethane (4 ml) solution of 4-methyl-5-[[(3-oxobutyl)thio](2,3,6-trifluorophenyl)methyl]pyridine-2-carboxamide (231 mg, 0.605 mmol) at 0°C, and then the mixture was stirred at room temperature for 4 hours. The reaction solution was diluted with dichloromethane, and then poured into ice-aqueous sodium hydrogen carbonate. The resulting mixture was extracted with dichloromethane, and then the extracts were washed with saturated aqueous sodium hydrogen carbonate, water and saturated saline. The extracts were dried over magnesium sulfate, and then concentrated. The resulting residue was subjected to flash silica gel column chromatography using hexane:ethyl acetate = 2:3 as the eluent. The resulting fractions were concentrated to give the title compound (75.4 mg, 0.187 mmol, 31%) as a yellow oil.
¹H-NMR (400 MHz, CDCl₃) δ: 1.59 (3H, t, J=18.6Hz), 2.03-2.25 (2H, m), 2.33 (3H, s), 2.63-2.76 (2H, m), 5.53-5.61 (2H, brm), 6.86 (1H, tdd, J=9.3, 3.7, 2.2Hz), 7.11 (1H, ddd, J=18.1, 9.3, 4.9Hz), 7.84 (1H, s), 7.98 (1H, s), 9.02 (1H, s).
MS (m/z): 405 (M⁺+H).

### Example 10: 5-[[(3,3-Difluorobutyl)sulfonyl](2,3,6-trifluorophenyl)methyl]-4-methylpyridine-2-carboxamide

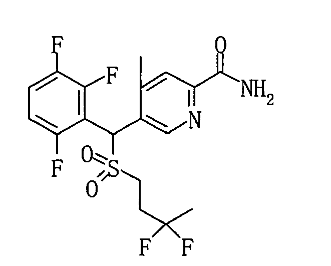

31% Aqueous hydrogen peroxide (1.0 ml) and hexaammonium heptamolybdate tetrahydrate (10.0 mg, 0.00809 mmol) were added to a methanol (3.0 ml) solution of 5-[[(3,3-difluorobutyl)thio](2,3,6-trifluorophenyl)methyl]-4-methylpyridine-2-carboxamide (75.0 mg, 0.186 mmol), and the mixture was stirred at room temperature for 3 days. Water was added to the reaction solution, followed by extraction with ethyl acetate. Then, the resulting extracts were washed sequentially with saturated aqueous sodium hydrogen carbonate, aqueous sodium thiosulfate, water and saturated saline. The extracts were dried over magnesium sulfate, and then concentrated. The resulting residue was subjected to flash silica gel column chromatography using hexane:ethyl acetate = 2:3 as the eluent. The resulting fractions were concentrated. The resulting solid was recrystallized from 2-propanol to give the title compound (57.8 mg, 0.133 mmol, 72%) as a white solid. ¹H-NMR (400 MHz, CDCl₃)δ: 1.68 (3H, t, J=18.3Hz), 2.30-2.55 (5H, m), 3.18-3.36 (2H, m), 5.56 (1H, s), 5.94 (1H, s), 6.99 (1H, tdd, J=9.3, 3.9, 2.2Hz), 7.23-7.32 (1H, m), 7.86 (1H, s), 8.08 (1H, s), 9.34 (1H, s).
IR (ATR) cm⁻¹: 3417, 1682, 1599, 1498, 1425, 1236, 1140, 1093, 895, 823, 729, 623, 598, 515, 482, 437.
mp: 165.0-166.0°C.
MS (m/z) : 437 (M⁺+H).
Elemental analysis: C₁₈H₁₇F₅N₂O₃S: Theoretical value: C 49.54; H 3.93; F 21.77; N 6.42; S 7.35. Observed value: C 49.70; H 3.88; F 21.84; N 6.29; S 7.35.

### Example 11: 5-[[(3,3-Difluorobutyl)sulfonyl](2,3,6-trifluorophenyl)methyl]-N-(hydroxymethyl)-4-methylpyridine-2-carboxamide

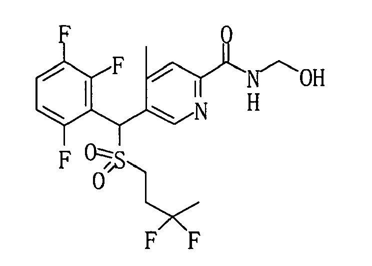

Aqueous formaldehyde (37%, 0.107 ml) and 1N aqueous sodium hydroxide (0.022ml) were added to a 1,2-dimethoxyethane (5 ml) solution of 5-[[(3,3-difluorobutyl)sulfonyl](2,3,6-trifluorophenyl)methyl]-4-methylpyridine-2-carboxamide (192 mg, 0.440 mmol), and the mixture was stirred at room temperature for 2 hours. Ethyl acetate was added to the reaction solution, and then the mixture was washed with 0.5 N hydrochloric acid, water and saturated saline. The mixture was dried over magnesium sulfate, and then concentrated. The resulting residue was subjected to flash silica gel column chromatography using hexane:ethyl acetate = 2:3 as the eluent. The resulting fractions were concentrated. The resulting solid was recrystallized from 2-propanol to give the title compound (120 mg, 0.258 mmol, 59%) as a white solid.
¹H-NMR (400 MHz, CDCl₃) δ: 1.68 (3H, t, J=18.4Hz), 2.32-2.55 (2H, m), 2.39 (3H, s), 3.09 (1H, t, J=7.8Hz), 3.18-3.35 (2H, m), 5.01 (2H, t, J=7.8Hz), 5.93 (1H, s), 6.96-7.03 (1H, m), 7.23-7.33 (1H, m), 8.06 (1H, s), 8.83-8.91 (1H, brm), 9.34 (1H, s). IR (ATR) cm⁻¹: 3336, 1672, 1541, 1498, 1306, 1254, 1140, 1093, 1047, 991, 893, 833, 721, 563, 515, 496, 480, 434.
mp: 161.0-161.5°C.
MS (m/z): 467 (M⁺+H).
Elemental analysis: C₁₉H₁₉F₅N₂O₄S: Theoretical value: C 48.93; H 4.11; F 20.37; N 6.01; S 6.87. Observed value: C 48.96; H 4.03; F 20.17; N 6.08; S 7.02.

### Example 12: 4-Methyl-5-[(propylsulfonyl)(2,3,6-trifluorophenyl)methyl]pyridine-2-carboxamide

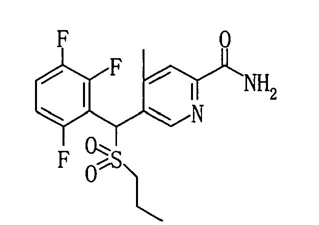

Under nitrogen atmosphere, sodium propane sulfinate (209 mg, 1.603 mmol) was added to a N,N-dimethylformamide (5 ml) solution of [6-(aminocarbonyl)-4-methylpyridin-3-yl](2,3,6-trifluorophenyl)methyl methanesulfonate (200 mg, 0.534 mmol), and the mixture was stirred at 80°C for 3 hours. Water was added to the reaction solution, followed by extraction with ethyl acetate. The resulting organic layer was washed sequentially with water and saturated saline. The organic layer was dried over anhydrous magnesium sulfate and then concentrated. The resulting residue was subjected to flash silica gel chromatography (hexane/ethyl acetate) to give the title compound (130 mg, 0.336 mmol, 63%) as a white powder.
¹H-NMR (400 MHz, CDCl₃) δ: 1.07 (3H, t, J=7.4Hz), 1.82-2.01 (2H, m), 2.37 (3H, s), 2.95-3.10 (2H, m), 5.56 (1H, br s), 5.89 (1H, s), 6.94-7.00 (1H, m), 7.21-7.29 (1H, m), 7.85 (1H, br s), 8.06 (1H, s), 9.37 (1H, s).
IR (ATR) cm⁻¹: 3413, 3149, 1693, 1637, 1602, 1550, 1494, 1419, 1382, 1348, 1319, 1245, 1205, 1132, 1083, 1016.
mp: 202-204°C.
MS (m/z) : 387 (M⁺+H).
Elemental analysis: C₁₇H₁₇F₃N₂O₃S: Theoretical value: C 52.84; H 4.43; F 14.75; N 7.25; S 8.30. Observed value: C 52.80; H 4.32; F 14.81; N 7.20; S 8.29.

### Reference Example 10: 5-[Chloro(2,3,6-trifluorophenyl)methyl]-4-methylpyridine-2-carboxamide

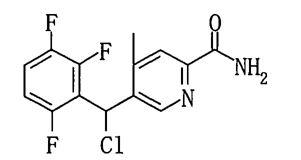

Under argon atmosphere, n-butyl lithium (1.59 M in hexane, 10 ml, 15.7 mmol) was added dropwise over 10 minutes to a toluene (150 ml) solution of 2-bromo-5-[chloro(2,3,6-trifluorophenyl)methyl]-4-methylpyridine (5.0 g, 14.3 mmol) at - 72°C. The temperature of the reaction mixture was raised to - 40°C over 30 minutes. The reaction mixture was then stirred for 10 minutes and cooled again to -72°C. The system was replaced with carbon dioxide gas. The mixture was stirred for 30 minutes whilst raising the temperature to 0°C. 1N hydrochloric acid (17 ml) and water were added to the reaction solution, followed by extraction with dichloromethane. The organic layer was dried over anhydrous magnesium sulfate, and then concentrated under reduced pressure.

Under nitrogen atmosphere, at -7°C, triethylamine (4.0 ml, 28.5 mmol) and butyl chloroformate (2.8 ml, 21.4 mmol) were added to a tetrahydrofuran (100 ml) solution of the resulting residue, and the mixture was stirred for 30 minutes. 7N ammonia methanol solution (15 ml) was added to the reaction solution, and the mixture was stirred at room temperature for 1 hour. The reaction solution was concentrated under reduced pressure. Water was added to the residue, followed by extraction with ethyl acetate. The organic layer was washed sequentially with saturated aqueous ammonium chloride and saturated saline, and then dried over anhydrous magnesium sulfate. The solution was then concentrated under reduced pressure. The resulting residue was washed with 2-propanol-hexane to give the title compound (1.5 g). The filtrates were subjected to flash silica gel column chromatography (hexane/ethyl acetate) to give the title compound (800 mg). The two products were combined to give the title compound (2.3 g, 7.31 mmol, 51%) as a pale yellow powder.
¹H-NMR (400 MHz, CDCl₃) δ: 2.33 (3H, s), 5.57 (1H, br s), 6.56 (1H, s), 6.85-6.93 (1H, m), 7.15-7.23 (1H, m), 7.85 (1H, br s), 7.99 (1H, s), 9.06 (1H, s).
MS (m/z) : 315 (M⁺+H).

### Reference Example 11: 5-[Mercapto(2,3,6-trifluorophenyl)methyl]-4-methylpyridine-2-carboxamide

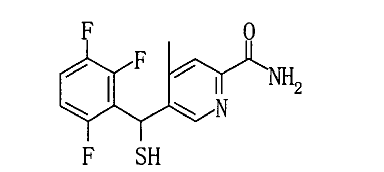

S-potassium thioacetate (17.2 g, 151 mmol) was added to an acetone (1 L) solution of 5-[chloro(2,3,6-trifluorophenyl)methyl]-4-methylpyridine-2-carboxamide (39.5 g, 126 mmol), and the mixture was stirred at 50°C for 16 hours. The reaction solution was filtered, and then concentrated under reduced pressure. Water was added to the resulting residue, followed by extraction with dichloromethane. The organic layer was dried over anhydrous magnesium sulfate, and then the solution was concentrated under reduced pressure.

1N aqueous sodium hydroxide (152 ml, 152 mmol) was added to an ethanol (1 1) solution of the resulting residue under ice-cold conditions, and the mixture was stirred at room temperature for 40 minutes. 1N hydrochloric acid (160 ml) was added to the reaction solution, and the mixture was concentrated under reduced pressure. Water (200 ml) was added to the resulting residue, followed by extraction with dichloromethane. The organic layer was dried over anhydrous magnesium sulfate, and then the solution was concentrated under reduced pressure. The resulting residue was washed with dichloromethane/hexane to give the title compound (33.5 g). The filtrates were subjected to flash silica gel column chromatography (hexane/ethyl acetate) to give the title compound (1.0 g). The two products were combined to give the title compound (34.5 g, 110 mmol, 88%) as a white powder.
¹H-NMR (400 MHz, CDCl₃) δ: 2.35 (3H, s), 2.68 (1H, d, J=8.8Hz), 5.55 (1H, br s), 5.71 (1H, d, J=8.8Hz), 6.83-6.90 (1H, m), 7.07-7.15 (1H, m), 7.82 (1H, br s), 7.97 (1H, s), 8.96 (1H, s).
MS (m/z): 313 (M⁺+H).

### Example 13: 5-[[(3,3-Dimethylbutyl)sulfonyl](2,3,6-trifluorophenyl)methyl]-4-methylpyridine-2-carboxamide

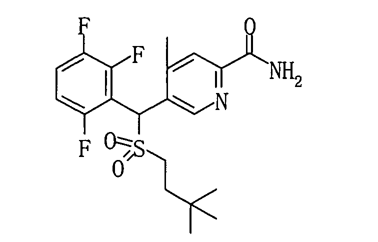

3,3-Dibutyl-4-methylbenzene sulfonate (296 mg, 1.15 mmol) and potassium carbonate (159 mg, 1.15 mmol) were added to a N,N-dimethylformamide (5 ml) solution of 5-[mercapto(2,3,6-trifluorophenyl)methyl]-4-methylpyridine-2-carboxamide (300 mg, 0.961 mmol) under ice-cold conditions, and the mixture was stirred at room temperature for 3 hours. Water was added to the reaction solution, followed by extraction with ethyl acetate, and the organic layer was washed sequentially with water and saturated saline. The resulting organic layer was dried over anhydrous magnesium sulfate, and then concentrated under reduced pressure.

Hexaammonium heptamolybdate tetrahydrate (30 mg) and 30% aqueous hydrogen peroxide (4 ml) were added to a solution of the resulting residue in a combined solvent of methanol (4 ml) and ethyl acetate (4 ml), and the mixture was stirred for 16 hours. Water was added to the reaction solution, followed by extraction with ethyl acetate, and then the extracts were washed sequentially with saturated aqueous sodium hydrogen carbonate, saturated aqueous sodium thiosulfate and saturated saline. The resulting organic layer was dried over magnesium sulfate, and then concentrated under reduced pressure. The resulting residue was subjected to flash silica gel column chromatography (hexane/ethyl acetate). The fractions obtained from an eluent portion were concentrated under reduced pressure to give the title compound (24 mg, 0.0560 mmol, 6%) as a white powder.
¹H-NMR (400 MHz, CDCl₃) δ: 0.88 (9H, s), 1.63 (1H, td, J=12.8, 4.7Hz), 1.80 (1H, td, J=12.8, 4.7Hz), 2.38 (3H, s), 2.95-3.11 (2H, m), 5.58 (1H, br s), 5.91 (1H, s), 6.94-7.00 (1H, m), 7.21-7.29 (1H, m), 7.86 (1H, br s), 8.06 (1H, s), 9.38 (1H, s).
IR (ATR) cm⁻¹: 3421, 3145, 2950, 1693, 1633, 1600, 1550, 1492, 1421, 1348, 1309, 1245, 1203, 1132, 1089.
mp: 143-144°C.
MS (m/z) : 429 (M⁺+H).
TOF-ESI-MS: 429.1440 (Calculated value as C₂₀H₂₄F₃N₂O₃S: 429.1460).

### Example 14: 5-[[(Cyclopropylmethyl)sulfonyl](2,3,6-trifluorophenyl)methyl]-4-methylpyridine-2-carboxamide

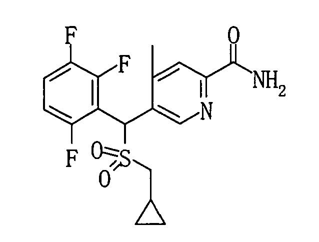

Under nitrogen atmosphere, (bromomethyl)cyclopropane (101 mg, 0.75 mmol) and potassium carbonate (104 mg, 0.75 mmol) were added at room temperature to a N,N-dimethylformamide (5 ml) solution of 5-[mercapto(2,3,6-trifluorophenyl)methyl]-4-methylpyridine-2-carboxamide (156 mg, 0.50 mmol) obtained in Reference Example 11, and the mixture was stirred for 2 hours. Ethyl acetate was added to the reaction mixture, and then the mixture was washed with water and then with saturated saline. The resulting organic layer was dried over anhydrous sodium sulfate, filtered, and then the filtrates were concentrated under reduced pressure.

The resulting residue was dissolved in methanol (6 ml), followed by addition of 31% aqueous hydrogen peroxide (3 ml) and hexaammonium heptamolybdate tetrahydrate (62 mg, 0.05 mmol), and then the mixture was stirred at room temperature for 20 hours. Ethyl acetate and water were added to the reaction mixture. Subsequently, ethyl acetate and methanol were distilled off under reduced pressure, then ethyl acetate was added to the residue, and the mixture was washed with saturated aqueous sodium hydrogen carbonate. The resulting organic layer was washed with 10% aqueous sodium thiosulfate and then with saturated saline. Then the organic layer was dried over anhydrous sodium sulfate and filtered, and then the filtrates were concentrated under reduced pressure. The resulting residue was subjected to flash silica gel chromatography using hexane:ethyl acetate = 1:1 as the eluent. The resulting fractions were concentrated under reduced pressure. The resulting residue was washed with ethanol, and then filtered to give the title compound (131 mg, 0.33 mmol, 66%) as a white solid.
¹H-NMR (400 MHz, CDCl₃) δ: 0.13-0.26 (1H, m), 0.34-0.44 (1H, m), 0.68-0.80 (2H, m), 1.11-1.23 (1H, m), 2.37 (3H, s), 2.93 (1H, dd, J=14.2, 8.1Hz) 3.16 (1H, dd, J=14.2, 6.4Hz), 5.58 (1H, br s), 6.03 (1H, s), 6.92-7.00 (1H, m), 7.20-7.29 (1H, m), 7.86 (1H, br s), 8.07 (1H, s), 9.46 (1H, t, J=2.2Hz).
IR (ATR) cm⁻¹: 3413, 3153, 1693, 1603, 1495, 1421, 1348, 1319, 1246, 1134.
MS (m/z): 399 (M⁺+H).
mp: 222-223°C.
Elemental analysis: C₁₈H₁₇F₃N₂O₃S: Theoretical value: C 54.27; H 4.30; F 14.31; N 7.03; S 8.05. Observed value: C 54.77; H 4.21; F 14.48; N 7.15; S 8.22.

### Example 15: 5-[(Butylsulfonyl)(2,3,6-trifluorophyenyl)methyl]-4-methylpyridine-2-carboxamide

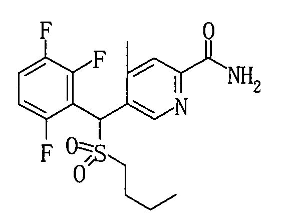

Under nitrogen atmosphere, 1-bromobutane (103 mg, 0.75 mmol) and potassium carbonate (104 mg, 0.75 mmol) were added at room temperature to a N,N-dimethylformamide (5 ml) solution of 5-[mercapto(2,3,6-trifluorophyenyl)methyl]-4-methylpyridine-2-carboxamide (156 mg, 0.50 mmol) obtained in Reference Example 11, and the mixture was stirred for 2 hours. Ethyl acetate was added to the reaction mixture, and the mixture was washed with water and then with saturated saline. The resulting organic layer was dried over anhydrous sodium sulfate, filtered, and then the filtrates were concentrated under reduced pressure.

The resulting residue was dissolved in methanol (6 ml), followed by addition of 31% aqueous hydrogen peroxide (3 ml) and hexaammonium heptamolybdate tetrahydrate (62 mg, 0.05 mmol), and then the mixture was stirred at room temperature for 20 hours. Ethyl acetate and water were added to the reaction mixture. Subsequently, ethyl acetate and methanol were distilled off under reduced pressure, and then ethyl acetate was added to the residue. The mixture was washed with saturated aqueous sodium hydrogen carbonate. The resulting organic layer was washed with 10% aqueous sodium thiosulfate and then with saturated saline. Then the organic layer was dried over anhydrous sodium sulfate and filtered, and then the filtrates were concentrated under reduced pressure. The resulting residue was subjected to flash silica gel chromatography using hexane:ethyl acetate = 1:1 as the eluent. The resulting fractions were concentrated under reduced pressure. The resulting residue was washed with ethanol and then filtered to give the title compound (146 mg, 0.36 mmol, 73%) as a white solid.
¹H-NMR (400 MHz, CDCl₃) δ: 0.93 (3H, t, J=7.4Hz), 1.39-1.50 (2H, m), 1.73-1.96 (2H, m), 2.37 (3H, s), 2.95-3.14 (2H, m), 5.56 (1H, br s), 5.89 (1H, s), 6.93-7.01 (1H, m), 7.21-7.30 (1H, m), 7.86 (1H, br s), 8.06 (1H, s), 9.37 (1H, t, J=2.0Hz).
IR (ATR) cm⁻¹: 3415, 3157, 1693, 1601, 1495, 1417, 1317, 1246, 1132, 987.
MS (m/z): 401 (M⁺+H).
mp: 203-204°C.
Elemental analysis: C₁₈H₁₉F₃N₂O₃S: Theoretical value: C 53.99; H 4.78; F 14.23; N 7.00; S 8.01. Observed value: C 53.82; H 4.69; F 14.52; N 6.94; S 8.22.

### Example 16: 5-[(Isobutylsulfonyl)(2,3,6-trifluorophenyl)methyl]-4-methylpyridine-2-carboxamide

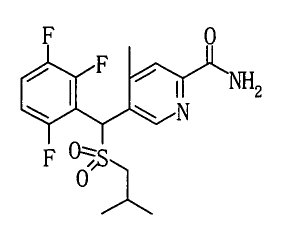

Under nitrogen atmosphere, 1-bromo-2-methylpropane (103 mg, 0.75 mmol) and potassium carbonate (104 mg, 0.75 mmol) were added at room temperature to a N,N-dimethylformamide (5 ml) solution of 5-[mercapto(2,3,6-trifluorophenyl)methyl]-4-methylpyridine-2-carboxamide (156 mg, 0.50 mmol) obtained in Reference Example 11, and the mixture was stirred for 4 hours. Ethyl acetate was added to the reaction mixture, and the mixture was washed with water and then with saturated saline. The resulting organic layer was dried over anhydrous sodium sulfate and filtered, and then the filtrates were concentrated under reduced pressure.

The resulting residue was dissolved in methanol (6 ml), followed by addition of 31% aqueous hydrogen peroxide (3 ml) and hexaammonium heptamolybdate tetrahydrate (62 mg, 0.05 mmol), and then the mixture was stirred at room temperature for 19 hours. Ethyl acetate and water were added to the reaction mixture. Subsequently, ethyl acetate and methanol were distilled off under reduced pressure, and then ethyl acetate was added to the residue. The mixture was washed with saturated aqueous sodium hydrogen carbonate. The resulting organic layer was washed with 10% aqueous sodium thiosulfate and then with saturated saline. Then the organic layer was dried over anhydrous sodium sulfate and filtered, and then the filtrates were concentrated under reduced pressure. The resulting residue was subjected to flash silica gel chromatography using hexane:ethyl acetate = 11:9 as the eluent. The resulting fractions were concentrated under reduced pressure. The resulting residue was washed with ethanol and then filtered to give the title compound (77 mg, 0.19 mmol, 38%) as a white solid.
¹H-NMR (400 MHz, CDCl₃) δ: 1.09 (3H, d, J=6.9Hz), 1.14 (3H, d, J=6.6Hz), 2.37 (3H, s), 2.38-2.48 (1H, m), 2.89 (1H, dd, J=13.5, 6.6Hz), 2.99 (1H, dd, J=13.5, 6.6Hz), 5.56 (1H, br s), 5.87 (1H, s), 6.93-7.01 (1H, m), 7.20-7.30 (1H, m), 7.86 (1H, br s), 8.06 (1H, s), 9.37 (1H, t, J=2.0Hz).
IR (ATR) cm⁻¹: 3417, 3157, 1693, 1603, 1495, 1421, 1344, 1304, 1134, 987.
MS (m/z): 401 (M⁺+H).
mp: 240-241°C.
Elemental analysis: C₁₈H₁₉F₃N₂O₃S: Theoretical value: C 53.99; H 4.78; F 14.23; N 7.00; S 8.01. Observed value: C 53.90; H 4.76; F 13.97; N 7.01; S 7.89.

### Example 17: 5-[[(Cyclobutylmethyl)sulfonyl](2,3,6-trifluorophenyl)methyl]-4-methylpyridine-2-carboxamide

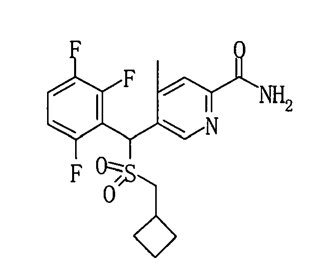

Under nitrogen atmosphere, (bromomethyl)cyclobutane (112 mg, 0.75 mmol) and potassium carbonate (104 mg, 0.75 mmol) was added at room temperature to a N,N-dimethylformamide (5 ml) solution of 5-[mercapto(2,3,6-trifluorophenyl)methyl]-4-methylpyridine-2-carboxamide (156 mg, 0.50 mmol) obtained in Reference Example 11, and the mixture was stirred for 4 hours. Ethyl acetate was added to the reaction mixture, and the mixture was washed with water and then with saturated saline. The resulting organic layer was dried over anhydrous sodium sulfate and filtered, and then the filtrates were concentrated under reduced pressure.

The resulting residue was dissolved in methanol (6 ml), followed by addition of 31% aqueous hydrogen peroxide (3 ml) and hexaammonium heptamolybdate tetrahydrate (62 mg, 0.05 mmol), and then the mixture was stirred at room temperature for 19 hours. Ethyl acetate and water were added to the reaction mixture. Subsequently, ethyl acetate and methanol were distilled off under reduced pressure, and then ethyl acetate was added to the residue. The mixture was washed with saturated aqueous sodium hydrogen carbonate. The resulting organic layer was washed with 10% aqueous sodium thiosulfate and then with saturated saline. Then the organic layer was dried over anhydrous sodium sulfate and filtered, and then the filtrates were concentrated under reduced pressure. The resulting residue was subjected to flash silica gel chromatography using hexane:ethyl acetate = 3:2 as the eluent. The resulting fractions were concentrated under reduced pressure. The resulting residue was washed with ethanol and then filtered to give the title compound (86 mg, 0.21 mmol, 42%) as a white solid.
¹H-NMR (400 MHz, CDCl₃) δ: 1.73-2.08 (4H, m), 2.16-2.29 (2H, m), 2.36 (3H, s), 2.88-3.01 (1H, m), 3.09-3.24 (2H, m), 5.58 (1H, br s), 5.82 (1H, s), 6.92-7.00 (1H, m), 7.20-7.30 (1H, m), 7.87 (1H, br s), 8.06 (1H, s), 9.36 (1H, t, J=2.0Hz).
IR (ATR) cm⁻¹: 3419, 3153, 1693, 1603, 1495, 1421, 1308, 1132, 985.
MS (m/z): 413 (M⁺+H).
mp: 214-215°C. Elemental analysis: C₁₉H₁₉F₃N₂O₃S: Theoretical value: C 55.33; H 4.64; F 13.82; N 6.79; S 7.77. Observed value: C 55.11; H 4.56; F 13.94; N 6.81; S 7.88.

### Example 18: 4-Methyl-5-[[(3,3,3-trichloropropyl)sulfonyl](2,3,6-trifluorophenyl)methyl]pyridine-2-carboxamide

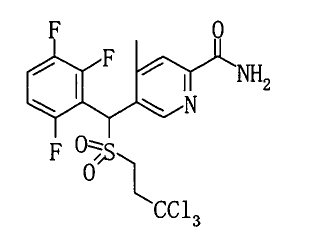

3-Bromo-1,1,1-trichloropropane (148 µl, 1.15 mmol) and potassium carbonate (159 mg, 1.15 mmol) were added to a N,N-dimethylformamide (7 ml) solution of 5-[mercapto(2,3,6-trifluorophenyl)methyl]-4-methylpyridine-2-carboxamide (300 mg, 0.961 mmol) obtained in Reference Example 11, and the mixture was stirred at room temperature for 1 hour. Water was added to the reaction solution, followed by extraction with ethyl acetate, and then the organic layer was washed sequentially with water and saturated saline. The resulting organic layer was dried over anhydrous magnesium sulfate, and then concentrated under reduced pressure.

Hexaammonium heptamolybdate tetrahydrate (30 mg) and 30% aqueous hydrogen peroxide (4 ml) were added to a solution of the resulting residue in a combined solvent of methanol (4 ml) and ethyl acetate (4 ml), and the mixture was stirred for 5 hours. Water was added to the reaction solution, followed by extraction with ethyl acetate, and then the extracts were washed sequentially with saturated aqueous sodium hydrogen carbonate, saturated aqueous sodium thiosulfate and saturated saline. The resulting organic layer was dried over magnesium sulfate, and then concentrated under reduced pressure. The resulting residue was subjected to flash silica gel column chromatography (hexane/ethyl acetate), to give the title compound (141 mg, 0.288 mmol, 30%) as a white powder.
¹H-NMR (400 MHz, CDCl₃) δ: 2.41 (3H, s), 3.14-3.31 (2H, m), 3.43-3.60 (2H, m), 5.57 (1H, br s), 5.97 (1H, s), 6.99-7.05 (1H, m), 7.23-7.34 (1H, m), 7.85 (1H, br s), 8.09 (1H, s), 9.34 (1H, s).
IR (ATR) cm⁻¹: 3453, 3297, 3239, 1698, 1666, 1581, 1548, 1490, 1417, 1322, 1257, 1241, 1199, 1168, 1132.
mp: 173-175°C.
MS (m/z) : 489 (M⁺+H).
Elemental analysis: C₁₇H₁₄Cl₃F₃N₂O₃S: Theoretical value: C 41.69; H 2.88; Cl 21.72; F 11.64; N 5.72; S 6.55. Observed value: C 41.83; H 2.80; Cl 21.22; F 11.44; N 5.70; S 6.54.

### Example 19: 4-Methyl-5-[[(3,3,4,4,4-pentafluorobutyl)sulfonyl](2,3,6-trifluorophenyl)methyl]pyridine-2-carboxamide

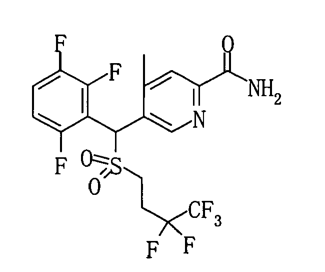

4-Iodo-1,1,1,2,2-pentafluorobutane (316 mg, 1.15 mmol) and potassium carbonate (159 mg, 1.15 mmol) were added at room temperature to a N,N-dimethylformamide (7 ml) solution of 5-[mercapto(2,3,6-trifluorophenyl)methyl]-4-methylpyridine-2-carboxamide (300 mg, 0.961 mmol) obtained in Reference Example 11, and the mixture was stirred for 1.5 hours. Water was added to the reaction solution, followed by extraction with ethyl acetate, and then the extracts were washed sequentially with water and saturated saline. The resulting organic layer was dried over anhydrous magnesium sulfate, and then concentrated under reduced pressure.

Hexaammonium heptamolybdate tetrahydrate (30 mg) and 30% aqueous hydrogen peroxide (5 ml) were added to a solution of the resulting residue in a combined solvent of methanol (5 ml) and ethyl acetate (5 ml), and the mixture was stirred for 6 hours. Water was added to the reaction solution, followed by extraction with ethyl acetate, and then the extracts were washed sequentially with saturated aqueous sodium hydrogen carbonate, saturated aqueous sodium thiosulfate and saturated saline. The resulting organic layer was dried over anhydrous magnesium sulfate, and then concentrated under reduced pressure. The resulting residue was subjected to flash silica gel column chromatography (hexane/ethyl acetate) to give the title compound (345 mg, 0.704 mmol, 72%) as a white powder.
¹H-NMR (400 MHz, CDCl₃) δ: 2.40 (3H, s), 2.53-2.73 (2H, m), 3.21-3.39 (2H, m), 5.58 (1H, br s), 5.96 (1H, s), 6.99-7.05 (1H, m), 7.25-7.34 (1H, m), 7.85 (1H, br s), 8.09 (1H, s), 9.31 (1H, s).
IR (ATR) cm⁻¹: 3413, 1697, 1635, 1602, 1550, 1494, 1419, 1324, 1253, 1203, 1184, 1137, 1124, 1058.
mp: 155-156°C.
MS (m/z): 491 (M⁺+H).
Elemental analysis: C₁₈H₁₄F₈N₂O₃S: Theoretical value: C 44.09; H 2.88; F 30.99; N 5.71; S 6.54. Observed value: C 44.22; H 2.81; F 30.81; N 5.71; S 6.10.

### Example 20: N-(Hydroxymethyl)-4-methyl-5-[[(3,3,4,4,4-pentafluorobutyl)sulfonyl](2,3,6-trifluorophenyl)methyl]pyridine-2-carboxamide

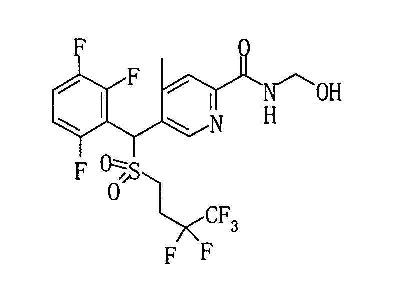

37% Aqueous formaldehyde (119 µl) and 1N aqueous sodium hydroxide (50 µl, 0.050 mmol) were added to an ethylene glycol dimethyl ether (7 ml) solution of 4-methyl-5-[[(3,3,4,4,4-pentafluorobutyl)sulfonyl](2,3,6-trifluorophenyl)methyl]pyridine-2-carboxamide (240 mg, 0.489 mmol), and the mixture was stirred at room temperature for 1.5 hours. Water was added to the reaction solution, followed by extraction with ethyl acetate, and then the organic layer was washed with saturated saline. The resulting organic layer was dried over anhydrous magnesium sulfate, and then concentrated under reduced pressure. The resulting residue was subjected to flash silica gel column chromatography (hexane/ethyl acetate) to give the title compound (214 mg, 0.411 mmol, 84%) as a white powder.
¹H-NMR (400 MHz, CDCl₃) δ: 2.40 (3H, s), 2.53-2.72 (2H, m), 3.14 (1H, t, J=7.7Hz), 3.21-3.39 (2H, m), 4.99-5.03 (2H, m), 5.96 (1H, s), 6.99-7.06 (1H, m), 7.24-7.34 (1H, m), 8.07 (1H, s), 8.86 (1H, br s), 9.30 (1H, s).
IR (ATR) cm⁻¹: 3340, 1652, 1602, 1515, 1498, 1444, 1421, 1334, 1303, 1249, 1193, 1141, 1093, 1058.
mp: 126-128°C.
MS (m/z): 521 (M⁺+H).
Elemental analysis: C₁₉H₁₆F₈N₂O₄S: Theoretical value: C 43.85; H 3.10; F 29.21; N 5.38; S 6.16. Observed value: C 43.60; H 3.01; F 29.08; N 5.17; S 6.19.

### Example 21: 4-Methyl-5-[(4,4-difluoro-3-butenylthio)(2,3,6-trifluorophenyl)methyl]pyridine-2-carboxamide

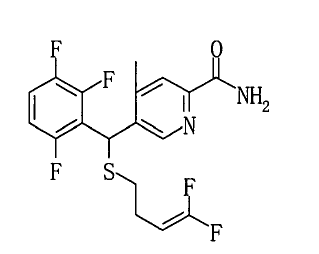

Potassium carbonate (83 mg, 0.6 mmol) was added to a N,N-dimethylformamide (3.0 ml) solution of 5-[mercapto(2,3,6-trifluorophenyl)methyl]-4-methylpyridine-2-carboxamide (156 mg, 0.5 mmol) obtained in Reference Example 11 and 4-bromo-1,1-difluoro-1-butene (94 mg, 0.55 mmol), and the mixture was stirred at room temperature for 2 hours. Water was added to the mixture, followed by extraction with ethyl acetate. The solution was washed with water and saturated saline, dried, and then concentrated under reduced pressure. The residue was purified by silica gel chromatography (hexane:ethyl acetate = 3:2) to give the title compound (132 mg, 0.328 mmol, 66%) as a needle-like crystal.
¹H-NMR (400 MHz, CDCl₃) δ: 9.05 (1H, s), 8.00 (1H, s), 7.90 (1H, br), 7.11 (1H, m), 6.86 (1H, m), 5.60 (1H, br), 5.59 (1H, s), 4.20 (1H, td, J=8.0, 24.8Hz), 2.60 (2H, m), 2.34 (3H, s), 2.32 (2H, m).
IR (ATR) cm⁻¹: 1749, 1693, 1492, 1245, 977, 829.
MS (m/z): 403 (M⁺+H).
mp: 97-98°C.

### Example 22: 4-Methyl-5-[(4,4-difluoro-3-butenylsulfonyl)(2,3,6-trifluorophenyl)methyl]pyridine-2-carboxamide

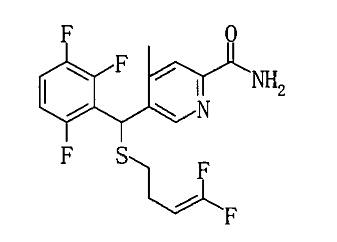

Methanol (6 ml) and 31% aqueous hydrogen peroxide (3 ml) were added to 4-methyl-5-[(4,4-difluoro-3-butenylthio)(2,3,6-trifluorophenyl)methyl]pyridine-2-carboxamide (129 mg, 0.32 mmol) and hexaammonium heptamolybdate tetrahydrate (30 mg), and the mixture was stirred at room temperature for 16 hours. Water was added to the mixture, followed by extraction with ethyl acetate. The extracts were washed with water, aqueous sodium thiosulfate and saturated saline, and then dried. The solution was concentrated under reduced pressure, and the resulting solid was washed with ethanol to give the title compound (108 mg, 0.249 mmol, 78%) as a white solid.
¹H-NMR (400 MHz, CDCl₃) δ: 9.34 (1H, s) ,8.08 (1H, s), 7.87 (1H, br), 7.28 (1H, m), 6.99 (1H, m), 5.91 (1H, s), 5.60 (1H, br), 4.26 (1H, tdd, J=7.8, 1.6, 24.2Hz), 3.2-3.0 (2H, m), 2.65-2.50 (2H, m), 2.34 (3H, s).
IR (ATR) cm⁻¹: 3430, 1693, 1493, 1417, 1246, 978, 517.
MS (m/z): 403 (M⁺+H).
mp: 179-181°C.
Elemental analysis: C₁₈H₁₅F₅N₂O₃S·0.25 H₂O: Theoretical value: C 49.26; H 3.56; N 6.38; S 7.31; F 21.64. Observed value: C 49.28; H 3.38; N 6.31; S 7.24; F 21.63.

### Example 23: 4-Methyl-5-[(3,4,4-trifluoro-3-butenylthio)(2,3,6-trifluorophenyl)methyl]pyridine-2-carboxamide

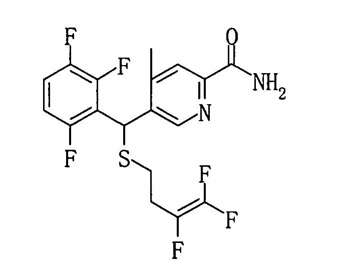

Potassium carbonate (83 mg, 0.6 mmol) was added to a dimethylformamide (3.0 ml) solution of 5-[mercapto(2,3,6-trifluorophenyl)methyl]-4-methylpyridine-2-carboxamide (156 mg, 0.5 mmol) obtained in Reference Example 11 and 4-bromo-1,1,2-trifluoro-1-butene (104 mg, 0.55 mmol), and the mixture was stirred at room temperature for 1 hour. The mixture was diluted with ethyl acetate, and then washed with water and saturated saline. The solution was dried, and then concentrated under reduced pressure. The residue was purified by silica gel chromatography (hexane:ethyl acetate = 2:1) to give the title compound (181 mg, 0.431 mmol, 86%) as an oil.
¹H-NMR (400 MHz, CDCl₃) δ: 9.03 (1H, s), 7.98 (1H, s), 7.86 (1H, br), 7.09 (1H, m), 6.85 (1H, m), 6.05 (1H, br), 5.60 (1H, s), 2.74 (2H, m), 2.57 (2H, m), 2.32 (3H, s).
MS (m/z) : 421 (M⁺+H).

### Example 24: 4-Methyl-5-[(3,4,4-trifluoro-3-butenylsulfonyl)(2,3,6-trifluorophenyl)methyl]pyridine-2-carboxamide

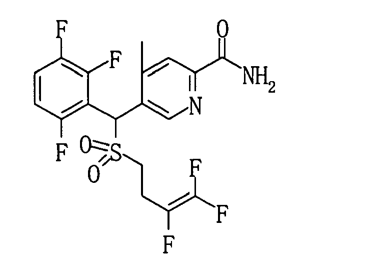

Methanol (6 ml) and 31% aqueous hydrogen peroxide (3 ml) were added to 4-methyl-5-[(3,4,4-trifluoro-3-butenylthio)(2,3,6-trifluorophenyl)methyl]pyridine-2-carboxamide (180 mg, 0.43 mmol) and hexaammonium heptamolybdate tetrahydrate (30 mg), and the mixture was stirred at room temperature for 20 hours. Water was added to the mixture, followed by extraction with ethyl acetate. The extracts were washed with water, aqueous sodium thiosulfate and saturated saline, and then dried. The solution was concentrated under reduced pressure, and the resulting residue was purified by silica gel chromatography (hexane:ethyl acetate = 2:1). The residue was further crystallized from ethanol to give the title compound (79 mg, 0.175 mmol, 40%) as a white solid.
¹H-NMR (400 MHz, CDCl₃) δ: 9.33 (1H, s), 8.08 (1H, s), 7.86 (1H, br), 7.28 (1H, m), 7.00 (1H, m), 5.93 (1H, s), 5.58 (1H, br), 3.35-3.2 (2H, m), 2.95-2.80 (2H, m), 2.38 (3H, s).
IR (ATR) cm⁻¹: 3407, 1803, 1697, 1493, 1236, 1140, 991, 817.
MS (m/z): 453 (M⁺+H).
mp: 170-172°C.
Elemental analysis: C₁₈H₁₄F₆N₂O₃S: Theoretical value: C 47.79; H 3.12; F 25.20; N 6.19; S 7.09. Observed value: C 47.82; H 3.05; F 25.23; N ,6.13; S 7.46.

### Example 25: 4-Methyl-5-[(2,2,3,3-tetrafluoropropylthio)(2,3,6-trifluorophenyl)methyl]pyridine-2-carboxamide

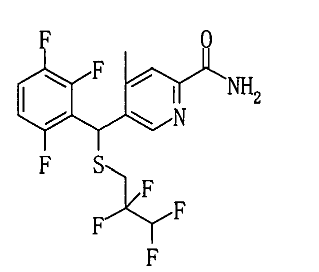

Lithium hydroxide monohydrate (71 mg, 1.7 mmol) dissolved in water (3 ml) was added to a tetrahydrofuran (6 ml) solution of 5-[mercapto(2,3,6-trifluorophenyl)methyl]-4-methylpyridine-2-carboxamide (467 mg, 1.5 mmol) obtained in Reference Example 11 and 3-iodo-1,1,2,2-tetrafluoropropane (363 mg, 1.5 mmol), and the mixture was stirred at room temperature for 4 hours. Aqueous ammonium chloride was added to the mixture, followed by extraction with ethyl acetate. The solution was washed with water and saturated saline, dried, and then concentrated under reduced pressure. The residue was purified by silica gel chromatography (hexane:ethyl acetate = 2:1) to give an approx. 3:1 oil mixture of the title compound and 4-methyl-5-[(2,3,6-trifluorophenyl)methyl]pyridine-2-carboxamide.
¹H-NMR (400 MHz, CDCl₃) δ: 9.30 (1H, s), 8.01 (1H, s), 7.88 (1H, s), 7.14 (1H, m), 6.90 (1H, m), 5.93 (1H, tt, J=3.2, 53.6Hz), 5.86 (1H, s), 3.10-2.97 (2H, m), 2.36 (3H, s).

### Example 26: 4-Methyl-5-[(2,2,3,3-tetrafluoropropylsulfonyl)(2,3,6-trifluorophenyl)methyl]pyridine-2-carboxamide

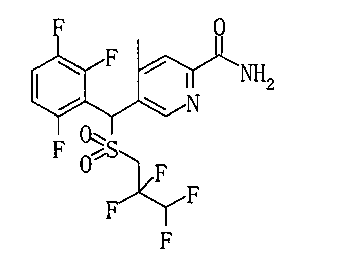

Metha-chloroperbenzoic acid (243 mg, 1.4 mmol) was added to a dichloromethane (4.0 ml) solution of a mixture containing 4-methyl-5-[(2,2,3,3-tetrafluoropropylthio)(2,3,6-trifluorophenyl)methyl]pyridine-2-carboxamide (300 mg), and the mixture was stirred at room temperature for 5 hours. Metha-chloroperbenzoic acid (40 mg) was further added, and the mixture was stirred for 17 hours. Aqueous sodium thiosulfate was added to the mixture, followed by extraction with ethyl acetate. The solution was washed with saturated aqueous sodium hydrogen carbonate and saturated saline, dried, and then concentrated under reduced pressure. The residue was purified by silica gel chromatography (hexane:ethyl acetate = 2:1) to give the title compound (93 mg, 0.203 mmol, 14%). The resulting product was crystallized from isopropanol-dichloromethane.
¹H-NMR (400 MHz, CDCl₃) δ: 9.33 (1H, s), 8.10 (1H, s), 7.85 (1H, s), 7.30 (1H, m), 7.01 (1H, m), 6.20 (1H, s), 6.06 (1H, tt, J=3.6, 53.2Hz), 5.62 (1H, s), 3.83-3.71 (2H, m), 2.39 (3H, s). IR (ATR) cm⁻¹: 1697, 1496, 1336, 1234, 1103, 831.
MS (m/z): 459 (M⁺+H).
mp: 160-161°C .
Elemental analysis: C₁₇H₁₃F₇N₂O₃S: Theoretical value: C 44.55; H 2.86; F 29.01; N 6.11; S 7.00. Observed value: C 44.81; H 2.74; F 28.74; N 6.23; S 6.96.

### Example 27: 4-Methyl-5-[(2,3,6-trifluorophenyl)[[(3,3,3-trifluoro-2-(trifluoromethyl)propyl)sulfonyl]methyl]pyridine-2-carboxamide

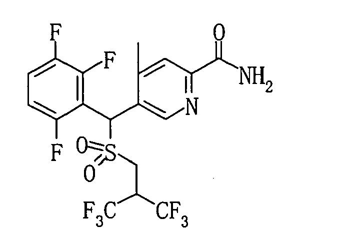

2-(Iodomethyl)-1,1,1,3,3,3-hexafluoropropane (337 mg, 1.15 mmol) and potassium carbonate (159 mg, 1.15 mmol) were added to a N,N-dimethylformamide (7 ml) solution of 5-[mercapto(2,3,6-trifluorophenyl)methyl]-4-methylpyridine-2-carboxamide (300 mg, 0.961 mmol) obtained in Reference Example 11, and the mixture was stirred at room temperature for 1.5 hours. Water was added to the reaction solution, followed by extraction with ethyl acetate, and then the organic layer was washed sequentially with water and saturated saline. The resulting organic layer was dried over anhydrous magnesium sulfate, and then concentrated under reduced pressure.

Under ice-cold conditions, 30% aqueous hydrogen peroxide (1.1 ml) was added to a formic acid (5 ml) solution from the resulting residue, and the mixture was stirred at room temperature for 17 hours. Water was added to the reaction solution, and the solid precipitated was filtered. The solid was dissolved in ethyl acetate, and the solution was washed sequentially with water and saturated saline. The resulting organic layer was dried over anhydrous magnesium sulfate, and then concentrated under reduced pressure. The resulting residue was subjected to flash silica gel column chromatography (hexane/ethyl acetate) to give the title compound (110 mg, 0.216 mmol, 23%) as a white powder.
¹H-NMR (400 MHz, CDCl₃) δ: 2.39 (3H, s), 3.43-3.56 (2H, m), 3.30-3.91 (1H, m), 5.60 (1H, br s), 6.01 (1H, s), 6.99-7.05 (1H, m), 7.26-7.35 (1H, m), 7.84 (1H, br s), 8.10 (1H, s), 9.27 (1H, s).
IR (ATR) cm⁻¹: 3428, 3153, 1695, 1635, 1602, 1550, 1494, 1421, 1380, 1344, 1322, 1290, 1240, 1220, 1197, 1153, 1132, 1099, 1060.
mp: 194-196°C.
MS (m/z): 509 (M⁺+H).
TOF-ESI-MS: 509.0536 (Calculated value as C₁₈H₁₄F₉N₂O₃S: 509.0581).

### Reference Example 12: 5-[[[2-(1,3-Dioxolan-2-yl)ethyl]thio](2,3,6-trifluorophenyl)methyl]-4-methylpyridine-2-carboxamide

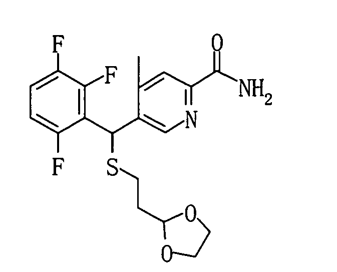

Under argon atmosphere, 2-(2-bromoethyl)-1,3-dioxolane (0.207 ml, 1.76 mmol), and then potassium carbonate (243 mg, 1.76 mmol) were added at 0°C to a N,N-dimethylformamide (10 ml) solution of 5-[mercapto(2,3,6-trifluorophenyl)methyl]-4-methylpyridine-2-carboxamide (500 mg, 1.60 mmol) obtained in Reference Example 11, and the mixture was stirred at room temperature for 4 hours. The reaction solution was cooled to 0°C, followed by sequential addition of ethyl acetate and aqueous ammonium chloride, and then the mixture was extracted with ethyl acetate. The extracts were washed with water and saturated saline, dried over magnesium sulfate, and then concentrated. The resulting residue was subjected to flash silica gel column chromatography using hexane:ethyl acetate = 2:3 as the eluent. The resulting fractions were concentrated to give the title compound (434 mg, 1.05 mmol, 66%) as a white solid.
¹H-NMR (400 MHz, CDCl₃) δ: 1.93-2.01 (2H, m), 2.32 (3H, s), 2.59-2.73 (2H, m), 3.81-3.98 (4H, m), 4.94 (1H, t, J=4.6Hz), 5.55 (1H, s), 5.59 (1H, s), 6.84 (1H, tdd, J=9.3, 3.9, 2.2Hz), 7.09 (1H, ddd, J=18.1, 9.3, 4.9Hz), 7.85 (1H, s), 7.96 (1H, s), 9.06 (1H, s).
MS (m/z): 413 (M⁺+H).

### Reference Example 13: 4-Methyl-5-[[(3-oxopropyl)thio](2,3,6-trifluorophenyl)methyl]pyridine-2-carboxamide

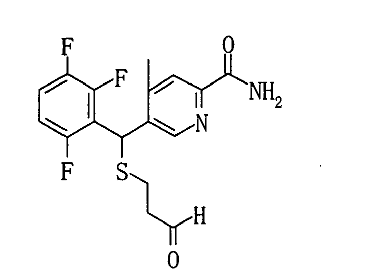

Water (1.5 ml) and concentrated hydrochloric acid (1.5 ml) were added to a 1,4-dioxane (4 ml) solution of 5-[[[2-(1,3-dioxolan-2-yl)ethyl]thio](2,3,6-trifluorophenyl)methyl]-4-methylpyridine-2-carboxamide (430 mg, 1.04 mmol), and the mixture was stirred at room temperature for 2 hours. Water was added to the reaction solution, followed by extraction with ethyl acetate. The resulting extracts were washed with water and saturated saline, dried over magnesium sulfate, and then concentrated. The resulting residue was dissolved in 1,4-dioxane (4 ml), followed by addition of water (1.5 ml) and concentrated hydrochloric acid (1.5 ml), and then the mixture was stirred at room temperature for 5 hours. Saturated saline was added to the reaction solution, followed by extraction with ethyl acetate. The resulting extracts were washed with water and saturated saline, dried over magnesium sulfate, and then concentrated. The resulting residue was subjected to flash silica gel column chromatography using hexane:ethyl acetate = 1:2 as the eluent. The resulting fractions were concentrated to give the title compound (188 mg, 0.511 mmol, 49%) as a white amorphous.
¹H-NMR (400 MHz, CDCl₃) δ: 2.34 (3H, s), 2.76-2.88 (4H, m), 5.56 (1H, s), 5.63 (1H, s), 6.86 (1H, tdd, J=9.3, 3.9, 2.2Hz), 7.10 (1H, ddd, J=18.7, 9.3, 5.1Hz), 7.84 (1H, s), 7.98 (1H, s), 9.02 (1H, s), 9.75 (1H, s).
MS (m/z): 369 (M⁺+H).

### Example 28: 5-[[(3,3-Difluoropropyl)thio](2,3,6-trifluorophenyl)methyl]-4-methylpyridine-2-carboxamide

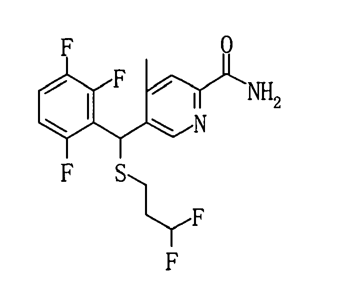

Under argon atmosphere, diethylamino sulfur trifluoride (0.338 ml, 2.56 mmol) was added to a dichloromethane (3 ml) solution of 4-methyl-5-[[(3-oxopropyl)thio](2,3,6-trifluorophenyl)methyl]pyridine-2-carboxamide (188 mg, 0.511 mmol), and then the mixture was stirred at room temperature for 5 hours. The reaction solution was diluted with dichloromethane, and then poured into ice-cold water. The resulting mixture was made alkaline with saturated aqueous sodium hydrogen carbonate. The organic layer was separated, and then washed with saturated aqueous sodium hydrogen carbonate, water and saturated saline. The organic layer was dried over magnesium sulfate, concentrated. The resulting residue was subjected to flash silica gel column chromatography using hexane:ethyl acetate = 2:3 as the eluent. The resulting fractions were concentrated to give the title compound (130 mg, 0.333 mmol, 65%) as a yellow oil.
¹H-NMR (400 MHz, CDCl₃) δ: 2.06-2.21 (2H, m), 2.33 (3H, s), 2.61-2.82 (2H, m), 5.51-5.60 (2H, brm), 5.91 (1H, tt, J=56.3, 4.1Hz), 6.87 (1H, tdd, J=9.3, 3.9, 2.2Hz), 7.12 (1H, ddd, J=18.0, 9.3, 4.9Hz), 7.83 (1H, s), 7.98 (1H, s), 9.02 (1H, s). MS (m/z): 391 (M⁺+H).

### Example 29: 5-[[(3,3-Difluoropropyl)sulfonyl](2,3,6-trifluorophenyl)methyl]-4-methylpyridine-2-carboxamide

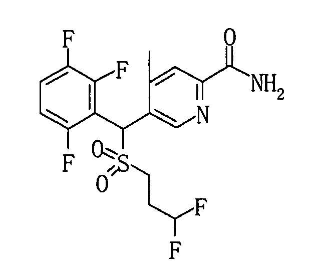

31% Aqueous hydrogen peroxide (2.0 ml) and hexaammonium heptamolybdate tetrahydrate (30.0 mg, 0.0243 mmol) were added to a methanol (6 ml) solution of 5-[[(3,3-difluoropropyl)thio](2,3,6-trifluorophenyl)methyl]-4-methylpyridine-2-carboxamide (130 mg, 0.217 mmol), and the mixture was stirred at room temperature for 2 days. Water was added to the reaction solution, and the mixture was concentrated to about half volume under reduced pressure. Ethyl acetate was added to the resulting residue. The mixture was washed sequentially with saturated aqueous sodium hydrogen carbonate, aqueous sodium thiosulfate, water and saturated saline, dried over magnesium sulfate, and then concentrated. The resulting residue was subjected to flash silica gel column chromatography using hexane:ethyl acetate = 2:3 as the eluent. The resulting fractions were concentrated. The resulting solid was recrystallized from 2-propanol to give the title compound (76.3 mg, 0.181 mmol, 54%) as a white solid.
¹H-NMR (400 MHz, CDCl₃) δ: 2.31-2.56 (2H, m), 3.14-3.33 (2H, m), 5.56 (1H, s), 5.95 (1H, s), 6.05 (1H, tt, J=55.9, 3.7Hz), 6.97-7.04 (1H, m), 7.23-7.33 (1H, m), 7.85 (1H, s), 8.08 (1H, s), 9.32 (1H, s).
IR (ATR) cm⁻¹: 3417, 1697, 1602, 1496, 1412, 1325, 1311, 1248, 1205, 1134, 1115, 1065, 1045, 985, 914, 831, 725, 654, 629, 617, 573, 523, 471, 409.
mp: 191.5-192.0°C.
MS (m/z): 423 (M⁺+H).
Elemental analysis: C₁₇H₁₅F₅N₂O₃S: Theoretical value: C 48.34; H 3.58; F 22.49; N 6.63; S 7.59. Observed value: C 48.42; H 3.40; F 22.55; N 6.60; S 7.61.

### Example 30: 5-[[(3-Chloropropyl)sulfonyl](2,3,6-trifluorophenyl)methyl]-4-methylpyridine-2-carboxamide

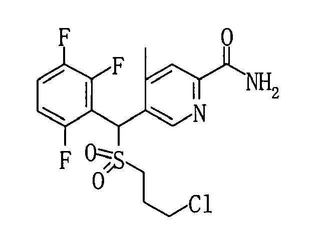

1-Bromo 3-chloropropane (190 µl, 1.92 mmol) and potassium carbonate (266 mg, 1.92 mmol) were added to a N,N-dimethylformamide (7 ml) solution of 5-[mercapto(2,3,6-trifluorophenyl)methyl]-4-methylpyridine-2-carboxamide (500 mg, 1.60 mmol) obtained in Reference Example 11, and the mixture was stirred at room temperature for 2 hours. Water was added to the reaction solution, followed by extraction with ethyl acetate, and then the organic layer was washed sequentially with water and saturated saline. The resulting organic layer was dried over anhydrous magnesium sulfate, and then concentrated under reduced pressure.

Hexaammonium heptamolybdate tetrahydrate (30 mg) and 30% aqueous hydrogen peroxide (4 ml) were added to a solution of the resulting residue in a combined solvent of methanol (4 ml) and ethyl acetate (4 ml), and the mixture was stirred for 16 hours. Water was added to the reaction solution, followed by extraction with ethyl acetate, and then the extracts were washed sequentially with saturated aqueous sodium hydrogen carbonate, saturated aqueous sodium thiosulfate and saturated saline. The resulting organic layer was dried over anhydrous magnesium sulfate, and then concentrated under reduced pressure. The resulting residue was subjected to flash silica gel column chromatography (hexane/ethyl acetate) to give the title compound (420 mg, 0.998 mmol, 62%) as a white powder.
¹H-NMR (400 MHz, CDCl₃) δ: 2.27-2.42 (2H, m), 2.39 (3H, s), 3.12-3.33 (2H, m), 3.67 (2H, t, J=6.1Hz), 5.57 (1H, br s), 5.92 (1H, s), 6.96-7.02 (1H, m), 7.23-7.31 (1H, m), 7.85 (1H, br s), 8.07 (1H, s), 9.34 (1H, s).
IR (ATR) cm⁻¹: 3411, 1693, 1637, 1602, 1550, 1496, 1419, 1346, 1324, 1303, 1245, 1205, 1130, 1014.
mp: 197-199°C.
MS (m/z): 421 (M⁺+H).
Elemental analysis: C₁₇H₁₆ClF₃N₂O₃S: Theoretical value: C 48.52; H 3.83; Cl 8.42; F 13.54; N 6.66; S 7.62. Observed value: C 48.49; H 3.67; Cl 8.44; F 13.49; N 6.56; S 7.56.

### Reference Example 14: 2-[2-[2-(Benzyloxy)ethyl]-1,3-dioxolan-2-yl]ethyl-4-methylbenzenesulfonate

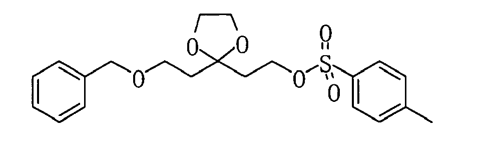

p-Toluenesulfonyl chloride (720 mg, 3.77 mmol) was added at 0°C to a dichloromethane (20 ml) solution of 2-[2-[2-(benzyloxy)ethyl]-1,3-dioxolan-2-yl]ethanol (730 mg, 2.90 mmol), triethylamine (0.605 ml, 4.35 mmol) and 4-dimethylaminopyridine (35.4 mg, 0.290 mmol), and the mixture was stirred at 0°C for 30 minutes, and then at room temperature for 24 hours. The reaction solution was cooled to 0°C, followed by addition of triethylamine (0.100 ml, 0.719 mmol) and p-toluenesulfonyl chloride (100 mg, 0.524 mmol), and then the mixture was stirred at room temperature for 4 hours. The reaction solution was concentrated to about half volume under reduced pressure, diluted with ethyl acetate, and then washed with water and saturated saline. The solution was dried over magnesium sulfate, and then concentrated. The resulting residue was subjected to flash silica gel column chromatography using hexane:ethyl acetate = 3:1 as the eluent. The resulting fractions were concentrated to give the title compound (979 mg, 2.41 mmol, 83%) as a colorless oil.
¹H-NMR (400 MHz, CDCl₃) δ: 1.89 (2H, t, J=6.8Hz), 2.07 (2H, t, J=7.3Hz), 2.43 (3H, s), 3.52 (2H, t, J=6.8Hz), 3.85-3.89 (4H, m), 4.14 (2H, t, J=7.3Hz), 4.45 (2H, s), 7.27-7.37 (7H, m), 7.78 (2H, d, J=8.3Hz).
MS (m/z) : 407 (M⁺+H).

### Reference Example 15: 2-[2-(Benzyloxy)ethyl]-2-(2-iodoethyl)-1,3-dioxolane

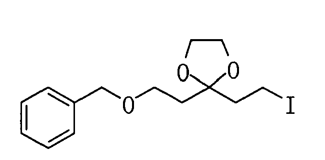

2-[2-[2-(Benzyloxy)ethyl]-1,3-dioxolan-2-yl]ethyl-4-methylbenzenesulfonate (750 mg, 1.85 mmol) was dissolved in N-methyl-2-pyrrolidone (10 ml), followed by addition of sodium iodide (1.11 g, 7.40 mmol), and then the mixture was stirred at 60°C. Ethyl acetate was added to the reaction solution, and then the mixture was washed sequentially with aqueous sodium thiosulfate, water and saturated saline, dried over magnesium sulfate, and then concentrated. The resulting residue was subjected to flash silica gel column chromatography using hexane:ethyl acetate = 6:1 as the eluent. The resulting fractions were concentrated to give the title compound (613 mg, 1.69 mmol, 91%) as a colorless oil.
¹H-NMR (400 MHz, CDCl₃) δ: 1.94 (2H, t, J=6.7Hz), 2.28-2.35 (2H, m), 3.13-3.20 (2H, m), 3.56 (2H, t, J=6.7Hz), 3.92-3.94 (4H, m), 4.49 (2H, s), 7.26-7.38 (5H, m).
MS (m/z) : 363 (M⁺+H).

### Reference Example 16: 5-[[[2-[2-[2-(Benzyloxy)ethyl]-1,3-dioxolan-2-yl]ethyl]thio](2,3,6-trifluorophenyl)methyl]-4-methylpyridine-2-carboxamide

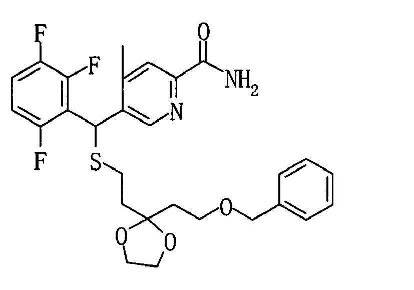

Under argon atmosphere, potassium carbonate (46.1 mg, 0.334 mmol) was added at 0°C to a N,N-dimethylformamide (2 ml) solution of 5-[mercapto(2,3,6-trifluorophenyl)methyl]-4-methylpyridine-2-carboxamide (94.8 mg, 0.304 mmol) obtained in Reference Example 11 and 2-[2-(benzyloxy)ethyl]-2-(2-iodoethyl)-1,3-dioxolane (110 mg, 0.304 mmol), and then the mixture was stirred at room temperature for 15 hours. The reaction solution was cooled to 0°C, followed by sequential addition of ethyl acetate and water. The organic layer was separated, washed with water and saturated saline, and then dried over magnesium sulfate, and concentrated. The resulting residue was subjected to flash silica gel column chromatography using hexane:ethyl acetate = 2:3 as the eluent. The resulting fractions were concentrated to give the title compound (134 mg, 0.245 mmol, 81%) as a colorless oil.
¹H-NMR (400 MHz, CDCl₃) δ: 1.89-2.04 (4H, m), 2.30 (3H, s), 2.54-2.69 (2H, m), 3.53 (2H, t, J=6.8Hz), 3.86-3.91 (4H, m), 4.47 (2H, s), 5.55 (2H, s), 6.78-6.86 (1H, m), 7.07 (1H, ddd, J=18.1, 9.0, 4.9Hz), 7.25-7.36 (5H, m), 7.84 (1H, s), 7.96 (1H, s), 9.04 (1H, s).
MS (m/z) : 547 (M⁺+H).

### Reference Example 17: 5-[[[5-(Benzyloxy)-3-oxopentyl]thio](2,3,6-trifluorophenyl)methyl]-4-methylpyridine-2-carboxamide

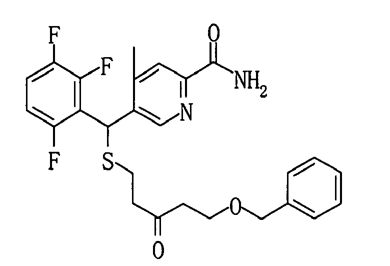

Water (2.0 ml) and concentrated hydrochloric acid (2.0 ml) were added to a 1,4-dioxane (10 ml) solution of 5-[[[2-[2-[2-(benzyloxy)ethyl]-1,3-dioxolan-2-yl]ethyl]thio](2,3,6-trifluorophenyl)methyl]-4-methylpyridine-2-carboxamide (733 mg, 1.34 mmol), and the mixture was stirred at room temperature for 5 hours. Saturated saline was added to the reaction solution, followed by extraction with ethyl acetate. The extracts were washed with water and saturated saline, dried over magnesium sulfate, and then concentrated. The resulting residue was subjected to flash silica gel column chromatography using hexane:ethyl acetate = 1:1 as the eluent. The resulting fractions were concentrated to give the title compound (557 mg, 1.11 mmol, 83%) as a colorless oil.
¹H-NMR (400 MHz, CDCl₃) δ: 2.31 (3H, s), 2.66 (2H, td, J=6.1, 1.5Hz), 2.73-2.92 (4H, m), 3.71 (2H, t, J=6.1Hz) 4.49 (2H, s), 5.53 (1H, br s), 5.61 (1H, s), 6.83 (1H, tdd, J=9.3, 3.7, 2.2Hz), 7.08 (1H, ddd, J=18.1, 9.3, 5.1Hz), 7.23-7.36 (5H, m), 7.84 (1H, s), 7.96 (1H, s), 9.03 (1H, s).
MS (m/z): 503 (M⁺+H).

### Reference Example 18: 5-[[[5-(Benzyloxy)-3,3-difluoropentyl]thio](2,3,6-trifluorophenyl)methyl]-4-methylpyridine-2-carboxamide

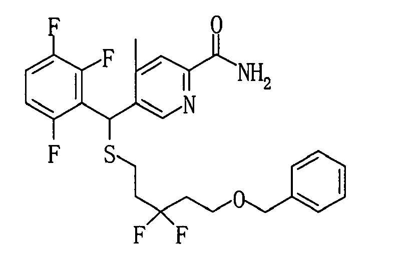

Under argon atmosphere, in a polyethylene (FEP) container, diethylamino sulfur trifluoride (1.31 ml, 9.94 mmol) was added at 0°C to a dichloromethane (6 ml) solution of 5-[[[5-(benzyloxy)-3-oxopentyl]thio](2,3,6-trifluorophenyl)methyl]-4-methylpyridine-2-carboxamide (500 mg, 0.994 mmol), and the mixture was stirred at room temperature for 18 hours. The reaction solution was diluted with dichloromethane, and then poured into ice-cold water. The resulting mixture was made alkaline with saturated aqueous sodium hydrogen carbonate, and then extracted with dichloromethane. The extracts were washed with water and saturated saline, dried over magnesium sulfate, and then concentrated. The resulting residue was subjected to flash silica gel column chromatography using hexane:ethyl acetate = 3:2 as the eluent. The resulting fractions were concentrated to give the title compound (166 mg, 0.316 mmol, 32%) as a yellow oil.
¹H-NMR (400 MHz, CDCl₃) δ: 2.09-2.23 (4H, m), 2.30 (3H, s), 2.65-2.77 (2H, m), 3.61 (2H, t, J=6.2Hz), 4.49 (2H, s), 5.49-5.58 (2H, brm), 6.80-6.88 (1H, m), 7.04-7.13 (1H, m), 7.26-7.39 (5H, m), 7.84 (1H, s), 7.97 (1H, s), 9.02 (1H, s).
MS (m/z) : 525 (M⁺+H).

### Reference Example 19: 5-[[[5-(Benzyloxy)-3,3-difluoropentyl]sulfonyl](2,3,6-trifluorophenyl)methyl]-4-methylpyridine-2-carboxamide

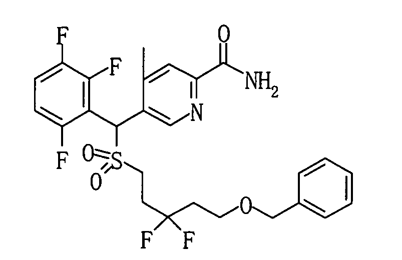

31% Aqueous hydrogen peroxide (15.0 ml) and hexaammonium heptamolybdate tetrahydrate (300 mg, 0.243 mmol) were added to a methanol (60 ml) solution of 5-[[[5-(benzyloxy)-3,3-difluoropentyl]thio](2,3,6-trifluorophenyl)methyl]-4-methylpyridine-2-carboxamide (1.62 g, 3.09 mmol), and the mixture was stirred at room temperature for 20 hours. The reaction solution was cooled to 0°C, followed by addition of water, and then the mixture was made alkaline with saturated aqueous sodium hydrogen carbonate. The resulting mixture was extracted with ethyl acetate, and then the extracts were washed sequentially with aqueous sodium thiosulfate, water and saturated saline. The solution was dried over magnesium sulfate, and concentrated. The resulting residue was subjected to flash silica gel column chromatography using hexane:ethyl acetate = 1:1 as the eluent. The resulting fractions were concentrated to give the title compound (1.11 g, 1.99 mmol, 64%) as a white amorphous.
¹H-NMR (400 MHz, CDCl₃) δ: 2.13-2.26 (2H, m), 2.33 (3H, s), 2.40-2.64 (2H, m), 3.19-3.37 (2H, m), 3.60 (2H, t, J=6.0Hz), 4.49 (2H, s), 5.58 (1H, br s), 5.91 (1H, s), 6.93-7.01 (1H, m), 7.21-7.37 (6H, m), 7.84 (1H, br s), 8.06 (1H, s), 9.34 (1H, s). MS (m/z) : 557 (M⁺+H).

### Reference Example 20: 5-[[(3,3-Difluoro-5-hydroxypentyl)sulfonyl](2,3,6-trifluorophenyl)methyl]-4-methylpyridine-2-carboxamide

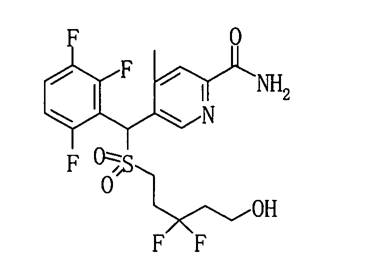

10% Palladium carbon (50% w/w H₂O, 600 mg) was added to an ethanol (30 ml) solution of 5-[[[5-(benzyloxy)-3,3-difluoropentyl]sulfonyl](2,3,6-trifluorophenyl)methyl]-4-methylpyridine-2-carboxamide (1.11 g, 2.01 mmol), and the mixture was stirred under hydrogen atmosphere at room temperature for 15 hours. The catalyst was filtered off, and then the solvent was distilled off under reduced pressure. The same procedure was repeated 4 times. Then, the resulting residue was dissolved in ethanol (30 ml), followed by addition of 10% palladium carbon (50% w/w H₂O, 800 mg), and the mixture was stirred under hydrogen atmosphere at room temperature for 18 hours. The catalyst was filtered off, and then the solvent was distilled off. The resulting residue was dissolved in dichloromethane, dried over magnesium sulfate, and then concentrated. The resulting residue was subjected to flash silica gel column chromatography using hexane:ethyl acetate = 1:4 as the eluent. The resulting fractions were concentrated to give the title compound (487 mg, 1.05 mmol, 52%) as a white amorphous.
¹H-NMR (400 MHz, CDCl₃) δ: 2.09-2.22 (2H, m), 2.36-2.61 (5H, m), 3.22-3.39 (2H, m), 3.85 (2H, q, J=5.6Hz), 5.61 (1H, s), 5.94 (1H, s), 6.95-7.03 (1H, m), 7.23-7.32 (1H, m), 7.86 (1H, s), 8.07 (1H, s), 9.34 (1H, s).
IR (ATR) cm⁻¹: 3458, 3356, 1680, 1601, 1572, 1495, 1419, 1315, 1244, 1134, 987, 922, 814, 725, 490.
HRMS (m/z): 467.10219 (Calculated value as C₁₉H₂₀F₅N₂O₄S: 467.10639).

### Example 31: 4-Methyl-5-[[(3,3,5-trifluoropentyl)sulfonyl](2,3,6-trifluorophenyl)methyl]pyridine-2-carboxamide

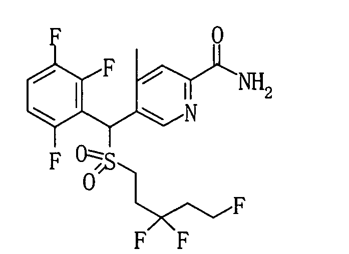

Diethylamino sulfur trifluoride (0.0467 ml, 0.354 mmol) was added at 0°C to a dichloromethane (2 ml) solution of 5-[[(3,3-difluoro-5-hydroxypentyl)sulfonyl](2,3,6-trifluorophenyl)methyl]-4-methylpyridine-2-carboxamide (110 mg, 0.236 mmol), and the mixture was stirred at room temperature for 2 hours. The reaction solution was poured into ice-cold water, and then extracted with ethyl acetate. The extracts were washed with water and saturated saline, dried over magnesium sulfate, and concentrated. The resulting residue was subjected to flash silica gel column chromatography using hexane:ethyl acetate = 2:3 as the eluent. The resulting fractions were concentrated. The resulting solid was recrystallized from isopropanol to give the title compound (8.0 mg, 0.0171 mmol, 7%) as a white solid. ¹H-NMR (400 MHz, CDCl₃) δ: 2.22-2.61 (4H, m), 2.57 (3H, s), 3.21-3.38 (2H, m), 4.62 (2H, dt, J=46.9, 5.6Hz), 5.61 (1H, s), 5.94 (1H, s), 6.95-7.04 (1H, m), 7.23-7.32 (1H, m), 7.86 (1H, s), 8.07 (1H, s), 9.34 (1H, s).
HRMS (m/z): 469.09851 (calculated as C₁₉H₁₉F₆N₂O₃S: 469.10206).

### Reference Example 21: (3,3-Difluorocyclobutyl)methyl methanesulfonate

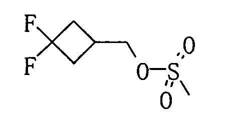

Under nitrogen atmosphere, ethanol (0.144 ml) and bis(2-methoxyethyl)amino sulfur trifluoride (5.61 ml, 30.4 mmol) were added at 0°C to a dichloromethane (60 ml) solution of ethyl 3-oxocyclobutanecarboxylate (1.73 g, 12.17 mmol). The reaction mixture was stirred at room temperature for 17 hours, poured into 0°C-cooled saturated aqueous sodium hydrogen carbonate, and then the mixture was stirred at room temperature for 3 hours. The reaction mixture was extracted with dichloromethane, and the organic layer was washed with saturated aqueous ammonium chloride, and then with saturated saline. The resulting organic layer was dried over anhydrous sodium sulfate, filtered, and then the filtrates were concentrated under reduced pressure.

Under argon atmosphere, a tetrahydrofuran solution of lithium aluminum hydride (1.00 M, 26.8 ml, 26.8 mmol) was added at -50°C to a tetrahydrofuran (50 ml) solution of the resulting residue, and the reaction mixture was stirred at -30°C for 1 hour. Subsequently, the temperature of the mixture was raised to -10°C, and the mixture was stirred for 1 hour. Saturated aqueous sodium sulfate (3 ml), and then diethylether (30 ml) were added to the reaction mixture at the same temperature, and then the mixture was stirred for 30 minutes. Precipitate was filtered by Celite, and the filtrates were concentrated under reduced pressure.

Under nitrogen atmosphere, methanesulfonyl chloride (1.41 ml, 18.3 mmol), and then triethylamine (5.09 ml, 36.5 mmol) were added at 0°C to a dichloromethane (40 ml) solution of the resulting residue, and the mixture was stirred at room temperature for 1 hour. The reaction mixture was washed with saturated aqueous ammonium chloride, saturated aqueous sodium hydrogen carbonate and then with saturated saline. Subsequently, the mixture was dried over anhydrous sodium sulfate and filtered, and then the filtrates were concentrated under reduced pressure. The resulting residue was subjected to flash silica gel chromatography using hexane:dichloromethane = 2:3 as the eluent. The resulting fractions were concentrated under reduced pressure to give the title compound (1.44 g, 7.19 mmol, 59%) as a colorless oil.
¹H-NMR (400 MHz, CDCl₃) δ: 2.34-2.50 (2H, m), 2.51-2.65 (1H, m), 2.66-2.80 (2H, m), 3.04 (3H, s), 4.27 (2H, d, J=6.6Hz).

### Example 32: 5-[[[(3,3-Difluorocyclobutyl)methyl]sulfonyl](2,3,6-trifluorophenyl)methyl]-4-methylpyridine-2-carboxamide

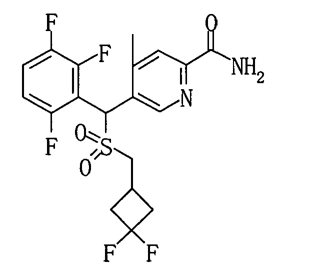

Under nitrogen atmosphere, (3,3-difluorocyclobutyl)methyl methanesulfonate (120 mg, 0.60 mmol) and potassium carbonate (83 mg, 0.60 mmol) were added at room temperature to a N,N-dimethylformamide (5 ml) solution of 5-[mercapto(2,3,6-trifluorophenyl)methyl]-4-methylpyridine-2-carboxamide (156 mg, 0.50 mmol) obtained in Reference Example 11, and the mixture was stirred for 5 hours. Ethyl acetate was added to the reaction mixture, and the mixture was washed with water. The resulting organic layer was washed twice with saturated saline, dried over anhydrous sodium sulfate and filtered. Then the filtrates were concentrated under reduced pressure. The resulting residue was subjected to flash silica gel chromatography using hexane:ethyl acetate = 3:2 as the eluent. The resulting fractions were concentrated under reduced pressure.

Methanol (3 ml), 31% aqueous hydrogen peroxide (3 ml) and hexaammonium heptamolybdate tetrahydrate (62 mg, 0.05 mmol) were added at room temperature to an ethyl acetate (3 ml) solution of the resulting residue, and the mixture was stirred at room temperature for 17 hours. Ethyl acetate was added to the reaction mixture, and the mixture was washed with saturated aqueous sodium hydrogen carbonate, 10% aqueous sodium thiosulfate, and then with saturated saline. The resulting organic layer was dried over anhydrous sodium sulfate and filtered. Then the filtrates were concentrated under reduced pressure. The resulting residue was subjected to flash silica gel column chromatography using hexane:ethyl acetate = 1:1 as the eluent. The resulting fractions were concentrated under reduced pressure. The resulting residue was washed with a combined solvent of 2-propanol and hexane, and then filtered to give the title compound (11 mg, 0.025 mmol, 5%) as a white solid.
¹H-NMR (400 MHz, CDCl₃) δ: 2.35-2.57 (2H, m), 2.38 (3H, s), 2.73-2.99 (3H, m), 3.19 (1H, dd, J=13.2, 7.3Hz), 3.30 (1H, dd, J=13.2, 7.6Hz), 5.59 (1H, br s), 5.88 (1H, s), 6.96-7.03 (1H, m), 7.23-7.33 (1H, m), 7.85 (1H, br s), 8.08 (1H, s), 9.31 (1H, s).
mp: 205-207°C.
HRMS (m/z): 449.09860 (calculated as C₁₉H₁₈F₅N₂O₃S: 449.09583).

### Example 33: 5-[[(3-Chloro-3,3-difluoropropyl)thio](2,3,6-trifluorophenyl)methyl]-4-methylpyridine-2-carboxamide

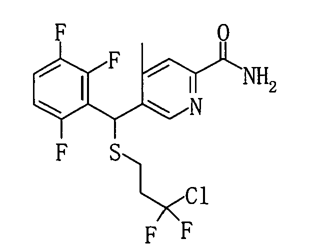

Under nitrogen atmosphere, in a sealed tube, S-potassium thioacetate (0.92 g, 8.06 mmol) was added to an acetone (8 ml) solution of 1,3-dichloro-1,1-difluoropropane (1.0 g, 6.71 mmol), and the mixture was stirred at 50°C for 20 hours. Water was added to the reaction solution, followed by extraction with dichloromethane. The resulting organic layer was dried over anhydrous magnesium sulfate, and then concentrated under reduced pressure.

Under nitrogen atmosphere, potassium carbonate (649 mg, 4.70 mmol) and 5-[chloro(2,3,6-trifluorophenyl)methyl]-4-methylpyridine-2-carboxamide (1.5 g, 4.70 mmol) obtained in Reference Example 10 were added to a methanol (20 ml) solution of the resulting residue, and the mixture was stirred at 50°C for 3 hours. The reaction solution was concentrated under reduced pressure, followed by addition of water and extraction with dichloromethane. The organic layer was washed with saturated saline. The resulting organic layer was dried over anhydrous magnesium sulfate and then concentrated under reduced pressure. The resulting residue was subjected to flash silica gel column chromatography (hexane/ethyl acetate) to give the title compound (1.72 g, 4.05 mmol, 60%) as a pale yellow powder. ¹H-NMR (400 MHz, CDCl₃) δ: 2.34 (3H, s), 2.50-2.66 (2H, m), 2.72-2.83 (2H, m), 5.59 (1H, s), 5.60 (1H, br s), 6.85-6.91 (1H, m), 7.09-7.17 (1H, m), 7.84 (1H, br s), 7.99 (1H, s), 9.01 (1H, s).
MS (m/z): 425 (M⁺+H).

### Example 34: 5-[[(3-Chloro-3,3-difluoropropyl)sulfonyl](2,3,6-trifluorophenyl)methyl]-4-methylpyridine-2-carboxamide

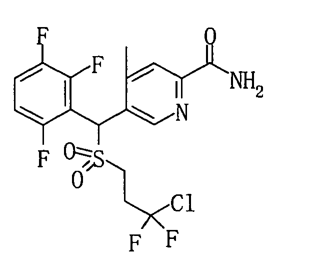

Hexaammonium heptamolybdate tetrahydrate (50 mg) and 30% aqueous hydrogen peroxide (5 ml) were added to a solution of 5-[[(3-chloro-3,3-difluoropropyl)thio](2,3,6-trifluorophenyl)methyl]-4-methylpyridine-2-carboxamide (119) (300 mg, 0.706 mmol) in a combined solvent of methanol (5 ml) and ethyl acetate (5 ml), and the mixture was stirred for 6 hours. Water was added to the reaction solution, followed by extraction with ethyl acetate, and then the solution was washed sequentially with saturated aqueous sodium thiosulfate and saturated saline. The resulting organic layer was dried over anhydrous magnesium sulfate, and then concentrated under reduced pressure. The resulting residue was subjected to flash silica gel column chromatography (hexane/ethyl acetate) to give the title compound (270 mg, 0.591 mmol, 84%) as a white powder. ¹H-NMR (400 MHz, CDCl₃) δ: 2.39 (3H, s), 2.78-2.96 (2H, m), 3.23-3.38 (2H, m), 5.57 (1H, br s), 5.95 (1H, s), 6.99-7.05 (1H, m), 7.22-7.34 (1H, m), 7.84 (1H, br s), 8.09 (1H, s), 9.31 (1H, s).
IR (ATR) cm⁻¹: 3419, 3154, 1697, 1637, 1602, 1548, 1494, 4121, 1373, 1348, 1321, 1247, 1205, 1172, 1133, 1103, 1025.
mp: 181-183°C.
MS (m/z): 457 (M⁺+H).
TOF-ESI-MS: 457.0379 (Calculated as C₁₇H₁₅ClF₅N₂O₃S: 457.0412). Elemental analysis: C₁₇H₁₄ClF₅N₂O₃S: Theoretical value: C 44.70; H 3.09; Cl 7.76; F 20.79; N 6.13; S 7.02. Observed value: C 45.01; H 3.15; Cl 7.79; F 20.59; N 6.21; S 6.79.

### Example 35: 5-[[(3-Chloro-3,3-difluoropropyl)sulfonyl](2,3,6-trifluorophenyl)methyl]-N-(hydroxymethyl)-4-methylpyridine-2-carboxamide

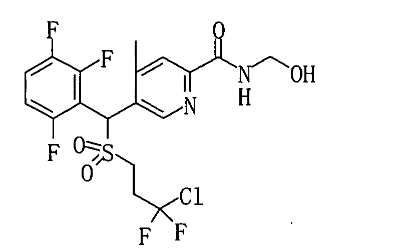

37% Aqueous formaldehyde (80 µl) and 1N aqueous sodium hydroxide (30 µl, 0.030 mmol) were added to an ethylene glycol dimethylether (5 ml) solution of 5-[[(3-chloro-3,3-difluoropropyl)sulfonyl](2,3,6-trifluorophenyl)methyl]-4-methylpyridine-2-carboxamide (150 mg, 0.328 mmol), and the mixture was stirred at room temperature for 1 hour. Water was added to the reaction solution, followed by extraction with ethyl acetate, and then the organic layer was washed with saturated saline. The resulting organic layer was dried over anhydrous magnesium sulfate, and then concentrated under reduced pressure. The resulting residue was subjected to flash silica gel column chromatography (hexane/ethyl acetate) to give the title compound (120 mg, 0.246 mmol, 75%) as a white powder. ¹H-NMR (400 MHz, CDCl₃) δ: 2.40 (3H, s), 2.78-2.96 (2H, m), 3.17 (1H, t, J=7.7Hz), 3.23-3.40 (2H, m), 4.99-5.03 (2H, m), 5.94 (1H, s), 6.99-7.05 (1H, m), 7.26-7.34 (1H, m), 8.07 (1H, s), 8.87 (1H, br s), 9.31 (1H,s).
IR (ATR) cm⁻¹: 3355, 1671, 1602, 1517, 1496, 1438, 1373, 1336, 1295, 1247, 1199, 1180, 1141, 1103, 1083, 1031.
mp: 150-152°C.
MS (m/z): 487 (M⁺+H).
Elemental analysis: C₁₈H₁₆ClF₅N₂O₄S: Theoretical value: C 44.41; H 3.31; Cl 7.28; F 19.51; N 5.75; S 6.59. Observed value: C 44.08; H 3.28; Cl 7.38; F 19.48; N 5.53; S 6.59.

### Example 36: 5-[[(3,3-Difluoroprop-2-en-1-yl)thio](2,3,6-trifluorophenyl)methyl]-4-methylpyridine-2-carboxamide

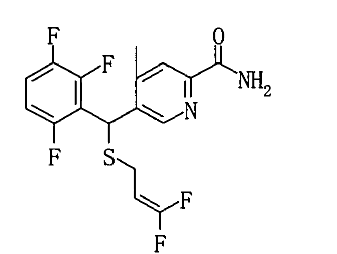

1,8-Diazabicyclo[5.4.0]-7-undecene (317 µl, 2.12 mmol) was added to a toluene (3 ml) solution of 5-[[(3-chloro-3,3-difluoropropyl)thio](2,3,6-trifluorophenyl)methyl]-4-methylpyridine-2-carboxamide (100 mg, 0.235 mmol) obtained in Example 33, and the mixture was stirred with heating under reflux for 7 hours, then stirred at room temperature for 3 days. Saturated aqueous ammonium chloride and water were added to the reaction solution, followed by extraction with ethyl acetate, and then the organic layer was washed with saturated saline. The resulting organic layer was dried over anhydrous magnesium sulfate, and then concentrated under reduced pressure. The resulting residue was subjected to flash silica gel column chromatography (hexane/ethyl acetate) to give the title compound (187 mg, 0.482 mmol, 68%) as a pale yellow oil.
¹H-NMR (400 MHz, CDCl₃) δ: 2.34 (3H, s), 3.15-3.18 (2H, m), 4.35 (1H, dtd, J=23.7, 8.1, 1.5Hz), 5.58 (1H, s), 5.60 (1H, br s), 6.82-6.89 (1H, m), 7.06-7.15 (1H, m), 7.84 (1H, br s), 7.98 (1H, s), 9.03 (1H, s).
MS (m/z): 389 (M⁺+H).

### Example 37: 5-[[(3,3-Difluoroprop-2-en-1-yl)sulfonyl](2,3,6-trifluorophenyl)methyl]-4-methylpyridine-2-carboxamide

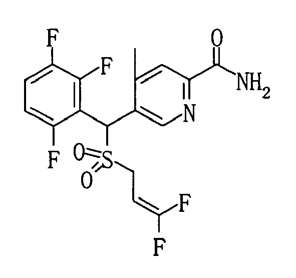

Under ice-cold conditions, 30% aqueous hydrogen peroxide (379 µl) was added to a formic acid (4 ml) solution of 5-[[(3,3-difluoroprop-2-en-1-yl)thio](2,3,6-trifluorophenyl)methyl]-4-methylpyridine-2-carboxamide (130 mg, 0.335 mmol) and the mixture was stirred at room temperature for 2 hours. Water was added to the reaction solution, followed by extraction with ethyl acetate. The organic layer was washed sequentially with water and saturated saline. The resulting organic layer was dried over anhydrous magnesium sulfate, and then concentrated under reduced pressure. The resulting residue was subjected to flash silica gel column chromatography (hexane/ethyl acetate) to give the title compound (105 mg, 0.250 mmol, 74%) as a white powder.
¹H-NMR (400 MHz, CDCl₃) δ: 2.38 (3H, s), 3.75 (1H, dt, J=8.1, 1.5Hz), 4.51 (1H, dt, J=22.7, 8.1Hz), 5.59 (1H, br s), 5.98 (1H, s), 6.96-7.02 (1H, m), 7.23-7.31 (1H, m), 7.86 (1H, br s), 8.08 (1H, s), 9.37 (1H, s).
IR (ATR) cm⁻¹: 3417, 3154, 1752, 1695, 1637, 1602, 1548, 1494, 1421, 1322, 1247, 1160, 1133, 1087, 1016.
mp: 171-172°C.
MS (m/z): 421 (M⁺+H).
Elemental analysis: C₁₇H₁₃F₅N₂O₃S: Theoretical value: C 48.57; H 3.12; F 22.60; N 6.66; S 7.63. Observed value: C 48.45; H 3.01; F 22.37; N 6.51; S 7.61.

### Example 38: 5-[[(3,3-Difluoroprop-2-en-1-yl)sulfonyl](2,3,6-trifluorophenyl)methyl]-N-(hydroxymethyl)-4-methylpyridine-2-carboxamide

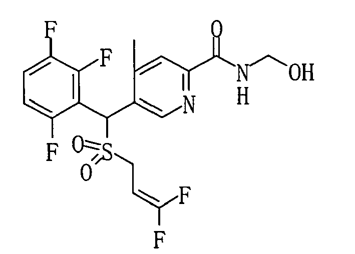

37% Aqueous formaldehyde (116 µl) and 1N aqueous sodium hydroxide (95 µl, 0.095 mmol) were added to an ethylene glycol dimethylether (4 ml) solution of 5-[[(3,3-difluoroprop-2-en-1-yl)sulfonyl](2,3,6-trifluorophenyl)methyl]-4-methylpyridine-2-carboxamide (200 mg, 0.476 mmol). The same amount was added after 1.5 hours and after 3 hours at room temperature, and the mixture was stirred for a further 1 hour. Water was added to the reaction solution, followed by extraction with ethyl acetate, and the organic layer was washed with saturated saline. The resulting organic layer was dried over anhydrous magnesium sulfate, and then concentrated under reduced pressure. The resulting residue was subjected to flash silica gel column chromatography (hexane/ethyl acetate) to give the title compound (81 mg, 0.180 mmol, 38%) as a white powder.
¹H-NMR (400 MHz, CDCl₃) δ: 2.38 (3H, s), 3.03 (1H, t, J=7.7Hz), 3.74 (2H, d, J=8.1Hz), 4.50 (1H, dt, J=22.8, 8.1Hz), 4.99-5.03 (2H, m), 5.98 (1H, s), 6.96-7.02 (1H, m), 7.23-7.32 (1H, m), 8.06 (1H, s), 8.86 (1H, br s), 9.37 (1H, s).
IR (ATR) cm⁻¹: 3324, 1745, 1658, 1600, 1517, 1496, 1394, 1326, 1245, 1209, 1160, 1135, 1089, 1041, 1016.
MS (m/z): 451 (M⁺+H).
TOF-ESI-MS: 451.0721 (calculated as C₁₈H₁₆F₅N₂O₄S: 451.0751).

### Example 39: 4-Methyl-5-[(2,3,6-trifluorophenyl)[(3,3,3-trifluoropropyl)thio]methyl]pyridine-2-carbaldehyde

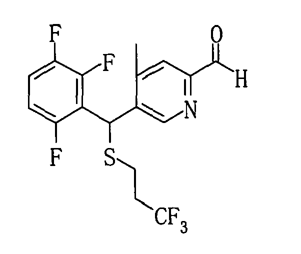

Under argon atmosphere, n-butyl lithium (1.59 M hexane solution, 3.7 ml, 5.94 mmol) was added at -70°C to a toluene (50 ml) solution of 2-bromo-4-methyl-5-[(2,3,6-trifluorophenyl)[(3,3,3-trifluoropropyl)thio]methyl]pyridine (2.40 g, 5.40 mmol) obtained in Example 1. The reaction mixture was stirred at -40°C for 10 minutes, and then cooled again to - 75°C, followed by addition of N,N-dimethylformamide (0.46 ml, 5.94 mmol). After dropwise addition was completed, the temperature of the reaction mixture was raised to room temperature, and water was added to the mixture at the same temperature. The reaction mixture was extracted with ethyl acetate, and the organic layer was washed with saturated saline. The organic layer was dried over anhydrous magnesium sulfate, and then concentrated under reduced pressure. The resulting residue was subjected to flash silica gel chromatography (hexane/ethyl acetate) to give the title compound (1.27 g, 3.23 mmol, 60%) as a pale yellow oil.
¹H-NMR (400 MHz, CDCl₃) δ: 2.35 (3H, s), 2.31-2.50 (2H, m), 2.68-2.80 (2H, m), 5.59 (1H, s), 6.85-6.91 (1H, m), 7.10-7.18 (1H, m), 7.74 (1H, s), 9.22 (1H, s).
MS (m/z) : 394 (M⁺+H).

### Example 40: 4-Methyl-5-[(2,3,6-trifluorophenyl)[(3,3,3-trifluoropropyl)sulfonyl]methyl]pyridine-2-carboxylic acid

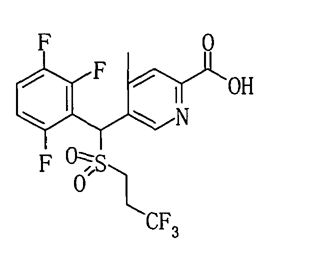

30% Aqueous hydrogen peroxide (3.6 ml) was added to a formic acid (10 ml) solution of 4-methyl-5-[(2,3,6-trifluorophenyl)[(3,3,3-trifluoropropyl)thio]methyl]pyridine-2-carbaldehyde (1.25 g, 3.18 mmol), and the mixture was stirred at 0°C for 1 hour, and then at room temperature for 3 hours. Water was added to the reaction mixture, followed by extraction with ethyl acetate, and the mixture was washed with water, and then with saturated saline. The resulting organic layer was dried over anhydrous magnesium sulfate, and then concentrated under reduced pressure. The resulting residue was recrystallized from ethanol to give the title compound (1.20 g, 2.72 mmol, 86%) as a white solid.
¹H-NMR (400 MHz, CDCl₃) δ: 2.43 (3H, s), 2.61-2.80 (2H, m), 3.19-3.36 (2H, m), 5.94 (1H, s), 7.01-7.07 (1H, m), 7.29-7.37 (1H, m), 8.11 (1H, s), 9.38 (1H, s).
IR (ATR) cm⁻¹: 1697, 1600, 1494, 1446, 1409, 1382, 1336, 1299, 1272, 1249, 1214, 1141, 1091, 1041.
mp: 85-87°C.
MS (m/z) : 442 (M⁺+H).

### Example 41: N,N,4-Trimethyl-5-[(2,3,6-trifluorophenyl)[(3,3,3-trifluoropropyl)sulfonyl]methyl]pyridine-2-carboxamide

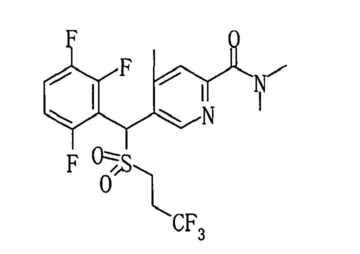

N-Methylmorpholine (120 µl, 1.09 mmol), 1-hydroxybenzotriazole (19 mg, 0.544 mmol), 1-ethyl-3-(3-dimethylaminopropyl)carbodiimide hydrochloride (104 mg, 0.544 mmol) and dimethylethylamine hydrochloride (44 mg, 0.544 mmol) were added to a dichloromethane (5 ml) solution of 4-methyl-5-[(2,3,6-trifluorophenyl)[(3,3,3-trifluoropropyl)sulfonyl]methyl]pyridine-2-carboxylic acid (200 mg, 0.453 mmol), and the mixture was stirred at room temperature for 17 hours. Water was added to the reaction solution, followed by extraction with dichloromethane. The resulting organic layer was dried over anhydrous magnesium sulfate, and then concentrated under reduced pressure. The resulting residue was subjected to flash silica gel column chromatography (hexane/ethyl acetate) to give the title compound (114 mg, 0.243 mmol, 54%) as a white powder.
¹H-NMR (400 MHz, CDCl₃) δ: 2.36 (3H, s), 2.58-2.78 (2H, m), 3.14 (3H, s), 3.15 (3H, s), 3.18-3.38 (2H, m), 5.94 (1H, s), 6.97-7.04 (1H, m), 7.25-7.33 (1H, m), 7.57 (1H, s), 9.26 (1H, s).
IR (ATR) cm⁻¹: 1629, 1598, 1550, 1498, 1446, 1405, 1334, 1297, 1284, 1274, 1259, 1213, 1145, 1137, 1085, 1033.
mp: 203-204°C.
MS (m/z): 469 (M⁺+H).
Elemental analysis: C₁₉H₁₈F₆N₂O₃S: Theoretical value: C 48.72; H 3.87; F 24.38; N 5.98; S 6.85. Observed value: C 48.63; H 3.75; F 24.38; N 5.77; S 6.93.

### Example 42: N-(Hydroxyethyl)-4-methyl-5-[(2,3,6-trifluorophenyl)[(3,3,3-trifluoropropyl)sulfonyl]methyl]pyridine-2-carboxamide

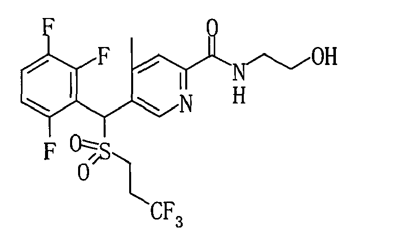

N-Methylmorpholine (60 µl, 0.544 mmol), 1-hydroxybenzotriazole (19 mg, 0.544 mmol), 1-ethyl-3-(3-dimethylaminopropyl)carbodiimide hydrochloride (104 mg, 0.544 mmol) and ethanolamine (33 µl, 0.544 mmol) were added to a dichloromethane (5 ml) solution of 4-methyl-5-[(2,3,6-trifluorophenyl)[(3,3,3-trifluoropropyl)sulfonyl]methyl]pyridine-2-carboxylic acid (200 mg, 0.453 mmol) obtained in Example 40, and the mixture was stirred at room temperature for 17 hours. Water was added to the reaction solution, followed by extraction with dichloromethane. The resulting organic layer was dried over anhydrous magnesium sulfate, and then concentrated under reduced pressure. The resulting residue was subjected to flash silica gel column chromatography (hexane/ethyl acetate) to give the title compound (81 mg, 0.167 mmol, 37%) as a white powder.
¹H-NMR (400 MHz, CDCl₃) δ: 2.39 (3H, s), 2.51 (1H, t, J=5.4Hz), 2.59-2.78 (2H, m), 3.17-3.35 (2H, m), 3.64-3.68 (2H, m), 3.84-3.87 (2H, m), 5.94 (1H, s), 6.98-7.04 (1H, m), 7.26-7.34 (1H, m), 8.08 (1H, s), 8.42 (1H, br s), 9.29 (1H, s).
IR (ATR) cm⁻¹: 3386, 1666, 1637, 1602, 1531, 1500, 1442, 1386, 1305, 1282, 1255, 1209, 1141, 1132, 1103, 1079.
mp: 146-147°C.
MS (m/z): 485 (M⁺+H).
Elemental analysis: C₁₉H₁₈F₆N₂O₄S: Theoretical value: C 47.11; H 3.75; F 23.53; N 5.78; S 6.62. Observed value : C 47.05; H 3.63; F 23.51; N 5.69; S 6.64.

### Reference Example 22: (2-Bromo-5-chloropyridin-4-yl) (2,3,6-trifluorophenyl)methanol

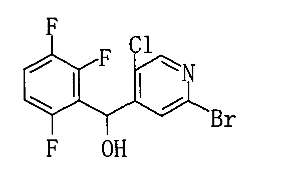

Under argon atmosphere, n-butyl lithium (1.58 M hexane solution, 23 ml, 36.4 mmol) was added at -75°C to a tetrahydrofuran (50 ml) solution of diisopropylamine (5.5 ml, 39.0 mmol), and the mixture was stirred for 1 hour. A tetrahydrofuran (25 ml) solution of 2-bromo-5-chloropyridine (5.0 g, 26.0 mmol) was added dropwise to the reaction solution, and the mixture was stirred for 1 hour. 2,3,6-Trifluorobenzaldehyde (3.34 ml, 28.6 mmol) was added dropwise to the reaction solution, and the mixture was stirred for 2.5 hours whilst raising the temperature to room temperature. Water was added to the reaction solution, followed by concentration under reduced pressure, and then extraction was carried out with ethyl acetate. The resulting organic layer was washed with saturated saline, dried over magnesium sulfate, and then concentrated under reduced pressure. The resulting residue was subjected to flash silica gel column chromatography (hexane/ethyl acetate), and then washed with dichloromethane/hexane to give the title compound (5.08 g, 14.4 mmol, 55%) as a white powder.
¹H-NMR (400 MHz, CDCl₃) δ: 2.71 (1H, d, J=5.2Hz), 6.28 (1H, d, J=5.2Hz), 6.82-6.88 (1H, m), 7.12-7.21 (1H, m), 8.06 (1H, s), 8.25 (1H, s).
MS (m/z) : 352 (M⁺+H).

### Reference Example 23: 2-Bromo-5-chloro-4-[chloro(2,3,6-trifluorophenyl)methyl]pyridine

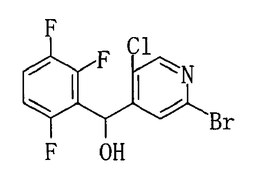

Under nitrogen atmosphere, thionyl chloride (20.6 ml, 284 mmol) was added to a solution of (2-bromo-5-chloropyridin-4-yl)(2,3,6-trifluorophenyl)methanol (5.0 g, 14.2 mmol) in dichloromethane (60 ml) and N,N-dimethylformamide (1 ml), and the mixture was stirred with heating under reflux for 19 hours. The reaction solution was concentrated under reduced pressure, followed by addition of ethyl acetate, and then washed sequentially with saturated aqueous hydrogen carbonate and saturated saline. The resulting organic layer was dried over magnesium sulfate, and then concentrated under reduced pressure. The resulting residue was subjected to flash silica gel column chromatography (hexane/ethyl acetate) to give the title compound (4.56 g, 12.3 mmol, 87%) as a pale yellow oil.
¹H-NMR (400 MHz, CDCl₃) δ: 6.51 (1H, s), 6.84-6.90 (1H, m), 7.15-7.23 (1H, m), 8.18 (1H, s), 8.29 (1H, s).
MS (m/z) : 370 (M⁺+H).

### Example 43: 2-Bromo-5-chloro-4-[(2,3,6-trifluorophenyl)[(3,3,3-trifluoropropyl)thio]methyl]pyridine

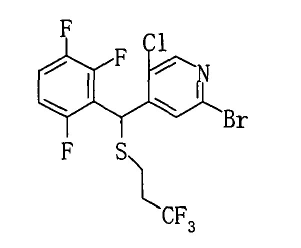

Under nitrogen atmosphere, S-potassium thioacetate (1.8 g, 15.8 mmol) was added to an acetone (50 ml) solution of 1,1,1-trifluoro-3-iodopropane (1.9 ml, 15.8 mmol), and the mixture was stirred at 50°C for 17 hours. The reaction solution was cooled to room temperature, followed by addition of methanol (20 ml), potassium carbonate (1.8 g, 13.3 mmol), and 2-bromo-5-chloro-4-[chloro(2,3,6-trifluorophenyl)methyl]pyridine (4.5 g, 12.1 mmol), and then the mixture was stirred at 50°C for 2 hours. The reaction solution was concentrated under reduced pressure, and then water was added. The mixture was extracted with ethyl acetate, and the organic layer was washed with saturated saline. The resulting organic layer was dried over anhydrous magnesium sulfate, and then concentrated under reduced pressure. The resulting residue was subjected to silica gel column chromatography using hexane:ethyl acetate = 30:1 as the eluent. The resulting fractions were concentrated under reduced pressure to give the title compound (1.56 g, 3.38 mmol, 28%) as a pale yellow oil.
¹H-NMR (400 MHz, CDCl₃) δ: 2.35-2.50 (2H, m), 2.68-2.80 (2H, m), 5.61 (1H, s), 6.84-6.90 (1H, m), 7.10-7.18 (1H, m), 8.08 (1H, s), 8.32 (1H, s).
MS (m/z) : 464 (M⁺+H).

### Example 44: 5-Chloro-4-[(2,3,6-trifluorophenyl)[(3,3,3-trifluoropropyl)thio]methyl]pyridine-2-carboxamide

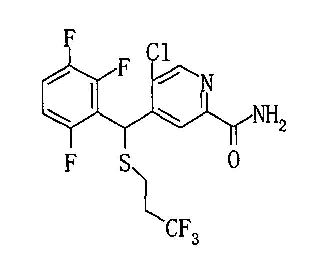

Under argon atmosphere, n-butyl lithium (1.59 M hexane solution, 0.975 ml, 1.55 mmol) was added at -75°C to a toluene (12 ml) solution of 2-bromo-5-chloro-4-[(2,3,6-trifluorophenyl)[(3,3,3-trifluoropropyl)thio]methyl]pyridine (600 mg, 1.29 mmol), and the mixture was stirred for 1 hour. Subsequently, the system was replaced with carbon dioxide gas, and then the mixture was stirred for 1 hour whilst raising the temperature to room temperature. 1N hydrochloric acid (1.5 ml) was added to the reaction solution, followed by concentration under reduced pressure. Water was added to the reaction solution, followed by extraction with dichloromethane. The organic layer was dried over anhydrous magnesium sulfate, and then concentrated under reduced pressure.

Under nitrogen atmosphere, triethylamine (360 µl, 2.58 mmol) and isobutyl chloroformate (251 µl, 1.94 mmol) were added at -10°C to a solution of the resulting residue in tetrahydrofuran (8 ml), and the mixture was stirred for 30 minutes. 7N ammonia methanol solution (5 ml) was added to the reaction solution, and the mixture was stirred at room temperature for 30 minutes. The reaction solution was concentrated under reduced pressure, followed by addition of water, and then extraction with ethyl acetate was carried out. The organic layer was washed sequentially with water and saturated saline, dried over anhydrous magnesium sulfate, and then concentrated under reduced pressure. The resulting residue was subjected to flash silica gel column chromatography (hexane/ethyl acetate) to give the title compound (240 mg, 0.560 mmol, 43%) as a white powder.
¹H-NMR (400 MHz, CDCl₃) δ: 2.34-2.49 (2H, m), 2.67-2.80 (2H, m), 5.61 (1H, br s), 5.69 (1H, s), 6.81-6.88 (1H, m), 7.08-7.16 (1H, m), 7.71 (1H, br s), 8.50 (1H, s), 8.80 (1H, s).
MS (m/z): 429 (M⁺+H).

### Example 45: 5-Chloro-4-[(2,3,6-trifluorophenyl)[(3,3,3-trifluoropropyl)sulfonyl]methyl]pyridine-2-carboxamide

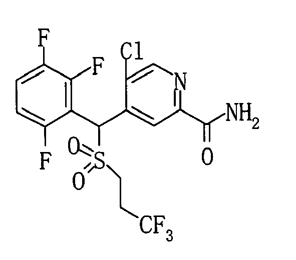

Hexaammonium heptamolybdate tetrahydrate (10 mg) and 30% aqueous hydrogen peroxide (5 ml) were added to a solution of 5-chloro-4-[(2,3,6-trifluorophenyl)[(3,3,3-trifluoropropyl)thio]methyl]pyridine-2-carboxamide (230 mg, 0.536 mmol) in a combined solvent of methanol (5 ml) and ethyl acetate (10 ml), and the mixture was stirred for 15 hours. Water was added to the reaction solution, followed by extraction with ethyl acetate, and the organic layer was washed sequentially with saturated aqueous hydrogen carbonate, saturated aqueous sodium thiosulfate and saturated saline. The resulting organic layer was dried over anhydrous magnesium sulfate, and then concentrated under reduced pressure. The resulting residue was subjected to flash silica gel column chromatography (hexane/ethyl acetate), and then washed with hexane/ethyl acetate to give the title compound (180 mg, 0.391 mmol, 73%) as a white powder.
¹H-NMR (400 MHz, CDCl₃) δ: 2.63-2.77 (2H, m), 3.21-3.54 (2H, m), 5.65 (1H, br s), 6.17 (1H, s), 6.96-7.02 (1H, m), 7.24-7.33 (1H, m), 7.67 (1H, br s), 8.62 (1H, s), 9.05 (1H, s).
IR (ATR) cm⁻¹: 3436, 1693, 1637, 1596, 1585, 1536, 1496, 1405, 1330, 1313, 1272, 1257, 1211, 1159, 1132, 1091, 1051, 1020.
mp: 237-239°C.
MS (m/z): 461 (M⁺+H).
Elemental analysis: C₁₆H₁₁ClF₆N₂O₃S: Theoretical value: C 41.71; H 2.41; Cl 7.69; F 24.74; N 6.08; S 6.96. Observed value : C 41.89; H 2.27; Cl 7.85; F 24.42; N 5.95; S 6.77.

### Example 46: 5-Chloro-N-(hydroxymethyl)-4-[(2,3,6-trifluorophenyl)[(3,3,3-trifluoropropyl)sulfonyl]methyl]pyridine-2-carboxamide

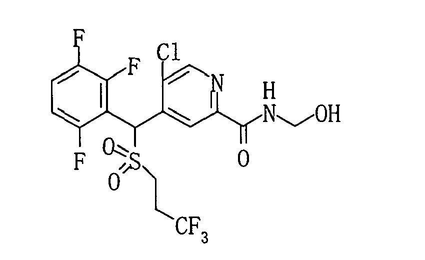

37% Aqueous formaldehyde (74 µl) and 1N aqueous sodium hydroxide (30 µl, 0.0304 mmol) were added to an ethylene glycol dimethylether (3 ml) solution of 5-chloro-4-[(2,3,6-trifluorophenyl)[(3,3,3-trifluoropropyl)sulfonyl]methyl]pyridine-2-carboxamide (140 mg, 0.304 mmol), and the mixture was stirred at room temperature for 1.5 hours. Water was added to the reaction solution, followed by extraction with ethyl acetate, and the organic layer was washed with saturated saline. The organic layer was dried over anhydrous magnesium sulfate, and then concentrated under reduced pressure. The resulting residue was subjected to flash silica gel column chromatography (hexane/ethyl acetate) to give the title compound (90 mg, 0.183 mmol, 60%) as a white amorphous. ¹H-NMR (400 MHz, CDCl₃) δ: 2.62-2.79 (2H, m), 3.12 (1H, t, J=7.8Hz), 3.20-3.34 (2H, m), 5.01 (2H, t, J=7.8Hz), 6.17 (1H, s), 6.97-7.03 (1H, m), 7.26-7.34 (1H, m), 8.62 (1H, s), 8.67 (1H, t, J=7.8Hz), 9.06 (1H, s).
IR (ATR) cm⁻¹: 1675, 1637, 1590, 1521, 1496, 1459, 1386, 1338, 1305, 1272, 1247, 1211, 1143, 1093, 1033. MS (m/z) : 491 (M⁺+H).
Elemental analysis: C₁₇H₁₃ClF₆N₂O₄S: Theoretical value: C 41.60; H 2.67; Cl 7.22; F 23.23; N 5.71; S 6.53. Observed value: C 41.70; H 2.56; Cl 7.30; F 23.03; N 5.59; S 6.53.

### Example 47: 5-Chloro-N-(3,4-dimethoxybenzyl)-4-[(2,3,6-trifluorophenyl)[(3,3,3-trifluoropropyl)thio]methyl]pyridin-2-amine

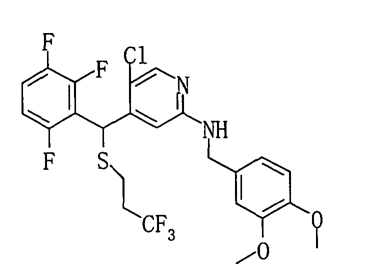

Under nitrogen atmosphere, 3,4-dimethoxybenzylamine (903 µl, 5.81 mmol) was added to a N-methyl-2-pyrrolidone (20 ml) solution of 2-bromo-5-chloro-4-[(2,3,6-trifluorophenyl)[(3,3,3-trifluoropropyl)thio]methyl]pyridine (900 mg, 1.94 mmol) obtained in Example 43, and the mixture was heated at 150°C for 4 hours. The reaction solution was cooled to room temperature, followed by addition of water, and then extraction with ethyl acetate was carried out. The organic layer was washed with saturated saline, dried over anhydrous magnesium sulfate, and then concentrated under reduced pressure. The resulting residue was subjected to flash silica gel column chromatography (hexane/ethyl acetate) to give the title compound (0.18 g, 0.327 mmol, 17%) as a pale yellow oil.
¹H-NMR (400 MHz, CDCl₃) δ: 2.28-2.40 (2H, m), 2.63 (2H, t, J=7.9Hz), 3.87 (3H, s), 3.88 (3H, s), 4.41-4.56 (2H, m), 4.97 (1H, t, J=5.3Hz), 5.57 (1H, s), 6.77-6.93 (4H, m), 6.99 (1H, s), 7.04-7.13 (1H, m), 8.02 (1H, s).
MS (m/z) : 551 (M⁺+H).

### Example 48: 5-Chloro-4-[(2,3,6-trifluorophenyl)[(3,3,3-trifluoropropyl)sulfonyl]methyl]pyridin-2-amine

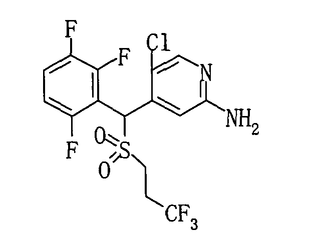

Hexaammonium heptamolybdate tetrahydrate (10 mg) and 30% aqueous hydrogen peroxide (3 ml) were added to a solution of 5-chloro-N-(3,4-dimethoxybenzyl)-4-[(2,3,6-trifluorophenyl)[(3,3,3-trifluoropropyl)thio]methyl]pyridin-2-amine (180 mg, 0.327 mmol) in a combined solvent of methanol (3 ml) and ethyl acetate (5 ml), and the mixture was stirred for 7 hours. Water was added to the reaction solution, followed by extraction with ethyl acetate, and then the organic layer was washed sequentially with saturated aqueous hydrogen carbonate, saturated aqueous sodium thiosulfate and saturated saline. The resulting organic layer was dried over anhydrous magnesium sulfate, and then concentrated under reduced pressure.

Under nitrogen atmosphere, trifluoroacetic acid (3 ml) was added to the resulting residue, and the mixture was stirred at 60°C for 1 hour. The reaction solution was concentrated under reduced pressure, followed by addition of ethyl acetate, and the mixture was washed sequentially with saturated aqueous sodium hydrogen carbonate and saturated saline. The resulting organic layer was dried over magnesium sulfate, and then concentrated under reduced pressure. The resulting residue was subjected to flash silica gel column chromatography (hexane/ethyl acetate). 1N hydrochloric acid (240 µl) was added to an ethanol (1 ml) solution of the resulting residue. The precipitated solid was filtered off and washed with ethanol to give the title compound (42 mg, 0.0720 mmol, 26%) as a white powder.
¹H-NMR (400 MHz, DMSO-d₆) δ: 2.77-2.88 (2H, m), 3.62-3.84 (2H, m), 6.35 (1H, s), 7.26-7.32 (1H, m), 7.45 (1H, s), 7.66-7.74 (1H, m), 8.06 (1H, s). IR (ATR) cm⁻¹: 3139, 1668, 1616, 1498, 1465, 1380, 1336, 1297, 1270, 1251, 1214, 1145, 1091, 1037.
mp: 155-159°C.
MS (m/z): 433 (M⁺+H).

### Example 49: 7-Chloro-2-hydroxy-8-[(2,3,6-trifluorophenyl)[(3,3,3-trifluoropropyl)sulfonyl]methyl]-4H-pyrido[1,2-a]pyrimidin-4-one

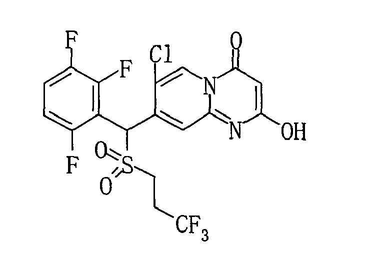

Under nitrogen atmosphere, bis(2,4,6-trichlorophenyl) malonate ester (1.03 g, 2.22 mmol) was added to a toluene (15 ml) solution of 5-chloro-4-[(2,3,6-trifluorophenyl)[(3,3,3-trifluoropropyl)sulfonyl]methyl]pyridin-2-amine (640 mg, 1.48 mmol), and the mixture was stirred with heating under reflux for 2 hours. The solid precipitated was filtered, and then washed sequentially with toluene, hexane and dichloromethane to give the title compound (550 mg, 1.10 mmol, 74%) as a pale yellow powder.
¹H-NMR (400 MHz, CD₃OD) δ: 2.72-2.88 (3H, m), 3.61-3.67 (2H, m), 5.45 (1H, s), 7.12-7.19 (1H, m), 7.48-7.56 (1H, m), 8.44 (1H, s), 9.07 (1H, s).
IR (ATR) cm⁻¹: 1708, 1621, 1517, 1500, 1477, 1442, 1386, 1361, 1332, 1305, 1276, 1245, 1216, 1141, 1095, 1041, 1027.
mp: 264°C (decompt.).
MS (m/z) : 501 (M⁺+H).
TOF-ESI-MS: 501.0079 (calculated as C₁₈H₁₂C1F₆N₂O₄S: 501.0111). Elemental analysis: C₁₈H₁₁ClF₆N₂O₉S: Theoretical value: C 43.17; H 2.21; Cl 7.08; F 22.76; N 5.59; S 6.40. Observed value: C 43.19; H 2.07; Cl 7.25; F 22.34; N 5.47; S 6.42.

### Example 50: 7-Chloro-2-[(3,4-dimethoxybenzyl)amino]-8-[(2,3,6-trifluorophenyl)[(3,3,3-trifluoropropyl)sulfonyl]methyl]-4H-pyrido[1,2-a]pyrimidin-4-one

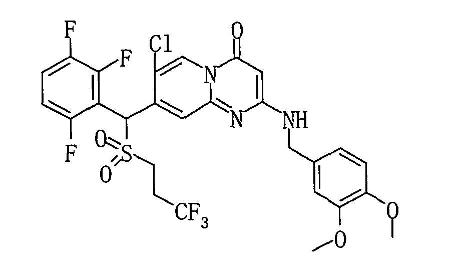

Under nitrogen atmosphere, 4-dimethylaminopyridine (117 mg, 0.959 mmol) and p-toluenesulfonyl chloride (183 mg, 0.959 mmol) were added to a dichloromethane (10 ml) solution of 7-chloro-2-hydroxy-8-[(2,3,6-trifluorophenyl)[(3,3,3-trifluoropropyl)sulfonyl]methyl]-4H-pyrido[1,2-a]pyrimidin-4-one (400 mg, 0.799 mmol), and the mixture was stirred at room temperature for 30 minutes. Water was added to the reaction solution, followed by extraction with dichloromethane. The solution was dried over anhydrous magnesium sulfate, and then concentrated under reduced pressure. The resulting residue was subjected to silica gel column chromatography (hexane/ethyl acetate).

Under nitrogen atmosphere, 3,4-dimethoxybenzylamine (372 µl, 2.40 mmol) was added to a 1,4-dioxane (10 ml) solution of the resulting residue, and the mixture was stirred at 50°C for 3 hours. The reaction solution was concentrated under reduced pressure, and the resulting residue was subjected to flash silica gel column chromatography (hexane/ethyl acetate) to give the title compound (280 mg, 0.431 mmol, 60%) as a pale yellow oil.
¹H-NMR (400 MHz, CDCl₃) δ: 2.63-2.81 (2H, m), 3.23-3.39 (2H, m), 3.88 (6H, s), 4.43 (2H, br s), 5.33 (1H, br s), 5.47 (1H, s), 6.06 (1H, s), 6.84-6.91 (3H, m), 6.99-7.05 (1H, m), 7.28-7.37
(1H, m), 8.23 (1H, s), 8.97 (1H, s).
MS (m/z): 650 (M⁺+H).

### Example 51: 2-Amino-7-chloro-8-[(2,3,6-trifluorophenyl)[(3,3,3-trifluoropropyl)sulfonyl]methyl]-4H-pyrido[1,2-a]pyrimidin-4-one

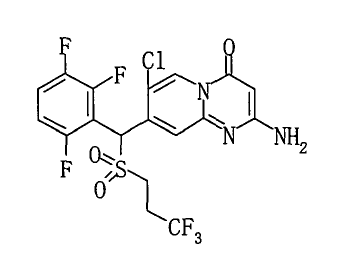

Under nitrogen atmosphere, trifluoroacetic acid (3 ml) was added to 7-chloro-2-[(3,4-dimethoxybenzyl)amino]-8-[(2,3,6-trifluorophenyl)[(3,3,3-trifluoropropyl)sulfonyl]methyl]-4H-pyrido[1,2-a]pyrimidin-4-one (270 mg, 0.415 mmol), and the mixture was stirred at 60°C for 30 minutes. The reaction solution was concentrated under reduced pressure, followed by addition of ethyl acetate, and then the solution was washed sequentially with saturated aqueous sodium hydrogen carbonate and saturated saline. The resulting organic layer was dried over magnesium sulfate, and then concentrated under reduced pressure. The resulting residue was subjected to flash silica gel column chromatography (hexane/ethyl acetate), and then recrystallization from hexane/ethyl acetate to give the title compound (95 mg, 0.190 mmol, 46%) as a white powder.
¹H-NMR (400 MHz, CDCl₃) δ: 2.62-2.81 (2H, m), 3.23-3.39 (2H, m), 4.88 (2H, s), 5.56 (1H, s), 6.07 (1H, s), 6.98-7.04 (1H, m), 7.28-7.36 (1H, m), 8.27 (1H, s), 9.02 (1H, s).
IR (ATR) cm⁻¹: 3149, 1679, 1637, 1563, 1531, 1498, 1446, 1386, 1340, 1295, 1280, 1247, 1216, 1141, 1095, 1045.
mp: 247°C (decompt.).
MS (m/z) : 500 (M⁺+H).
TOF-ESI-MS: 500.0260 (Calculated value as C₁₈H₁₃ClF₆N₃O₃S: 500.0270).

### Reference Example 24: 2,5-Dichloro-4-[chloro(2,3,6-trifluorophenyl)methyl]pyridine

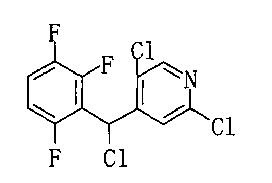

Under argon atmosphere, n-butyl lithium (1.54 M hexane solution, 51 ml, 81.1 mmol) was added at -70°C to a tetrahydrofuran (250 ml) solution of diisopropylamine (11.4 ml, 67.6 mmol), and the mixture was stirred for 1 hour. A tetrahydrofuran (50 ml) solution of 2,5-dichloropyridine (48) (10.0 g, 67.6 mmol) was added dropwise to the reaction solution, and the mixture was stirred for 1 hour. 2,3,6-Trifluorobenzaldehyde (10.3 ml, 87.8 mmol) was added dropwise to the reaction solution, and the mixture was stirred for 1 hour whilst raising the temperature to 0°C. Water was added to the reaction solution, followed by concentration under reduced pressure, and then extraction with ethyl acetate was carried out. The resulting organic layer was washed with saturated saline, dried over magnesium sulfate, and then concentrated under reduced pressure. The resulting residue was washed with dichloromethane/hexane.

Under nitrogen atmosphere, thionyl chloride (76 ml, 1.05 mol) was added to a solution of the resulting residue in dichloromethane (200 ml) and N,N-dimethylformamide (5 ml), and the mixture was stirred with heating under reflux for 18 hours. The reaction solution was concentrated under reduced pressure, followed by addition of saturated aqueous hydrogen carbonate, and extraction with ethyl acetate was carried out. The organic layer was washed with saturated saline, dried over magnesium sulfate, and then concentrated under reduced pressure to give the title compound (16.9 g, 51.8 mmol, 77%) as a white powder. ¹H-NMR (400 MHz, CDCl₃) δ: 6.52 (1H, s), 6.84-6.90 (1H, m), 7.15-7.23 (1H, m), 8.04 (1H, s), 8.31 (1H, s).
MS (m/z): 326 (M⁺+H).

### Example 52: 2,5-Dichloro-4-[(2,3,6-trifluorophenyl)[(3,3,3-trifluoropropyl)sulfonyl]methyl]pyridine

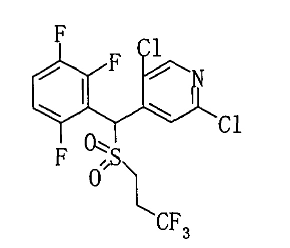

Under nitrogen atmosphere, S-potassium thioacetate (2.3 g, 19.9 mmol) was added to an acetone (50 ml) solution of 1,1,1-trifluoro-3-iodopropane (2.4 ml, 19.9 mmol), and the mixture was stirred at 50°C for 16 hours. The reaction solution was cooled to room temperature, followed by addition of methanol (50 ml), potassium carbonate (2.3 g, 16.8 mmol), 2,5-dichloro-4-[chloro(2,3,6-trifluorophenyl)methyl]pyridine (5.0 g, 15.3 mmol), and the mixture was stirred at 50°C for 1.5 hours. The reaction solution was concentrated under reduced pressure, followed by addition of water, and extraction with dichloromethane was carried out. The organic layer was dried over anhydrous magnesium sulfate, and then concentrated under reduced pressure.

Hexaammonium heptamolybdate tetrahydrate (100 mg) and 30% aqueous hydrogen peroxide (20 ml) were added to a solution of the resulting residue in a combined solvent of methanol (20 ml) and ethyl acetate (30 ml), and the mixture was stirred for 21 hours. Water was added to the reaction solution, followed by extraction with ethyl acetate, and the organic layer was washed sequentially with saturated aqueous hydrogen carbonate, saturated aqueous sodium thiosulfate and saturated saline. The resulting organic layer was dried over anhydrous magnesium sulfate, and then concentrated under reduced pressure. The resulting residue was subjected to silica gel column chromatography using hexane:ethyl acetate = 9:1 as the eluent. There resulting fractions were concentrated under reduced pressure to give the title compound (1.86 g, 4.11 mmol, 27%) as a pale yellow oil.
¹H-NMR (400 MHz, CDCl₃) δ: 2.59-2.81 (2H, m), 3.19-3.35 (2H, m), 6.08 (1H, s), 6.98-7.04 (1H, m), 7.28-7.36 (1H, m), 8.26 (1H, s), 8.45 (1H, s).
MS (m/z) : 452 (M⁺+H).

### Example 53: 5-Chloro-2-hydrazino-4-[(2,3,6-trifluoropheynyl)[(3,3,3-trifluoropropyl)sulfonyl]methyl]pyridine

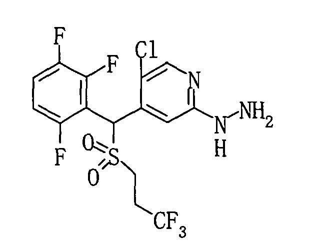

Hydrazine monohydrate (1.0 ml) was added to an ethanol (5 ml) solution of 2,5-dichloro-4-[(2,3,6-trifluorophenyl)[(3,3,3-trifluoropropyl)sulfonyl]methyl]pyridine (190 mg, 0.420 mmol), and the mixture was stirred with heating under reflux for 4 hours. The reaction solution was concentrated under reduced pressure, and the resulting residue was subjected to flash silica gel column chromatography (hexane/ethyl acetate) to give the title compound (130 mg, 0.290 mmol, 69%) as a colorless amorphous.
¹H-NMR (400 MHz, CDCl₃) δ: 2.60-2.79 (2H, m), 3.19-3.34 (2H, m), 3.86 (2H, br s), 6.03 (1H, br s), 6.10 (1H, s), 6.94-7.01 (1H, m), 7.23-7.31 (1H, m), 7.65 (1H, s), 8.14 (1H, s).
MS (m/z): 448 (M⁺+H).

### Example 54: 6-Chloro-7-[(2,3,6-trifluorophenyl)[(3,3,3-trifluoropropyl)sulfonyl]methyl][1,2,4]triazolo[4,3-a]pyridin-3(2H)-one

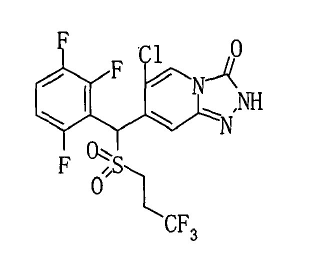

Under nitrogen atmosphere, triphosgene (129 mg, 0.435 mmol) was added under ice-cold conditions to a toluene (5 ml) solution of 5-chloro-2-hydrazino-4-[(2,3,6-trifluorophenyl)[(3,3,3-trifluoropropyl)sulfonyl]methyl]pyridine (130 mg, 0.290 mmol), and the mixture was stirred at room temperature for 15 minutes, and then stirred with heating under reflux for 2 hours. Ethyl acetate was added to the reaction solution, and the mixture was washed sequentially with saturated aqueous hydrogen carbonate and saturated saline. The resulting organic layer was dried over anhydrous magnesium sulfate, and then concentrated under reduced pressure. The resulting residue was subjected to flash silica gel column chromatography (hexane/ethyl acetate), recrystallized from diethylether-hexane to give the title compound (82 mg, 0.173 mmol, 59%) as a pale yellow powder.
¹H-NMR (400 MHz, CDCl₃) δ: 2.62-2.81 (2H, m), 3.21-3.38 (2H, m), 5.99 (1H, s), 6.98-7.04 (1H, m), 7.28-7.36 (1H, m), 7.62 (1H, s), 8.22 (1H, s), 9.60 (1H, br s).
IR (ATR) cm⁻¹: 1722, 1710, 1637, 1604, 1542, 1496, 1450, 1386, 1336, 1305, 1274, 1247, 1211, 1093.
mp: 209-211°C.
MS (m/z): 474 (M⁺+H).
Elemental analysis: C₁₆H₁₀ClF₆N₃O₃S: Theoretical value: C 40.56; H 2.13; Cl 7.48; F 24.06; N 8.87; S 6.77. Observed value: C 40.59; H 2.17; Cl 7.55; F 23.93; N 8.98; S 6.83.

### (Test Example 1) Screening system using cells for the identification of substances which inhibit production secretion of β-amyloid protein

Inhibitory activity of compounds against production of β-amyloid protein was measured by quantitative determination of the amount of β-amyloid protein (Aβ) secreted into the culture medium by sandwich enzyme-linked immunosorbent assay (ELISA) method, using E35 cells prepared by transfecting APP751 gene, which is a human wild type β-amyloid protein precursor protein gene, into human glioma cells (H4 cells).

That is, E35 cells were inoculated into a 96-well plate followed by cultivation in an incubator equilibrated with 5% carbon dioxide at 37°C, using Dulbecco's Modified Eagle's Medium supplemented with inactivated 10% fetal bovine serum as the medium. Test compounds dissolved in DMSO solution were added 24 hours after inoculation. Test compounds in DMSO solution were prepared to have a 2000-fold concentration of the final concentration, so that the DMSO concentration in the medium becomes 0.05%. After further cultivation for 24 hours, supernatant was collected, and was added to a 96-well plate for ELISA fixed with monoclonal antibody 25-1 against Aβ. Incubation was carried out at 4°C for 16-20 hours. The plate was washed with phosphate buffer (pH 7.4), followed by addition of biotinylated monoclonal antibody MA32-40 against Aβ, and the plate was allowed to stand at 4°C for 2 hours. After the plate was washed with phosphate buffer again, streptavidin conjugated alkaline phosphatase was added. Biotin was allowed to conjugate with streptavidin, and then the plate was washed with phosphate buffer. Blue Phos (KPL Inc.) was added to the plate, and then after incubation for an appropriate period of time, absorbance was measured for each of the wells. The amount of Aβ contained in the supernatnat of conditioned medium was obtained from a calibration curve which was prepared separately. The concentration at which production of Aβ is inhibited by 50% (EC₅₀), when compared with the control cells without the addition of the compound, was calculated. Here, 25-1 antibody and MA32-40 antibody used in ELISA are mouse monoclonal antibodies derived from hybridoma cell clone cell lines, which specifically recognize each antigen which was prepared and selected in accordance with a standard method by using Aβ25-35 and Aβ1-8 as antigens, respectively.

The cytotoxicity-expressing concentration was obtained by the following test. H4 cells were cultured on a 96-well plate until semi-confluent, followed by addition of test compounds, and then cultivation was continued further. After 24 hours, Alamar Blue (BIOSOURCE Inc.) was used to allow formation of color, and dye concentration was measured to obtain viable cell count. The concentration at which the viable cell count becomes 80% or less than the control cell without the addition of the compound, was taken as the cytotoxicity-expressing concentration. The compound having a deviated cytotoxicity-expressing concentration with respect to EC₅₀ was determined as the compound having activity to inhibit production of β-amyloid protein.

Results for the evaluation made by the aforementioned assay with respect to compound (I) of the present invention are shown in Table 1. Notation of ++++ is provided when EC₅₀ is 5 nM or lower, +++ when 5-50 nM, and ++ when 50-500 nM.

**[Table 1]**

| Example No. | Activity |
|---|---|
| 3 | ++ |
| 4 | +++ |
| 10 | ++ |
| 15 | ++ |
| 18 | ** |
| 19 | +++ |
| 30 | ++ |
| 34 | +++ |
| 35 | +++ |
| 37 | ++ |
| 45 | ++ |
| 48 | +++ |
| 49 | +++ |
| 51 | ++++ |
| 54 | +++ |

### [Industrial Applicability]

The compound represented by the general formula (I) of the present invention is useful as a preventive therapeutic drug for diseases associated with abnormal production and/or secretion of β-amyloid protein, such as Alzheimer's disease, Down's syndrome and other diseases associated with amyloid deposition.

## Claims

1. A compound represented by the general formula (I): 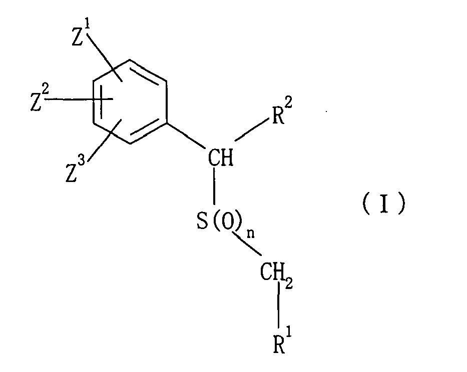 [wherein,
R¹ represents a Cl-C6 alkyl group which may have 1 to 6 halogen atoms as substituent groups, a C2-C6 alkenyl group which may have 1 to 6 halogen atoms as substituent groups, or a C3-C7 cycloalkyl group which may have 1 to 6 halogen atoms as substituent groups;
R² represents a 6-membered nitrogen-containing monocyclic aromatic heterocyclic group having 1 to 3 substituent groups, or a 9- or 10-membered nitrogen-containing bicyclic heterocyclic group having 1 to 4 substituent groups;
Z¹, Z² and Z³ each independently represent a hydrogen atom, a halogen atom or a cyano group; and
n represents 0, 1 or 2],
salts thereof, or solvates thereof.

2. The compound according to Claim 1, salts thereof, or solvates thereof, wherein R² in the general formula (I) is any one of a group selected from the group consisting of a pyridyl group, a triazolopyridyl group and a pyridopyrimidinyl group, having 1 to 3 substituent groups.

3. The compound according to Claim 1, salts thereof, or solvates thereof, wherein R² in the general formula (I) is a pyridyl group having 1 to 3 substituent groups.

4. The compound according to Claim 1, salts thereof, or solvates thereof, wherein R² in the general formula (I) is a group represented by the following formula (1): 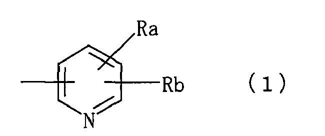 [wherein
Rₐ represents a halogen atom or a C1-C6 alkyl group; and R_{b} represents a carboxy C1-C6 alkyl group, a carbamoyl group, a N-C1-C6 alkylcarbamoyl group, a N,N-di(C1-C6 alkyl)carbamoyl group, a N-(hydroxy C1-C6 alkyl)carbamoyl group, a carboxy group, an amino group, a N-(hydroxy C1-C6 alkyl)amino group or a C1-C6 alkylsulfonylamino group].

5. The compound according to Claim 4, salts thereof, or solvates thereof, wherein Rₐ is a C1-C6 alkyl group and R_{b} is a N-(hydroxy C1-C6 alkyl)carbamoyl group in formula (1).

6. The compound according to Claim 1, salts thereof, or solvates thereof, wherein R² in the general formula (I) is a group represented by the following formula (2): 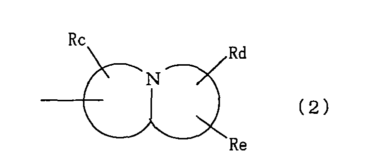 [wherein
the group of nitrogen-containing ring structure represents a triazolopyridyl group or a pyridopyrimidinyl group; R_{c} represents a halogen atom or a C1-C6 alkyl group; R_{d} represents a hydrogen atom, a hydroxy group, an amino group, a N-(C1-C6 alkyl)amino group or a N-(hydroxy C1-C6 alkyl)amino group; and Rₑ represents a hydroxy group or an oxo group].

7. The compound according to any one of Claims 1 to 6, salts thereof, or solvates thereof, wherein R¹ in the general formula (I) is a C1-C6 alkyl group which may have 1 to 6 halogen atoms as substituent groups.

8. The compound according to any one of Claims 1 to 6, salts thereof, or solvates thereof, wherein R¹ in the general formula (I) is a 2,2,2-trifluoroethyl group, a 2-chloro-2,2-difluoroethyl group, a 2,2-difluoropropyl group or a 2,2,3,3,3-pentafluoropropyl group.

9. The compound according to any one of Claims 1 to 8, salts thereof, or solvates thereof, wherein Z¹ and Z² are both fluorine atoms and Z³ is a hydrogen atom, or Z¹, Z² and Z³ are each a fluorine atom, in the general formula (I).

10. The compound according to any one of Claims 1 to 9, salts thereof, or solvates thereof, wherein n in the general formula (I) is 2.

11. A medicament containing the compound according to any one of Claims 1 to 10, salts thereof, or solvates thereof.

12. The medicament according to Claim 11, which is a drug for preventing or treating a disease associated with abnormal generation and/or secretion of β-amyloid protein.

13. The medicament according to Claim 12, wherein the disease associated with abnormal generation and/or secretion of β-amyloid protein is Alzheimer's disease or Down's syndrome.

14. A pharmaceutical composition containing the compound according to any one of Claims 1 to 10, salts thereof, or solvates thereof, and a pharmaceutically acceptable carrier.

15. Use of the compound according to any one of Claims 1 to 10, salts thereof, or solvates thereof, for the manufacture of a medicament.

16. The use according to Claim 15, wherein the medicament is a drug for preventing or treating a disease associated with abnormal generation and/or secretion of β-amyloid protein.

17. The use according to Claim 16, wherein the disease associated with abnormal generation and/or secretion of β-amyloid protein is Alzheimer's disease or Down's syndrome.

18. A method for treating a disease associated with abnormal generation and/or secretion of β-amyloid protein, comprising administration of an effective amount of the compound according to any one of Claims 1 to 10, salts thereof, or solvates thereof.

19. The treatment method according to Claim 18, wherein the disease associated with abnormal generation and/or secretion of β-amyloid protein is Alzheimer's disease or Down's syndrome or the like.
